(19) Europäisches Patentamt
European Patent Office
Office européen des brevets

(11) **EP 3 047 852 A1**

(12) **EUROPEAN PATENT APPLICATION**
published in accordance with Art. 153(4) EPC

(43) Date of publication:
**27.07.2016 Bulletin 2016/30**

(21) Application number: **14846851.5**

(22) Date of filing: **26.09.2014**

(51) Int Cl.:
***A61K 31/7088*** (2006.01) ***A61K 35/76*** (2015.01)
***A61K 48/00*** (2006.01) ***A61P 1/00*** (2006.01)
***A61P 11/00*** (2006.01) ***A61P 11/06*** (2006.01)
***A61P 17/00*** (2006.01) ***A61P 17/02*** (2006.01)
***A61P 35/00*** (2006.01) ***C12N 15/113*** (2010.01)

(86) International application number:
**PCT/JP2014/075683**

(87) International publication number:
**WO 2015/046451 (02.04.2015 Gazette 2015/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **27.09.2013 JP 2013202051**

(71) Applicant: **Aqua Therapeutics Co., Ltd.**
**Kobe-shi, Hyogo 650-0035 (JP)**

(72) Inventors:
- **IZUHARA Kenji**
**Saga-shi**
**Saga 840-8502 (JP)**
- **ARIMA Kazuhiko**
**Saga-shi**
**Saga 840-8502 (JP)**
- **SUZUKI Shoichi**
**Saga-shi**
**Saga 840-8502 (JP)**
- **OHTA Shoichiro**
**Saga-shi**
**Saga 840-8502 (JP)**
- **YOSHIKAWA Kazunori**
**Kobe-shi**
**Hyogo 650-0035 (JP)**
- **TAKAO Kazumasa**
**Kobe-shi**
**Hyogo 650-0035 (JP)**
- **SHIMAHARA Akiko**
**Kobe-shi**
**Hyogo 650-0035 (JP)**

(74) Representative: **Tuxworth, Pamela M.**
**J A Kemp**
**14 South Square**
**Gray's Inn**
**London WC1R 5JJ (GB)**

# (54) DRUG FOR DISEASE CAUSED BY EXPRESSION OF PERIOSTIN EXCEPT EYE DISEASE, AND USE THEREOF

(57) The present invention is intended to provide a novel molecule that inhibits the expression of the periostin gene that is effective in treatment of diseases caused by the expression of periostin except for eye diseases. A drug for a disease caused by the expression of periostin except for an eye disease includes, as an expression inhibitory sequence for the periostin gene, a nucleic acid molecule including a nucleotide that has a base sequence represented by any one of SEQ ID NOs: 1 to 19. The drug for disease according to the present invention can inhibit the expression of the periostin gene. Thus, it can be used for treatment of diseases (except for eye diseases) caused by the expression of the periostin gene, specifically skin diseases, respiratory diseases, gastrointestinal diseases, and the like.

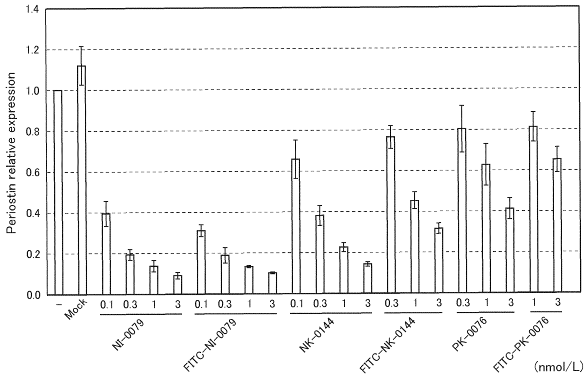


FIG. 5

EP 3 047 852 A1

[Sequence Listing] TF14029WO_2014.09.26_ST25.txt

SEQUENCE LISTING

<110> AQUA Therapeutics Co., Ltd.
SAGA UNIVERSITY

<120> Medicine for disease developed by the expression of periostin except ophthalmologic disease and use thereof

<130> TF14029WO

<150> JP2013-202051
<151> 2013-09-27

<160> 107

<170> PatentIn version 3.5

<210> 1
<211> 19
<212> RNA
<213> Artificial Sequence

<220>
<223> nucleic acid molecule

<400> 1
aaguauuucu uuuuggugc 19

<210> 2
<211> 19
<212> RNA
<213> Artificial Sequence

<220>
<223> nucleic acid molecule

<400> 2
gaaguauuuc uuuuuggug 19

<210> 3
<211> 19
<212> RNA
<213> Artificial Sequence

<220>
<223> nucleic acid molecule

<400> 3
aaucugguuc ccauggaug 19

<210> 4
<211> 19
<212> RNA
<213> Artificial Sequence

<220>
<223> nucleic acid molecule

<400> 4
uuucuaggac accucgugg 19

<210> 5
<211> 19
<212> RNA
<213> Artificial Sequence

<220>
<223> nucleic acid molecule

<400> 5
uuguuuggca gaaucagga 19

<210> 6
<211> 19
<212> RNA
<213> Artificial Sequence

<220>
<223> nucleic acid molecule

<400> 6
ucaauaacuu guuuggcag 19

<210> 7
<211> 19
<212> RNA
<213> Artificial Sequence

<220>
<223> nucleic acid molecule

<400> 7
agcucaauaa cuuguuugg 19

<210> 8
<211> 19
<212> RNA
<213> Artificial Sequence

<220>
<223> nucleic acid molecule

<400> 8
uugcuguuuu ccagccagc 19

<210> 9
<211> 19
<212> RNA
<213> Artificial Sequence

<220>
<223> nucleic acid molecule

<400> 9
ugguuugcug uuuuccagc 19

<210> 10

<211> 19
<212> RNA
<213> Artificial Sequence

<220>
<223> nucleic acid molecule

<400> 10
gaugccaagc cuaauuggg 19

<210> 11
<211> 19
<212> RNA
<213> Artificial Sequence

<220>
<223> nucleic acid molecule

<400> 11
ugauucgagc acaauuaac 19

<210> 12
<211> 19
<212> RNA
<213> Artificial Sequence

<220>
<223> nucleic acid molecule

<400> 12
uacuguuaua cugucaccg 19

<210> 13
<211> 19
<212> RNA
<213> Artificial Sequence

<220>
<223> nucleic acid molecule

<400> 13
uaagcacacg gucaaugac 19

<210> 14
<211> 19
<212> RNA
<213> Artificial Sequence

<220>
<223> nucleic acid molecule

<400> 14
uaauugggcu accaggucg 19

<210> 15
<211> 19
<212> RNA
<213> Artificial Sequence

<220>
<223> nucleic acid molecule

<400> 15
aucagaucgu ugauuuagg 19

<210> 16
<211> 19
<212> RNA
<213> Artificial Sequence

<220>
<223> nucleic acid molecule

<400> 16
uucaggauau uagugacuc 19

<210> 17
<211> 19
<212> RNA
<213> Artificial Sequence

<220>
<223> nucleic acid molecule

<400> 17
uccuuucuag gacaccucg 19

<210> 18
<211> 19
<212> RNA
<213> Artificial Sequence

<220>
<223> nucleic acid molecule

<400> 18
auccuuucua ggacaccuc 19

<210> 19
<211> 19
<212> RNA
<213> Artificial Sequence

<220>
<223> nucleic acid molecule

<400> 19
uuugcuguuu uccagccag 19

<210> 20
<211> 20
<212> RNA
<213> Artificial Sequence

<220>
<223> nucleic acid molecule

<220>
<221> misc_feature
<222> (20)..(20)
<223> n stands for any base and is 1 or more

<400> 20
aaguauuucu uuuuggugcn 20

<210> 21
<211> 20
<212> RNA
<213> Artificial Sequence

<220>
<223> nucleic acid molecule

<220>
<221> misc_feature
<222> (20)..(20)
<223> n stands for any base and is 1 or more

<400> 21
gaaguauuuc uuuuuggugn 20

<210> 22
<211> 20
<212> RNA
<213> Artificial Sequence

<220>
<223> nucleic acid molecule

<220>
<221> misc_feature
<222> (20)..(20)
<223> n stands for any base and is 1 or more

<400> 22
aaucugguuc ccauggaugn 20

<210> 23
<211> 20
<212> RNA
<213> Artificial Sequence

<220>
<223> nucleic acid molecule

<220>
<221> misc_feature
<222> (20)..(20)
<223> n stands for any base and is 1 or more

<400> 23
uuucuaggac accucguggn 20

<210> 24
<211> 20
<212> RNA
<213> Artificial Sequence

<220>
<223> nucleic acid molecule

<220>
<221> misc_feature
<222> (20)..(20)
<223> n stands for any base and is 1 or more

<400> 24
uuguuuggca gaaucaggan 20

<210> 25
<211> 20
<212> RNA
<213> Artificial Sequence

<220>
<223> nucleic acid molecule

<220>
<221> misc_feature
<222> (20)..(20)
<223> n stands for any base and is 1 or more

<400> 25
ucaauaacuu guuuggcagn 20

<210> 26
<211> 20
<212> RNA
<213> Artificial Sequence

<220>
<223> nucleic acid molecule

<220>
<221> misc_feature
<222> (20)..(20)
<223> n stands for any base and is 1 or more

<400> 26
agcucaauaa cuuguuuggn 20

<210> 27
<211> 20
<212> RNA
<213> Artificial Sequence

<220>
<223> nucleic acid molecule

<220>
<221> misc_feature
<222> (20)..(20)
<223> n stands for any base and is 1 or more

<400> 27
uugcuguuuu ccagccagcn 20

<210> 28
<211> 20
<212> RNA
<213> Artificial Sequence

<220>
<223> nucleic acid molecule

<220>
<221> misc_feature
<222> (20)..(20)
<223> n stands for any base and is 1 or more

<400> 28
ugguuugcug uuuuccagcn 20

<210> 29
<211> 20
<212> RNA
<213> Artificial Sequence

<220>
<223> nucleic acid molecule

<220>
<221> misc_feature
<222> (20)..(20)
<223> n stands for any base and is 1 or more

<400> 29
gaugccaagc cuaauugggn 20

<210> 30
<211> 20
<212> RNA
<213> Artificial Sequence

<220>
<223> nucleic acid molecule

<220>
<221> misc_feature
<222> (20)..(20)
<223> n stands for any base and is 1 or more

<400> 30
ugauucgagc acaauuaacn 20

<210> 31
<211> 20
<212> RNA
<213> Artificial Sequence

<220>
<223> nucleic acid molecule

<220>
<221> misc_feature
<222> (20)..(20)
<223> n stands for any base and is 1 or more

<400> 31
uacuguuaua cugucaccgn 20

<210> 32
<211> 20
<212> RNA
<213> Artificial Sequence

<220>
<223> nucleic acid molecule

<220>
<221> misc_feature
<222> (20)..(20)
<223> n stands for any base and is 1 or more

<400> 32
uaagcacacg gucaaugacn 20

<210> 33
<211> 20
<212> RNA
<213> Artificial Sequence

<220>
<223> nucleic acid molecule

<220>
<221> misc_feature
<222> (20)..(20)
<223> n stands for any base and is 1 or more

<400> 33
uaauugggcu accaggucgn 20

<210> 34
<211> 20
<212> RNA
<213> Artificial Sequence

<220>
<223> nucleic acid molecule

<220>
<221> misc_feature
<222> (20)..(20)
<223> n stands for any base and is 1 or more

<400> 34
aucagaucgu ugauuuaggn 20

<210> 35
<211> 20
<212> RNA
<213> Artificial Sequence

<220>
<223> nucleic acid molecule

<220>
<221> misc_feature
<222> (20)..(20)
<223> n stands for any base and is 1 or more

<400> 35
uucaggauau uagugacucn 20

<210> 36
<211> 20
<212> RNA
<213> Artificial Sequence

<220>
<223> nucleic acid molecule

<220>
<221> misc_feature
<222> (20)..(20)
<223> n stands for any base and is 1 or more

<400> 36
uccuuucuag gacaccucgn 20

<210> 37
<211> 20
<212> RNA
<213> Artificial Sequence

<220>
<223> nucleic acid molecule

<220>
<221> misc_feature
<222> (20)..(20)
<223> n stands for any base and is 1 or more

<400> 37
auccuuucua ggacaccucn 20

<210> 38
<211> 20
<212> RNA
<213> Artificial Sequence

<220>
<223> nucleic acid molecule

<220>
<221> misc_feature
<222> (20)..(20)
<223> n stands for any base and is 1 or more

<400> 38
uuugcuguuu uccagccagn 20

<210> 39
<211> 19
<212> RNA
<213> Artificial Sequence

<220>
<223> nucleic acid molecule

<400> 39
gcaccaaaaa gaaauacuu 19

<210> 40
<211> 19
<212> RNA
<213> Artificial Sequence

<220>
<223> nucleic acid molecule

<400> 40
caccaaaaag aaauacuuc 19

<210> 41
<211> 19
<212> RNA
<213> Artificial Sequence

<220>
<223> nucleic acid molecule

<400> 41
cauccauggg aaccagauu 19

<210> 42
<211> 19
<212> RNA
<213> Artificial Sequence

<220>
<223> nucleic acid molecule

<400> 42
ccacgaggug uccuagaaa 19

<210> 43
<211> 19
<212> RNA
<213> Artificial Sequence

<220>
<223> nucleic acid molecule

<400> 43
uccugauucu gccaaacaa 19

<210> 44
<211> 19
<212> RNA
<213> Artificial Sequence

<220>
<223> nucleic acid molecule

<400> 44
cugccaaaca aguuauuga 19

<210> 45
<211> 19
<212> RNA
<213> Artificial Sequence

<220>
<223> nucleic acid molecule

<400> 45
ccaaacaagu uauugagcu 19

<210> 46
<211> 19
<212> RNA
<213> Artificial Sequence

<220>
<223> nucleic acid molecule

<400> 46
gcuggcugga aaacagcaa 19

<210> 47
<211> 19
<212> RNA
<213> Artificial Sequence

<220>
<223> nucleic acid molecule

<400> 47
gcuggaaaac agcaaacca 19

<210> 48
<211> 19
<212> RNA
<213> Artificial Sequence

<220>
<223> nucleic acid molecule

<400> 48
cccaauuagg cuuggcauc 19

<210> 49
<211> 19
<212> RNA
<213> Artificial Sequence

<220>
<223> nucleic acid molecule

<400> 49
guuaauugug cucgaauca 19

<210> 50
<211> 19
<212> RNA
<213> Artificial Sequence

<220>
<223> nucleic acid molecule

<400> 50
cggugacagu auaacagua 19

<210> 51
<211> 19
<212> RNA
<213> Artificial Sequence

<220>
<223> nucleic acid molecule

<400> 51
gucauugacc gugugcuua 19

<210> 52
<211> 19
<212> RNA
<213> Artificial Sequence

<220>
<223> nucleic acid molecule

<400> 52
cgaccuggua gcccaauua 19

<210> 53

<211> 19
<212> RNA
<213> Artificial Sequence

<220>
<223> nucleic acid molecule

<400> 53
ccuaaaucaa cgaucugau 19

<210> 54
<211> 19
<212> RNA
<213> Artificial Sequence

<220>
<223> nucleic acid molecule

<400> 54
gagucacuaa uauccugaa 19

<210> 55
<211> 19
<212> RNA
<213> Artificial Sequence

<220>
<223> nucleic acid molecule

<400> 55
cgaggugucc uagaaagga 19

<210> 56
<211> 19
<212> RNA
<213> Artificial Sequence

<220>
<223> nucleic acid molecule

<400> 56
gagguguccu agaaaggau 19

<210> 57
<211> 19
<212> RNA
<213> Artificial Sequence

<220>
<223> nucleic acid molecule

<400> 57
cuggcuggaa aacagcaaa 19

<210> 58
<211> 20
<212> RNA
<213> Artificial Sequence

<220>
<223> nucleic acid molecule

<220>
<221> misc_feature
<222> (20)..(20)
<223> n stands for any base and is 1 or more

<400> 58
gcaccaaaaa gaaauacuun 20

<210> 59
<211> 20
<212> RNA
<213> Artificial Sequence

<220>
<223> nucleic acid molecule

<220>
<221> misc_feature
<222> (20)..(20)
<223> n stands for any base and is 1 or more

<400> 59
caccaaaaag aaauacuucn 20

<210> 60
<211> 20
<212> RNA
<213> Artificial Sequence

<220>
<223> nucleic acid molecule

<220>
<221> misc_feature
<222> (20)..(20)
<223> n stands for any base and is 1 or more

<400> 60
cauccauggg aaccagauun 20

<210> 61
<211> 20
<212> RNA
<213> Artificial Sequence

<220>
<223> nucleic acid molecule

<220>
<221> misc_feature
<222> (20)..(20)
<223> n stands for any base and is 1 or more

<400> 61
ccacgaggug uccuagaaan 20

<210> 62
<211> 20
<212> RNA
<213> Artificial Sequence

<220>
<223> nucleic acid molecule

<220>
<221> misc_feature
<222> (20)..(20)
<223> n stands for any base and is 1 or more

<400> 62
uccugauucu gccaaacaan 20

<210> 63
<211> 20
<212> RNA
<213> Artificial Sequence

<220>
<223> nucleic acid molecule

<220>
<221> misc_feature
<222> (20)..(20)
<223> n stands for any base and is 1 or more

<400> 63
cugccaaaca aguuauugan 20

<210> 64
<211> 20
<212> RNA
<213> Artificial Sequence

<220>
<223> nucleic acid molecule

<220>
<221> misc_feature
<222> (20)..(20)
<223> n stands for any base and is 1 or more

<400> 64
ccaaacaagu uauugagcun 20

<210> 65
<211> 20
<212> RNA
<213> Artificial Sequence

<220>
<223> nucleic acid molecule

<220>
<221> misc_feature
<222> (20)..(20)
<223> n stands for any base and is 1 or more

<400> 65
gcuggcugga aaacagcaan 20

<210> 66
<211> 20
<212> RNA
<213> Artificial Sequence

<220>
<223> nucleic acid molecule

<220>
<221> misc_feature
<222> (20)..(20)
<223> n stands for any base and is 1 or more

<400> 66
gcuggaaaac agcaaaccan 20

<210> 67
<211> 20
<212> RNA
<213> Artificial Sequence

<220>
<223> nucleic acid molecule

<220>
<221> misc_feature
<222> (20)..(20)
<223> n stands for any base and is 1 or more

<400> 67
cccaauuagg cuuggcaucn 20

<210> 68
<211> 20
<212> RNA
<213> Artificial Sequence

<220>
<223> nucleic acid molecule

<220>
<221> misc_feature
<222> (20)..(20)
<223> n stands for any base and is 1 or more

<400> 68
guuaauugug cucgaaucan 20

<210> 69
<211> 20
<212> RNA
<213> Artificial Sequence

<220>
<223> nucleic acid molecule

<220>
<221> misc_feature
<222> (20)..(20)
<223> n stands for any base and is 1 or more

<400> 69
cggugacagu auaacaguan 20

<210> 70
<211> 20
<212> RNA
<213> Artificial Sequence

<220>
<223> nucleic acid molecule

<220>
<221> misc_feature
<222> (20)..(20)
<223> n stands for any base and is 1 or more

<400> 70
gucauugacc gugugcuuan 20

<210> 71
<211> 20
<212> RNA
<213> Artificial Sequence

<220>
<223> nucleic acid molecule

<220>
<221> misc_feature
<222> (20)..(20)
<223> n stands for any base and is 1 or more

<400> 71
cgaccuggua gcccaauuan 20

<210> 72
<211> 20
<212> RNA
<213> Artificial Sequence

<220>
<223>  nucleic acid molecule

<220>
<221>  misc_feature
<222>  (20)..(20)
<223>  n stands for any base and is 1 or more

<400>  72
ccuaaaucaa cgaucugaun                                                    20

<210>  73
<211>  20
<212>  RNA
<213>  Artificial Sequence

<220>
<223>  nucleic acid molecule

<220>
<221>  misc_feature
<222>  (20)..(20)
<223>  n stands for any base and is 1 or more

<400>  73
gagucacuaa uauccugaan                                                    20

<210>  74
<211>  20
<212>  RNA
<213>  Artificial Sequence

<220>
<223>  nucleic acid molecule

<220>
<221>  misc_feature
<222>  (20)..(20)
<223>  n stands for any base and is 1 or more

<400>  74
cgaggugucc uagaaaggan                                                    20

<210>  75
<211>  20
<212>  RNA
<213>  Artificial Sequence

<220>
<223>  nucleic acid molecule

<220>
<221>  misc_feature
<222>  (20)..(20)
<223>  n stands for any base and is 1 or more

<400> 75
gaggugucc u agaaaggaun 20

<210> 76
<211> 20
<212> RNA
<213> Artificial Sequence

<220>
<223> nucleic acid molecule

<220>
<221> misc_feature
<222> (20)..(20)
<223> n stands for any base and is 1 or more

<400> 76
cuggcuggaa aacagcaaan 20

<210> 77
<211> 21
<212> DNA
<213> Artificial Sequence

<220>
<223> nucleic acid molecule

<400> 77
uacuauucga cacgcggagt t 21

<210> 78
<211> 21
<212> DNA
<213> Artificial Sequence

<220>
<223> nucleic acid molecule

<400> 78
cuucgcgugu cgaauaguat t 21

<210> 79
<211> 20
<212> DNA
<213> Artificial Sequence

<220>
<223> primer

<400> 79
tgcccagcag ttttgcccat 20

<210> 80
<211> 20
<212> DNA
<213> Artificial Sequence

<220>
<223>  primer

<400>  80
cgttgctctc caaacctcta                                                    20


<210>  81
<211>  23
<212>  DNA
<213>  Artificial Sequence

<220>
<223>  primer

<400>  81
gccacggctg cttccagctc ctc                                                23


<210>  82
<211>  25
<212>  DNA
<213>  Artificial Sequence

<220>
<223>  primer

<400>  82
aggtctttgc ggatgtccac gtcac                                              25


<210>  83
<211>  62
<212>  RNA
<213>  Artificial Sequence

<220>
<223>  nucleic acid molecule


<220>
<221>  misc_feature
<222>  (26)..(30)
<223>  n is a, c, g, or u

<220>
<221>  misc_feature
<222>  (57)..(61)
<223>  n is a, c, g, or u

<400>  83
agcaccaaaa agaaauacuu uucccnnnnn cggaaaagua uuucuuuuug gugcuunnnn        60

ng                                                                       62


<210>  84
<211>  62
<212>  RNA
<213>  Artificial Sequence

<220>

<223> nucleic acid molecule

<400> 84
agcaccaaaa agaaauacuu uuccccacac cggaaaagua uuucuuuuug gugcuucuuc 60

gg 62

<210> 85
<211> 62
<212> RNA
<213> Artificial Sequence

<220>
<223> nucleic acid molecule

<220>
<221> misc_feature
<222> (26)..(30)
<223> n is a, c, g, or u

<220>
<221> misc_feature
<222> (57)..(61)
<223> n is a, c, g, or u

<400> 85
auccugauuc ugccaaacaa uucccnnnnn cggaauuguu uggcagaauc aggauunnnn 60

ng 62

<210> 86
<211> 62
<212> RNA
<213> Artificial Sequence

<220>
<223> nucleic acid molecule

<400> 86
auccugauuc ugccaaacaa uuccccacac cggaauuguu uggcagaauc aggauucuuc 60

gg 62

<210> 87
<211> 62
<212> RNA
<213> Artificial Sequence

<220>
<223> nucleic acid molecule

<220>
<221> misc_feature
<222> (26)..(30)
<223> n is a, c, g, or u

<220>
<221> misc_feature

<222> (57)..(61)
<223> n is a, c, g, or u

<400> 87
acugccaaac aaguuauuga uucccnnnnn cggaaucaau aacuuguuug gcaguunnnn 60

ng 62

<210> 88
<211> 62
<212> RNA
<213> Artificial Sequence

<220>
<223> nucleic acid molecule

<400> 88
acugccaaac aaguuauuga uuccccacac cggaaucaau aacuuguuug gcaguucuuc 60

gg 62

<210> 89
<211> 62
<212> RNA
<213> Artificial Sequence

<220>
<223> nucleic acid molecule

<220>
<221> misc_feature
<222> (26)..(30)
<223> n is a, c, g, or u

<220>
<221> misc_feature
<222> (57)..(61)
<223> n is a, c, g, or u

<400> 89
acggugacag uauaacagua aacccnnnnn cgguuuacug uuauacuguc accgucnnnn 60

ng 62

<210> 90
<211> 62
<212> RNA
<213> Artificial Sequence

<220>
<223> nucleic acid molecule

<400> 90
acggugacag uauaacagua aaccccacac cgguuuacug uuauacuguc accguccuuc 60

gg 62

<210> 91

<211> 62
<212> RNA
<213> Artificial Sequence

<220>
<223> nucleic acid molecule

<220>
<221> misc_feature
<222> (26)..(30)
<223> n is a, c, g, or u

<220>
<221> misc_feature
<222> (57)..(61)
<223> n is a, c, g, or u

<400> 91
ugucauugac cgugugcuua cacccnnnnn cgguguaagc acacggucaa ugacaunnnn 60

ng 62

<210> 92
<211> 62
<212> RNA
<213> Artificial Sequence

<220>
<223> nucleic acid molecule

<400> 92
ugucauugac cgugugcuua caccccacac cgguguaagc acacggucaa ugacaucuuc 60

gg 62

<210> 93
<211> 51
<212> RNA
<213> Artificial Sequence

<220>
<223> nucleic acid molecule

<400> 93
agcaccaaaa agaaauacuu uuccggaaaa guauuucuuu uuggugcuuc g 51

<210> 94
<211> 51
<212> RNA
<213> Artificial Sequence

<220>
<223> nucleic acid molecule

<400> 94
auccugauuc ugccaaacaa uuccggaauu guuuggcaga aucaggauuc g 51

<210> 95

<211> 51
<212> RNA
<213> Artificial Sequence

<220>
<223> nucleic acid molecule

<400> 95
acugccaaac aaguuauuga uuccggaauc aauaacuugu uuggcaguuc g 51

<210> 96
<211> 51
<212> RNA
<213> Artificial Sequence

<220>
<223> nucleic acid molecule

<400> 96
acggugacag uauaacagua aaccgguuua cuguuauacu gucaccgucc g 51

<210> 97
<211> 51
<212> RNA
<213> Artificial Sequence

<220>
<223> nucleic acid molecule

<400> 97
ugucauugac cgugugcuua caccggugua agcacacggu caaugacauc g 51

<210> 98
<211> 51
<212> RNA
<213> Artificial Sequence

<220>
<223> nucleic acid molecule

<400> 98
auacuauucg acacgcgaag uuccggaacu ucgcgugucg aauaguauuc g 51

<210> 99
<211> 18
<212> DNA
<213> Mus musculus

<400> 99
aagctgcggc aagacaag 18

<210> 100
<211> 21
<212> DNA
<213> Mus musculus

<400> 100
gggctgtgtc aggagatctt t 21

<210> 101
<211> 20
<212> DNA
<213> Mus musculus

<400> 101
tgcaccacca actgcttagc 20

<210> 102
<211> 21
<212> DNA
<213> Mus musculus

<400> 102
ggcatggact gtggtcatga g 21

<210> 103
<211> 62
<212> RNA
<213> Artificial Sequence

<220>
<223> nucleic acid molecule

<400> 103
auacuauucg acacgcgaag uuccccacac cggaacuucg cgugucgaau aguauucuuc 60

gg 62

<210> 104
<211> 24
<212> DNA
<213> Mus musculus

<400> 104
gtcgtaccac aggcattgtg atgg 24

<210> 105
<211> 22
<212> DNA
<213> Mus musculus

<400> 105
gcaatgcctg ggtacatggt gg 22

<210> 106
<211> 20
<212> DNA
<213> Mus musculus

<400> 106
gaacgaatca ttacaggtcc 20

<210> 107
<211> 20

<212> DNA
<213> Mus musculus

<400> 107
ggagacctct ttttgcaaga 20

SEQUENCE LISTING

```
<110>  AQUA Therapeutics Co., Ltd.

<120>  Drug for disease developed by expression of periostin
       except eye disease, and use thereof

<130>  N407361

<140>  14846851.5
<141>  2014-09-26

<150>  JP2013-202051
<151>  2013-09-27

<160>  107

<170>  PatentIn version 3.5

<210>  1
<211>  19
<212>  RNA
<213>  Artificial Sequence

<220>
<223>  nucleic acid molecule

<400>  1
aaguauuucu uuuuggugc                                                    19


<210>  2
<211>  19
<212>  RNA
<213>  Artificial Sequence

<220>
<223>  nucleic acid molecule

<400>  2
gaaguauuuc uuuuuggug                                                    19


<210>  3
<211>  19
<212>  RNA
<213>  Artificial Sequence

<220>
<223>  nucleic acid molecule

<400>  3
aaucugguuc ccauggaug                                                    19


<210>  4
<211>  19
<212>  RNA
<213>  Artificial Sequence

<220>
<223>  nucleic acid molecule
```

<400> 4
uuucuaggac accucgugg 19

<210> 5
<211> 19
<212> RNA
<213> Artificial Sequence

<220>
<223> nucleic acid molecule

<400> 5
uuguuuggca gaaucagga 19

<210> 6
<211> 19
<212> RNA
<213> Artificial Sequence

<220>
<223> nucleic acid molecule

<400> 6
ucaauaacuu guuuggcag 19

<210> 7
<211> 19
<212> RNA
<213> Artificial Sequence

<220>
<223> nucleic acid molecule

<400> 7
agcucaauaa cuuguuugg 19

<210> 8
<211> 19
<212> RNA
<213> Artificial Sequence

<220>
<223> nucleic acid molecule

<400> 8
uugcuguuuu ccagccagc 19

<210> 9
<211> 19
<212> RNA
<213> Artificial Sequence

<220>
<223> nucleic acid molecule

<400> 9
ugguuugcug uuuuccagc 19

<210> 10
<211> 19
<212> RNA
<213> Artificial Sequence

<220>
<223> nucleic acid molecule

<400> 10
gaugccaagc cuaauuggg 19

<210> 11
<211> 19
<212> RNA
<213> Artificial Sequence

<220>
<223> nucleic acid molecule

<400> 11
ugauucgagc acaauuaac 19

<210> 12
<211> 19
<212> RNA
<213> Artificial Sequence

<220>
<223> nucleic acid molecule

<400> 12
uacuguuaua cugucaccg 19

<210> 13
<211> 19
<212> RNA
<213> Artificial Sequence

<220>
<223> nucleic acid molecule

<400> 13
uaagcacacg gucaaugac 19

<210> 14
<211> 19
<212> RNA
<213> Artificial Sequence

<220>
<223> nucleic acid molecule

<400> 14
uaauugggcu accaggucg 19

<210> 15
<211> 19

```
<212>  RNA
<213>  Artificial Sequence

<220>
<223>  nucleic acid molecule

<400>  15
aucagaucgu ugauuuagg                                                      19


<210>  16
<211>  19
<212>  RNA
<213>  Artificial Sequence

<220>
<223>  nucleic acid molecule

<400>  16
uucaggauau uagugacuc                                                      19


<210>  17
<211>  19
<212>  RNA
<213>  Artificial Sequence

<220>
<223>  nucleic acid molecule

<400>  17
uccuuucuag gacaccucg                                                      19


<210>  18
<211>  19
<212>  RNA
<213>  Artificial Sequence

<220>
<223>  nucleic acid molecule

<400>  18
auccuuucua ggacaccuc                                                      19


<210>  19
<211>  19
<212>  RNA
<213>  Artificial Sequence

<220>
<223>  nucleic acid molecule

<400>  19
uuugcuguuu uccagccag                                                      19


<210>  20
<211>  20
<212>  RNA
<213>  Artificial Sequence
```

<220>
<223> nucleic acid molecule

<220>
<221> misc_feature
<222> (20)..(20)
<223> n stands for any base and is 1 or more

<400> 20
aaguauuucu uuuuggugcn 20

<210> 21
<211> 20
<212> RNA
<213> Artificial Sequence

<220>
<223> nucleic acid molecule

<220>
<221> misc_feature
<222> (20)..(20)
<223> n stands for any base and is 1 or more

<400> 21
gaaguauuuc uuuuuggugn 20

<210> 22
<211> 20
<212> RNA
<213> Artificial Sequence

<220>
<223> nucleic acid molecule

<220>
<221> misc_feature
<222> (20)..(20)
<223> n stands for any base and is 1 or more

<400> 22
aaucugguuc ccauggaugn 20

<210> 23
<211> 20
<212> RNA
<213> Artificial Sequence

<220>
<223> nucleic acid molecule

<220>
<221> misc_feature
<222> (20)..(20)
<223> n stands for any base and is 1 or more

<400>  23
uuucuaggac accucguggn                                                     20

<210>  24
<211>  20
<212>  RNA
<213>  Artificial Sequence

<220>
<223>  nucleic acid molecule

<220>
<221>  misc_feature
<222>  (20)..(20)
<223>  n stands for any base and is 1 or more

<400>  24
uuguuuggca gaaucaggan                                                     20

<210>  25
<211>  20
<212>  RNA
<213>  Artificial Sequence

<220>
<223>  nucleic acid molecule

<220>
<221>  misc_feature
<222>  (20)..(20)
<223>  n stands for any base and is 1 or more

<400>  25
ucaauaacuu guuuggcagn                                                     20

<210>  26
<211>  20
<212>  RNA
<213>  Artificial Sequence

<220>
<223>  nucleic acid molecule

<220>
<221>  misc_feature
<222>  (20)..(20)
<223>  n stands for any base and is 1 or more

<400>  26
agcucaauaa cuuguuuggn                                                     20

<210>  27
<211>  20
<212>  RNA
<213>  Artificial Sequence

<220>
<223> nucleic acid molecule

<220>
<221> misc_feature
<222> (20)..(20)
<223> n stands for any base and is 1 or more

<400> 27
uugcuguuuu ccagccagcn 20

<210> 28
<211> 20
<212> RNA
<213> Artificial Sequence

<220>
<223> nucleic acid molecule

<220>
<221> misc_feature
<222> (20)..(20)
<223> n stands for any base and is 1 or more

<400> 28
ugguuugcug uuuuccagcn 20

<210> 29
<211> 20
<212> RNA
<213> Artificial Sequence

<220>
<223> nucleic acid molecule

<220>
<221> misc_feature
<222> (20)..(20)
<223> n stands for any base and is 1 or more

<400> 29
gaugccaagc cuaauugggn 20

<210> 30
<211> 20
<212> RNA
<213> Artificial Sequence

<220>
<223> nucleic acid molecule

<220>
<221> misc_feature
<222> (20)..(20)
<223> n stands for any base and is 1 or more

<400> 30
ugauucgagc acaauuaacn 20

<210> 31
<211> 20
<212> RNA
<213> Artificial Sequence

<220>
<223> nucleic acid molecule

<220>
<221> misc_feature
<222> (20)..(20)
<223> n stands for any base and is 1 or more

<400> 31
uacuguuaua cugucaccgn 20

<210> 32
<211> 20
<212> RNA
<213> Artificial Sequence

<220>
<223> nucleic acid molecule

<220>
<221> misc_feature
<222> (20)..(20)
<223> n stands for any base and is 1 or more

<400> 32
uaagcacacg gucaaugacn 20

<210> 33
<211> 20
<212> RNA
<213> Artificial Sequence

<220>
<223> nucleic acid molecule

<220>
<221> misc_feature
<222> (20)..(20)
<223> n stands for any base and is 1 or more

<400> 33
uaauugggcu accaggucgn 20

<210> 34
<211> 20
<212> RNA
<213> Artificial Sequence

<220>
<223>  nucleic acid molecule

<220>
<221>  misc_feature
<222>  (20)..(20)
<223>  n stands for any base and is 1 or more

<400>  34
aucagaucgu ugauuuaggn    20

<210>  35
<211>  20
<212>  RNA
<213>  Artificial Sequence

<220>
<223>  nucleic acid molecule

<220>
<221>  misc_feature
<222>  (20)..(20)
<223>  n stands for any base and is 1 or more

<400>  35
uucaggauau uagugacucn    20

<210>  36
<211>  20
<212>  RNA
<213>  Artificial Sequence

<220>
<223>  nucleic acid molecule

<220>
<221>  misc_feature
<222>  (20)..(20)
<223>  n stands for any base and is 1 or more

<400>  36
uccuuucuag gacaccucgn    20

<210>  37
<211>  20
<212>  RNA
<213>  Artificial Sequence

<220>
<223>  nucleic acid molecule

<220>
<221>  misc_feature
<222>  (20)..(20)
<223>  n stands for any base and is 1 or more

<400> 37
auccuuucua ggacaccucn 20

<210> 38
<211> 20
<212> RNA
<213> Artificial Sequence

<220>
<223> nucleic acid molecule

<220>
<221> misc_feature
<222> (20)..(20)
<223> n stands for any base and is 1 or more

<400> 38
uuugcuguuu uccagccagn 20

<210> 39
<211> 19
<212> RNA
<213> Artificial Sequence

<220>
<223> nucleic acid molecule

<400> 39
gcaccaaaaa gaaauacuu 19

<210> 40
<211> 19
<212> RNA
<213> Artificial Sequence

<220>
<223> nucleic acid molecule

<400> 40
caccaaaaag aaauacuuc 19

<210> 41
<211> 19
<212> RNA
<213> Artificial Sequence

<220>
<223> nucleic acid molecule

<400> 41
cauccauggg aaccagauu 19

<210> 42
<211> 19
<212> RNA
<213> Artificial Sequence

<220>
<223> nucleic acid molecule

<400> 42
ccacgaggug uccuagaaa 19

<210> 43
<211> 19
<212> RNA
<213> Artificial Sequence

<220>
<223> nucleic acid molecule

<400> 43
uccugauucu gccaaacaa 19

<210> 44
<211> 19
<212> RNA
<213> Artificial Sequence

<220>
<223> nucleic acid molecule

<400> 44
cugccaaaca aguuauuga 19

<210> 45
<211> 19
<212> RNA
<213> Artificial Sequence

<220>
<223> nucleic acid molecule

<400> 45
ccaaacaagu uauugagcu 19

<210> 46
<211> 19
<212> RNA
<213> Artificial Sequence

<220>
<223> nucleic acid molecule

<400> 46
gcuggcugga aaacagcaa 19

<210> 47
<211> 19
<212> RNA
<213> Artificial Sequence

<220>
<223> nucleic acid molecule

<400> 47
gcuggaaaac agcaaacca 19

<210> 48
<211> 19
<212> RNA
<213> Artificial Sequence

<220>
<223> nucleic acid molecule

<400> 48
cccaauuagg cuuggcauc 19

<210> 49
<211> 19
<212> RNA
<213> Artificial Sequence

<220>
<223> nucleic acid molecule

<400> 49
guuaauugug cucgaauca 19

<210> 50
<211> 19
<212> RNA
<213> Artificial Sequence

<220>
<223> nucleic acid molecule

<400> 50
cggugacagu auaacagua 19

<210> 51
<211> 19
<212> RNA
<213> Artificial Sequence

<220>
<223> nucleic acid molecule

<400> 51
gucauugacc gugugcuua 19

<210> 52
<211> 19
<212> RNA
<213> Artificial Sequence

<220>
<223> nucleic acid molecule

<400> 52
cgaccuggua gcccaauua 19

<210> 53
<211> 19
<212> RNA
<213> Artificial Sequence

<220>
<223> nucleic acid molecule

<400> 53
ccuaaaucaa cgaucugau 19

<210> 54
<211> 19
<212> RNA
<213> Artificial Sequence

<220>
<223> nucleic acid molecule

<400> 54
gagucacuaa uauccugaa 19

<210> 55
<211> 19
<212> RNA
<213> Artificial Sequence

<220>
<223> nucleic acid molecule

<400> 55
cgaggugucc uagaaagga 19

<210> 56
<211> 19
<212> RNA
<213> Artificial Sequence

<220>
<223> nucleic acid molecule

<400> 56
gagguguccu agaaaggau 19

<210> 57
<211> 19
<212> RNA
<213> Artificial Sequence

<220>
<223> nucleic acid molecule

<400> 57
cuggcuggaa aacagcaaa 19

<210> 58
<211> 20

<212> RNA
<213> Artificial Sequence

<220>
<223> nucleic acid molecule

<220>
<221> misc_feature
<222> (20)..(20)
<223> n stands for any base and is 1 or more

<400> 58
gcaccaaaaa gaaauacuun 20

<210> 59
<211> 20
<212> RNA
<213> Artificial Sequence

<220>
<223> nucleic acid molecule

<220>
<221> misc_feature
<222> (20)..(20)
<223> n stands for any base and is 1 or more

<400> 59
caccaaaaag aaauacuucn 20

<210> 60
<211> 20
<212> RNA
<213> Artificial Sequence

<220>
<223> nucleic acid molecule

<220>
<221> misc_feature
<222> (20)..(20)
<223> n stands for any base and is 1 or more

<400> 60
cauccauggg aaccagauun 20

<210> 61
<211> 20
<212> RNA
<213> Artificial Sequence

<220>
<223> nucleic acid molecule

<220>
<221> misc_feature

<222> (20)..(20)
<223> n stands for any base and is 1 or more

<400> 61
ccacgaggug uccuagaaan 20

<210> 62
<211> 20
<212> RNA
<213> Artificial Sequence

<220>
<223> nucleic acid molecule

<220>
<221> misc_feature
<222> (20)..(20)
<223> n stands for any base and is 1 or more

<400> 62
uccugauucu gccaaacaan 20

<210> 63
<211> 20
<212> RNA
<213> Artificial Sequence

<220>
<223> nucleic acid molecule

<220>
<221> misc_feature
<222> (20)..(20)
<223> n stands for any base and is 1 or more

<400> 63
cugccaaaca aguuauugan 20

<210> 64
<211> 20
<212> RNA
<213> Artificial Sequence

<220>
<223> nucleic acid molecule

<220>
<221> misc_feature
<222> (20)..(20)
<223> n stands for any base and is 1 or more

<400> 64
ccaaacaagu uauugagcun 20

<210> 65
<211> 20

<212> RNA
<213> Artificial Sequence

<220>
<223> nucleic acid molecule

<220>
<221> misc_feature
<222> (20)..(20)
<223> n stands for any base and is 1 or more

<400> 65
gcuggcugga aaacagcaan 20

<210> 66
<211> 20
<212> RNA
<213> Artificial Sequence

<220>
<223> nucleic acid molecule

<220>
<221> misc_feature
<222> (20)..(20)
<223> n stands for any base and is 1 or more

<400> 66
gcuggaaaac agcaaaccan 20

<210> 67
<211> 20
<212> RNA
<213> Artificial Sequence

<220>
<223> nucleic acid molecule

<220>
<221> misc_feature
<222> (20)..(20)
<223> n stands for any base and is 1 or more

<400> 67
cccaauuagg cuuggcaucn 20

<210> 68
<211> 20
<212> RNA
<213> Artificial Sequence

<220>
<223> nucleic acid molecule

<220>
<221> misc_feature

```
<222>  (20)..(20)
<223>  n stands for any base and is 1 or more

<400>  68
guuaauugug cucgaaucan                                                   20


<210>  69
<211>  20
<212>  RNA
<213>  Artificial Sequence

<220>
<223>  nucleic acid molecule


<220>
<221>  misc_feature
<222>  (20)..(20)
<223>  n stands for any base and is 1 or more

<400>  69
cggugacagu auaacaguan                                                   20


<210>  70
<211>  20
<212>  RNA
<213>  Artificial Sequence

<220>
<223>  nucleic acid molecule


<220>
<221>  misc_feature
<222>  (20)..(20)
<223>  n stands for any base and is 1 or more

<400>  70
gucauugacc gugugcuuan                                                   20


<210>  71
<211>  20
<212>  RNA
<213>  Artificial Sequence

<220>
<223>  nucleic acid molecule


<220>
<221>  misc_feature
<222>  (20)..(20)
<223>  n stands for any base and is 1 or more

<400>  71
cgaccuggua gcccaauuan                                                   20


<210>  72
<211>  20
```

<212> RNA
<213> Artificial Sequence

<220>
<223> nucleic acid molecule

<220>
<221> misc_feature
<222> (20)..(20)
<223> n stands for any base and is 1 or more

<400> 72
ccuaaaucaa cgaucugaun 20

<210> 73
<211> 20
<212> RNA
<213> Artificial Sequence

<220>
<223> nucleic acid molecule

<220>
<221> misc_feature
<222> (20)..(20)
<223> n stands for any base and is 1 or more

<400> 73
gagucacuaa uauccugaan 20

<210> 74
<211> 20
<212> RNA
<213> Artificial Sequence

<220>
<223> nucleic acid molecule

<220>
<221> misc_feature
<222> (20)..(20)
<223> n stands for any base and is 1 or more

<400> 74
cgaggugucc uagaaaggan 20

<210> 75
<211> 20
<212> RNA
<213> Artificial Sequence

<220>
<223> nucleic acid molecule

<220>
<221> misc_feature

<222> (20)..(20)
<223> n stands for any base and is 1 or more

<400> 75
gagguguccu agaaaggaun 20

<210> 76
<211> 20
<212> RNA
<213> Artificial Sequence

<220>
<223> nucleic acid molecule

<220>
<221> misc_feature
<222> (20)..(20)
<223> n stands for any base and is 1 or more

<400> 76
cuggcuggaa aacagcaaan 20

<210> 77
<211> 21
<212> DNA
<213> Artificial Sequence

<220>
<223> nucleic acid molecule

<400> 77
uacuauucga cacgcggagt t 21

<210> 78
<211> 21
<212> DNA
<213> Artificial Sequence

<220>
<223> nucleic acid molecule

<400> 78
cuucgcgugu cgaauaguat t 21

<210> 79
<211> 20
<212> DNA
<213> Artificial Sequence

<220>
<223> primer

<400> 79
tgcccagcag ttttgcccat 20

<210> 80
<211> 20

<212> DNA
<213> Artificial Sequence

<220>
<223> primer

<400> 80
cgttgctctc caaacctcta 20

<210> 81
<211> 23
<212> DNA
<213> Artificial Sequence

<220>
<223> primer

<400> 81
gccacggctg cttccagctc ctc 23

<210> 82
<211> 25
<212> DNA
<213> Artificial Sequence

<220>
<223> primer

<400> 82
aggtctttgc ggatgtccac gtcac 25

<210> 83
<211> 62
<212> RNA
<213> Artificial Sequence

<220>
<223> nucleic acid molecule

<220>
<221> misc_feature
<222> (26)..(30)
<223> n is a, c, g, or u

<220>
<221> misc_feature
<222> (57)..(61)
<223> n is a, c, g, or u

<400> 83
agcaccaaaa agaaauacuu uucccnnnnn cggaaaagua uuucuuuuug gugcuunnnn 60

ng 62

<210> 84
<211> 62
<212> RNA
<213> Artificial Sequence

<220>
<223> nucleic acid molecule

<400> 84
agcaccaaaa agaaauacuu uuccccacac cggaaaagua uuucuuuuug gugcuucuuc 60

gg 62

<210> 85
<211> 62
<212> RNA
<213> Artificial Sequence

<220>
<223> nucleic acid molecule

<220>
<221> misc_feature
<222> (26)..(30)
<223> n is a, c, g, or u

<220>
<221> misc_feature
<222> (57)..(61)
<223> n is a, c, g, or u

<400> 85
auccugauuc ugccaaacaa uucccnnnnn cggaauuguu uggcagaauc aggauunnnn 60

ng 62

<210> 86
<211> 62
<212> RNA
<213> Artificial Sequence

<220>
<223> nucleic acid molecule

<400> 86
auccugauuc ugccaaacaa uuccccacac cggaauuguu uggcagaauc aggauucuuc 60

gg 62

<210> 87
<211> 62
<212> RNA
<213> Artificial Sequence

<220>
<223> nucleic acid molecule

<220>
<221> misc_feature
<222> (26)..(30)
<223> n is a, c, g, or u

<220>
<221> misc_feature
<222> (57)..(61)
<223> n is a, c, g, or u

<400> 87
acugccaaac aaguuauuga uucccnnnnn cggaaucaau aacuuguuug gcaguunnnn 60

ng 62

<210> 88
<211> 62
<212> RNA
<213> Artificial Sequence

<220>
<223> nucleic acid molecule

<400> 88
acugccaaac aaguuauuga uuccccacac cggaaucaau aacuuguuug gcaguucuuc 60

gg 62

<210> 89
<211> 62
<212> RNA
<213> Artificial Sequence

<220>
<223> nucleic acid molecule

<220>
<221> misc_feature
<222> (26)..(30)
<223> n is a, c, g, or u

<220>
<221> misc_feature
<222> (57)..(61)
<223> n is a, c, g, or u

<400> 89
acggugacag uauaacagua aacccnnnnn cgguuuacug uuauacuguc accgucnnnn 60

ng 62

<210> 90
<211> 62
<212> RNA
<213> Artificial Sequence

<220>
<223> nucleic acid molecule

<400> 90
acggugacag uauaacagua aaccccacac cgguuuacug uuauacuguc accguccuuc 60

gg 62

<210> 91
<211> 62
<212> RNA
<213> Artificial Sequence

<220>
<223> nucleic acid molecule

<220>
<221> misc_feature
<222> (26)..(30)
<223> n is a, c, g, or u

<220>
<221> misc_feature
<222> (57)..(61)
<223> n is a, c, g, or u

<400> 91
ugucauugac cgugugcuua cacccnnnnn cgguguaagc acacggucaa ugacaunnnn 60

ng 62

<210> 92
<211> 62
<212> RNA
<213> Artificial Sequence

<220>
<223> nucleic acid molecule

<400> 92
ugucauugac cgugugcuua caccccacac cgguguaagc acacggucaa ugacaucuuc 60

gg 62

<210> 93
<211> 51
<212> RNA
<213> Artificial Sequence

<220>
<223> nucleic acid molecule

<400> 93
agcaccaaaa agaaauacuu uuccggaaaa guauuucuuu uuggugcuuc g 51

<210> 94
<211> 51
<212> RNA
<213> Artificial Sequence

<220>
<223> nucleic acid molecule

<400> 94
auccugauuc ugccaaacaa uuccggaauu guuuggcaga aucaggauuc g 51

<210> 95
<211> 51
<212> RNA
<213> Artificial Sequence

<220>
<223> nucleic acid molecule

<400> 95
acugccaaac aaguuauuga uuccggaauc aauaacuugu uuggcaguuc g 51

<210> 96
<211> 51
<212> RNA
<213> Artificial Sequence

<220>
<223> nucleic acid molecule

<400> 96
acggugacag uauaacagua aaccgguuua cuguuauacu gucaccgucc g 51

<210> 97
<211> 51
<212> RNA
<213> Artificial Sequence

<220>
<223> nucleic acid molecule

<400> 97
ugucauugac cgugugcuua caccggugua agcacacggu caaugacauc g 51

<210> 98
<211> 51
<212> RNA
<213> Artificial Sequence

<220>
<223> nucleic acid molecule

<400> 98
auacuauucg acacgcgaag uuccggaacu ucgcgugucg aauaguauuc g 51

<210> 99
<211> 18
<212> DNA
<213> Mus musculus

<400> 99
aagctgcggc aagacaag 18

<210> 100
<211> 21
<212> DNA
<213> Mus musculus

<400>  100
gggctgtgtc aggagatctt t                                                21

<210>  101
<211>  20
<212>  DNA
<213>  Mus musculus

<400>  101
tgcaccacca actgcttagc                                                  20

<210>  102
<211>  21
<212>  DNA
<213>  Mus musculus

<400>  102
ggcatggact gtggtcatga g                                                21

<210>  103
<211>  62
<212>  RNA
<213>  Artificial Sequence

<220>
<223>  nucleic acid molecule

<400>  103
auacuauucg acacgcgaag uuccccacac cggaacuucg cgugucgaau aguauucuuc      60

gg                                                                     62

<210>  104
<211>  24
<212>  DNA
<213>  Mus musculus

<400>  104
gtcgtaccac aggcattgtg atgg                                             24

<210>  105
<211>  22
<212>  DNA
<213>  Mus musculus

<400>  105
gcaatgcctg ggtacatggt gg                                               22

<210>  106
<211>  20
<212>  DNA
<213>  Mus musculus

<400>  106
gaacgaatca ttacaggtcc                                                  20

```
<210>  107
<211>  20
<212>  DNA
<213>  Mus musculus

<400>  107
ggagacctct ttttgcaaga                                                   20
```

## Description

Technical Field

The present invention relates to a drug for a disease caused by the expression of periostin except for an eye disease, and the use thereof.

Background Art

Nowadays, the number of atopic dermatitis patients is increasing. The atopic dermatitis is chronic eczema caused by the physiological dysfunction of skin, the sensitization due to nonspecific irritation and specific allergens, and the like. The atopic dermatitis is accompanied by chronic itching and this causes a problem affecting the QOL of patients.

Steroids and the like are used for the treatment of atopic dermatitis. However, the long term use of steroids causes side effects such as skin atrophy or the like. Thus, provision of a new candidate substance that can be a therapeutic drug for skin diseases such as atopic dermatitis and the like is desired. Such a problem is caused also in diseases except for dermatitides such as atopic dermatitis and the like.

It has been reported that the expression of the periostin gene is involved in skin diseases such as atopic dermatitis, wounds, scleroderma, keloids, hypertrophic scars, and melanoma; respiratory diseases such as bronchial asthma, idiopathic interstitial pneumonia, and non-idiopathic interstitial pneumonia; and gastrointestinal diseases such as cholangiocarcinoma. (Patent Documents 1 to 4, Non-Patent Documents 1 and 2).

Citation List

Patent Document(s)

Patent Document 1: Japanese Patent No. 4155561
Patent Document 2: WO 2009/148184
Patent Document 3: JP 2010-145294 A
Patent Document 4: WO 2011/068176

Non-Patent Document(s)

Non-Patent Document 1: Masutaka FURUE et al., "atopic dermatitis practice guideline", The Japanese Journal of Dermatology, 2009, vol. 119, no. 8, pp. 1515 to 1534
Non-Patent Document 2: Miho Masuoka et al., "Periostin promotes chronic allergic inflammation in response to Th2 cytokines.", The Journal of Clinical Investigation, July 2 2012, vol. 122, no. 7, pp. 2590 to 2600

Brief Summary of the Invention

Problem to be Solved by the Invention

Hence, the present invention is intended to provide a drug for a disease caused by the expression of periostin except for an eye disease, and the use thereof such as for a method of treating a disease.

Means for Solving Problem

In order to achieve the above objective, the present invention provides a drug for a disease caused by expression of periostin except for an eye disease, including an expression inhibitory nucleic acid molecule for the periostin gene, the expression inhibitory nucleic acid molecule including the following nucleotide (as1), (as2), or (as3):

(as1) a nucleotide that has a base sequence represented by any one of SEQ ID NOs: 1 to 19;
(as2) a nucleotide that has a base sequence obtained by deletion, substitution, and/or addition of one to several bases in the base sequence of the nucleotide (as1) and has a function of inhibiting expression of the periostin gene; and

(as3) a nucleotide that has a base sequence with an identity of at least 90% to the base sequence of the nucleotide (as1) and has a function of inhibiting expression of the periostin gene.

The present invention also provides a method of treating a disease caused by expression of periostin except for an eye disease, the method including the step of administering the drug for a disease caused by expression of periostin except for an eye disease according to the present invention to a patient.

Effects of the Invention

According to the expression inhibitory nucleic acid molecule of the present invention, it is possible to inhibit the expression of the periostin gene. Thus, the present invention is effective for treatment of the diseases caused by the expression of the periostin gene. Specifically, the present invention is effective for treatment of skin diseases such as atopic dermatitis, wounds, psoriasis, scleroderma, keloids, hypertrophic scars, and melanoma; respiratory diseases such as bronchial asthma, idiopathic interstitial pneumonia, and non-idiopathic interstitial pneumonia; and gastrointestinal diseases such as cholangiocarcinoma.

Brief Description of Drawings

[FIG. 1] FIG. 1 shows schematic views of an example of the nucleic acid molecule according to the present invention.
[FIG. 2] FIG. 2 shows schematic views of another example of the nucleic acid molecule according to the present invention.
[FIG. 3] FIG. 3 shows schematic views of still another example of the nucleic acid molecule according to the present invention.
[FIG. 4] FIG. 4 shows schematic views of yet another example of the nucleic acid molecule according to the present invention.
[FIG. 5] FIG. 5 is a graph showing the relative value of the periostin gene expression level in vitro in Example 1 of the present invention.
[FIG. 6] FIG. 6 is a graph showing the relative value of the periostin gene expression level in vitro in Example 2 of the present invention.
[FIG. 7] FIG. 7 is a graph showing the relative value of the periostin gene expression level in vitro in Example 3 of the present invention.
[FIG. 8] FIG. 8 is a graph showing the relative value of the periostin gene expression level in vitro in Example 4 of the present invention.
[FIG. 9] FIG. 9 is a graph showing the degree of the scar formation on the rabbit auricle in Example 5 of the present invention.

Mode for Carrying out the Invention

In the drug for disease according to the present invention, for example, the expression inhibitory nucleic acid molecule is a single-stranded nucleic acid molecule, the single-stranded nucleic acid molecule includes, in sequence from a 5' side to a 3' side: a 5' side region (Xc); an inner region (Z); and a 3' side region (Yc), the inner region (Z) is composed of an inner 5' side region (X) and an inner 3' side region (Y) that are linked to each other, the 5' side region (Xc) is complementary to the inner 5' side region (X), the 3' side region (Yc) is complementary to the inner 3' side region (Y), and the inner region (Z) includes the expression inhibitory sequence.
The drug for disease according to the present invention, for example, further includes: a linker region (Lx) between the 5' side region (Xc) and the inner 5' side region (X); and a linker region (Ly) between the 3' side region (Yc) and the inner 3' side region (Y), wherein the 5' side region (Xc) and the inner 5' side region (X) are linked to each other via the linker region (Lx), and the 3' side region (Yc) and the inner 3' side region (Y) are linked to each other via the linker region (Ly).
In the drug for disease according to the present invention, for example, the linker regions (Lx) and (Ly) each include a nucleotide residue.
In the drug for disease according to the present invention, for example, the linker regions (Lx) and (Ly) each include a non-nucleotide residue.
In the drug for disease according to the present invention, for example, the non-nucleotide residue includes at least one of a pyrrolidine skeleton and a piperidine skeleton.
In the drug for disease according to the present invention, for example, the linker regions (Lx) and (Ly) are

each represented by the following formula (I):

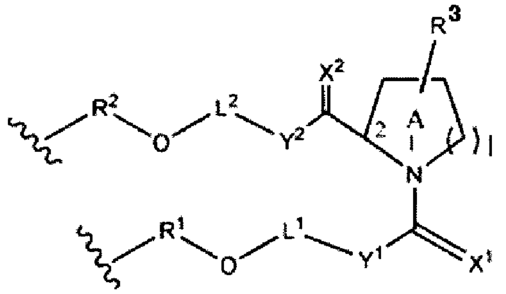

・・・ ( I )

where:

$X^1$ and $X^2$ are each independently $H_2$, O, S, or NH;
$Y^1$ and $Y^2$ are each independently a single bond, $CH_2$, NH, O, or S;
$R^3$ is a hydrogen atom or a substituent that is bound to C-3, C-4, C-5, or C-6 on a ring A;
$L^1$ is an alkylene chain composed of n atoms, and a hydrogen atom on an alkylene carbon atom may or may not be substituted with OH, $OR^a$, $NH_2$, $NHR^a$, $NR^aR^b$, SH, or $SR^a$, or
$L^1$ is a polyether chain obtained by substituting at least one carbon atom on the alkylene chain with an oxygen atom, provided that: when $Y^1$ is NH, O, or S, an atom bound to $Y^1$ in $L^1$ is carbon, an atom bound to $OR^1$ in $L^1$ is carbon, and oxygen atoms are not adjacent to each other;
$L^2$ is an alkylene chain composed of m atoms, and a hydrogen atom on an alkylene carbon atom may or may not be substituted with OH, $OR^c$, $NH_2$, $NHR^c$, $NR^cR^d$, SH, or $SR^c$, or
$L^2$ is a polyether chain obtained by substituting at least one carbon atom on the alkylene chain with an oxygen atom, provided that: when $Y^2$ is NH, O, or S, an atom bound to $Y^2$ in $L^2$ is carbon, an atom bound to $OR^2$ in $L^2$ is carbon, and oxygen atoms are not adjacent to each other;
$R^a$, $R^b$, $R^c$, and $R^d$ are each independently a substituent or a protecting group; l is 1 or 2;
m is an integer in the range from 0 to 30;
n is an integer in the range from 0 to 30;
on the ring A, one carbon atom other than C-2 may be substituted with nitrogen, oxygen, or sulfur,
the ring A may include carbon-carbon double bond or a carbon-nitrogen double bond; and
the regions (Xc) and (X) are each linked to the linker region (Lx) via $-OR^1-$ or $-OR^2-$, where $R^1$ and $R^2$ may or may not be present, and when they are present, $R^1$ and $R^2$ are each independently a nucleotide residue or the structure of the formula (I).

In the drug for disease according to the present invention, for example, the number of bases (Z) in the inner region (Z), the number of bases (X) in the inner 5' side region (X), the number of bases (Y) in the inner 3' side region (Y), the number of bases (Xc) in the 5' side region (Xc), and the number of bases (Yc) in the 3' side region (Yc) satisfy conditions of Expressions (1) and (2) below:

$$Z = X + Y \qquad \dots (1)$$

$$Z \geq Xc + Yc \qquad \dots (2).$$

In the drug for disease according to the present invention, for example, the difference between the number of bases (X) in the inner 5' side region (X) and the number of bases (Xc) in the 5' side region (Xc), and the difference between the number of bases (Y) in the inner 3' side region (Y) and the number of bases (Yc) in the 3' side region (Yc) satisfy the following conditions:

(a) Conditions of Expressions (11) and (12) are satisfied;

$$X - Xc = 1, 2, \text{ or } 3 \quad \dots (11)$$

$$Y - Yc = 0 \quad \dots (12)$$

(b) Conditions of Expressions (13) and (14) are satisfied;

$$X - Xc = 0 \quad \dots (13)$$

$$Y - Yc = 1, 2, \text{ or } 3 \quad \dots (14)$$

(c) Conditions of Expressions (15) and (16) are satisfied;

$$X - Xc = 1, 2, \text{ or } 3 \quad \dots (15)$$

$$Y - Yc = 1, 2, \text{ or } 3 \quad \dots (16)$$

(d) Conditions of Expressions (17) and (18) are satisfied;

$$X - Xc = 0 \quad \dots (17)$$

$$Y - Yc = 0 \quad \dots (18).$$

In the drug for disease according to the present invention, for example, the expression inhibitory sequence has a length of 18- to 32-mer.

In the drug for disease according to the present invention, for example, the sequence of the expression inhibitory nucleic acid molecule is at least one base sequence selected from the group consisting of SEQ ID NOs: 83 to 97.

In the drug for disease according to the present invention, for example, the expression inhibitory sequence further includes an overhang sequence, and the overhang sequence is added to the 3' end of the nucleotide.

In the drug for disease according to the present invention, for example, the expression inhibitory sequence is a nucleotide that has a base sequence represented by any one of SEQ ID NOs: 20 to 38 (where n is a positive integer) including the nucleotide (as1).

In the drug for disease according to the present invention, for example, the expression inhibitory sequence has a length of 18- to 32-mer.

The drug for disease according to the present invention, for example, further includes a complementary sequence that anneals to the expression inhibitory sequence, wherein the complementary sequence includes a nucleotide that is complementary to the nucleotide (as1), (as2), or (as3) in the expression inhibitory sequence.

In the drug for disease according to the present invention, for example, the nucleotide in the complementary sequence is the following nucleotide (s1), (s2), or (s3):

(s1) a nucleotide that has a base sequence complementary to the base sequence of the nucleotide (as1);
(s2) a nucleotide that has a base sequence complementary to the base sequence of the nucleotide (as2); and
(s3) a nucleotide that has a base sequence complementary to the base sequence of the nucleotide (as3).

In the drug for disease according to the present invention, for example, the nucleotide (s1) has a base sequence represented by any one of SEQ ID NOs: 39 to 57.

In the drug for disease according to the present invention, for example, the complementary sequence further includes an overhang sequence, and the overhang sequence is added to the 5' end of the nucleotide.

In the drug for disease according to the present invention, for example, the complementary sequence is a nucleotide that has a base sequence represented by any one of SEQ ID NOs: 58 to 76 (where n is a positive integer)

including the nucleotide (s1).

In the drug for disease according to the present invention, for example, the overhang sequence has a length of 1- to 3-mer.

In the drug for disease according to the present invention, for example, the expression inhibitory nucleic acid molecule is a double-stranded nucleic acid molecule composed of two single strands, and an antisense strand thereof includes the expression inhibitory sequence and a sense strand thereof includes the complementary sequence.

Regarding the drug for disease according to the present invention, for example, the disease caused by expression of periostin except for an eye disease is at least one selected from the group consisting of skin diseases, respiratory diseases, and gastrointestinal diseases.

Regarding the drug for disease according to the present invention, for example, the skin disease is at least one selected from the group consisting of atopic dermatitis, wounds, psoriasis, scleroderma, keloids, hypertrophic scars, and melanoma.

Regarding the drug for disease according to the present invention, for example, the respiratory disease is at least one selected from the group consisting of bronchial asthma, idiopathic interstitial pneumonia, and non-idiopathic interstitial pneumonia.

Regarding the drug for disease according to the present invention, for example, the gastrointestinal disease is cholangiocarcinoma.

Regarding the treatment method according to the present invention, the disease caused by expression of periostin except for an eye disease is at least one selected from the group consisting of skin diseases, respiratory diseases, and gastrointestinal diseases.

Regarding the treatment method according to the present invention, for example, the skin disease is at least one selected from the group consisting of atopic dermatitis, wounds, psoriasis, scleroderma, keloids, hypertrophic scars, and melanoma.

Regarding the treatment method according to the present invention, for example, the respiratory disease is at least one selected from the group consisting of bronchial asthma, idiopathic interstitial pneumonia, and non-idiopathic interstitial pneumonia.

Regarding the treatment method according to the present invention, for example, the gastrointestinal disease is cholangiocarcinoma.

Terms used in the present specification each have a meaning commonly used in the art, unless otherwise stated.

<Drug for Disease>

As described above, the drug for a disease caused by the expression of periostin except for an eye disease according to the present invention is characterized in that it includes an expression inhibitory nucleic acid molecule for the periostin gene, the expression inhibitory nucleic acid molecule including the following nucleotide (as1), (as2), or (as3):

(as1) a nucleotide that has a base sequence represented by any one of SEQ ID NOs: 1 to 19;
(as2) a nucleotide that has a base sequence obtained by deletion, substitution, and/or addition of one to several bases in the base sequence of the nucleotide (as1) and has a function of inhibiting expression of the periostin gene; and (as3) a nucleotide that has a base sequence with an identity of at least 90% to the base sequence of the nucleotide (as1) and has a function of inhibiting expression of the periostin gene.

The drug for disease according to the present invention is characterized in that it includes the expression inhibitory nucleic acid molecule, and other configurations are by no means limited. The drug for disease according to the present invention can also be referred to as a composition for disease or a therapeutic drug for disease, for example. Hereinafter, in the present invention, the expression inhibitory nucleic acid molecule is also referred to as a "nucleic acid molecule".

Hereinafter, the nucleotides (as1), (as2), and (as3) are collectively referred to as "as nucleotides", and they are referred to as "as1 nucleotide", "as2 nucleotide", and "as3 nucleotide", respectively.

In the present invention, there is no particular limitation on the inhibition of the expression of the periostin gene. For example, it may be inhibition of the transcription of the gene itself, or may be inhibition by degrading a transcription product of the gene. Furthermore, inhibition of the expression of the periostin gene may be, for example, inhibition of the expression of a periostin protein having its original function, and when a protein is expressed, the expressed protein may be such that the above-described function is inhibited or deleted, for example.

The expression inhibitory sequence may consist of the as nucleotide or may include the as nucleotide, for example.

The length of the expression inhibitory sequence is not particularly limited, and is, for example, 18- to 32-mer, preferably 19- to 30-mer, and more preferably 19-, 20-, or 21-mer. In the present invention, for example, the numerical

range regarding the number of bases discloses all the positive integers falling within that range. For example, the description "1 to 4 bases" discloses all of "1, 2, 3, and 4 bases" (the same applies hereinafter).

The sequences of the as1 nucleotide are shown below. The names shown below (the names indicated ahead of the sequence identification numbers) also can be used to refer to nucleotides having the base sequences represented by SEQ ID NOs: 1 to 19, expression inhibitory sequences consisting of the nucleotides, expression inhibitory sequences including the nucleotides, and nucleic acid molecules including the nucleotides.

NI-0079 (SEQ ID NO: 1)
5'-AAGUAUUUCUUUUUGGUGC-3'
NI-0080 (SEQ ID NO: 2)
5'-GAAGUAUUUCUUUUUGGUG-3'
NI-0081 (SEQ ID NO: 3)
5'-AAUCUGGUUCCCAUGGAUG-3'
NI-0082 (SEQ ID NO: 4)
5'-UUUCUAGGACACCUCGUGG-3'
NI-0083 (SEQ ID NO: 5)
5'-UUGUUUGGCAGAAUCAGGA-3'
NI-0084 (SEQ ID NO: 6)
5'-UCAAUAACUUGUUUGGCAG-3'
NI-0085 (SEQ ID NO: 7)
5'-AGCUCAAUAACUUGUUUGG-3'
NI-0086 (SEQ ID NO: 8)
5'-UUGCUGUUUUCCAGCCAGC-3'
NI-0087 (SEQ ID NO: 9)
5'-UGGUUUGCUGUUUUCCAGC-3'
NI-0088 (SEQ ID NO: 10)
5'-GAUGCCAAGCCUAAUUGGG-3'
NI-0091 (SEQ ID NO: 11)
5'-UGAUUCGAGCACAAUUAAC-3'
NI-0092 (SEQ ID NO: 12)
5'-UACUGUUAUACUGUCACCG-3'
NI-0093 (SEQ ID NO: 13)
5'-UAAGCACACGGUCAAUGAC-3'
NI-0094 (SEQ ID NO: 14)
5'-UAAUUGGGCUACCAGGUCG-3'
NI-0095 (SEQ ID NO: 15)
5'-AUCAGAUCGUUGAUUUAGG-3'
NI-0096 (SEQ ID NO: 16)
5'-UUCAGGAUAUUAGUGACUC-3'
NI-0097 (SEQ ID NO: 17)
5'-UCCUUUCUAGGACACCUCG-3'
NI-0098 (SEQ ID NO: 18)
5'-AUCCUUUCUAGGACACCUC-3'
NI-0099 (SEQ ID NO: 19)
5'-UUUGCUGUUUUCCAGCCAG-3'

In the as2 nucleotide, there is no particular limitation on "one to several". The "one to several" may mean, for example, 1 to 7, preferably 1 to 5, more preferably 1 to 4, and still more preferably 1, 2, or 3. The as2 nucleotide is not limited as long as it has the same function as the as1 nucleotide, for example, and more specifically, as long as it has a function of inhibiting the expression of the periostin gene. The term "and/or" means "at least one of", and also can be expressed as "at least one selected from the group consisting of" (the same applies hereinafter).

In the as3 nucleotide, the identity is, for example, at least 90%, preferably at least 93%, 94%, 95%, 96%, 97%, 98%, or 99%. The as3 nucleotide is not limited as long as it has the same function as the as1 nucleotide, for example, and more specifically, as long as it has a function of inhibiting the expression of the periostin gene. The identity can be calculated with analysis software such as BLAST or FASTA using default parameters, for example (the same applies hereinafter).

As described above, the expression inhibitory sequence may consist of the as nucleotide or may include the as nucleotide. In the latter case, the expression inhibitory sequence may further include an overhang(s), for example.

In the expression inhibitory sequence, the overhang may be added to at least one of the 3' end and the 5' end of the as nucleotide, preferably to the 3' end of the as nucleotide, for example.

There is no particular limitation on the overhang, and, for example, there are no particular limitations on the length and sequence thereof. The overhang can be represented by $(N)_n$, for example. N represents a base, which may be a natural base or an artificial base, for example. Examples of the natural base include A, C, G, U, and T. n represents a positive integer, which indicates the base length of the overhang. The length (n) of the overhang $(N)_n$ is, for example, 1-, 2-, or 3-mer, preferably 1- or 2-mer, and more preferably 2-mer. When the overhang $(N)_n$ has a length of 2-mer or more ($n \geq 2$), the successive bases (N) may be the same or different, for example. As the sequence of the overhang $(N)_n$, an overhang of an antisense strand of siRNA is applicable, for example. Examples of $(N)_n$ include, from the 3' side or the 5' side, UU, CU, UC, GA, AG, GC, UA, AA, CC, GU, UG, CG, AU, and TT.

When the expression inhibitory sequence has the overhang, the expression inhibitory sequence may be such that the as1 nucleotide and the overhang are linked to each other, for example. Specific examples thereof include a nucleotide having a base sequence represented by any one of SEQ ID NOs: 20 to 38 (where n is a positive integer) in which the as1 nucleotide and the overhang are linked to each other. In each of the following sequences, $(N)_n$ is an overhang, which is not particularly limited and is as described above, and the length (n) thereof preferably is 2-mer. Although an example of the overhang (in the 5' to 3' direction) is shown beside each sequence, the present invention is not limited thereto. In each of the following sequences, a region excluding $(N)_n$ may be the as2 nucleotide or the as3 nucleotide.

NI-0079 (SEQ ID NO: 20)
TT 5'-AAGUAUUUCUUUUUGGUGC$(N)_n$-3'
NI-0080 (SEQ ID NO: 21)
TT 5'-GAAGUAUUUCUUUUUGGUG$(N)_n$-3'
NI-0081 (SEQ ID NO: 22)
TT 5'-AAUCUGGUUCCCAUGGAUG$(N)_n$-3'
NI-0082 (SEQ ID NO: 23)
TT 5'-UUUCUAGGACACCUCGUGG$(N)_n$-3'
NI-0083 (SEQ ID NO: 24)
TT 5'-UUGUUUGGCAGAAUCAGGA$(N)_n$-3'
NI-0084 (SEQ ID NO: 25)
TT 5'-UCAAUAACUUGUUUGGCAG$(N)_n$-3'
NI-0085 (SEQ ID NO: 26)
TT 5'-AGCUCAAUAACUUGUUUGG$(N)_n$-3'
NI-0086 (SEQ ID NO: 27)
TT 5'-UUGCUGUUUUCCAGCCAGC(N)n-3'
NI-0087 (SEQ ID NO: 28)
TT 5'-UGGUUUGCUGUUUUCCAGC$(N)_n$-3'
NI-0088 (SEQ ID NO: 29)
TT 5'-GAUGCCAAGCCUAAUUGGG$(N)_n$-3'
NI-0091 (SEQ ID NO: 30)
AG 5'-UGAUUCGAGCACAAUUAAC$(N)_n$-3'
NI-0092 (SEQ ID NO: 31)
UC 5'-UACUGUUAUACUGUCACCG$(N)_n$-3'
NI-0093 (SEQ ID NO: 32)
AU 5'-UAAGCACACGGUCAAUGAC$(N)_n$-3'
NI-0094 (SEQ ID NO: 33)
GU 5'-UAAUUGGGCUACCAGGUCG$(N)_n$-3'
NI-0095 (SEQ ID NO: 34)
AU 5'-AUCAGAUCGUUGAUUUAGG$(N)_n$-3'
NI-0096 (SEQ ID NO: 35)
CG 5'-UUCAGGAUAUUAGUGACUC$(N)_n$-3'
NI-0097 (SEQ ID NO: 36)
UG 5'-UCCUUUCUAGGACACCUCG$(N)_n$-3'
NI-0098 (SEQ ID NO: 37)
GU 5'-AUCCUUUCUAGGACACCUC$(N)_n$-3'
NI-0099 (SEQ ID NO: 38)
CU 5'-UUUGCUGUUUUCCAGCCAG$(N)_n$-3'

Preferably, the nucleic acid molecule of the present invention further includes a complementary sequence that anneals to the expression inhibitory sequence. Examples of the complementary sequence include a nucleotide complementary to the as nucleotide in the expression inhibitory sequence (hereinafter referred to as "complementary nucleotide"). The complementary sequence may include the complementary nucleotide or may consist of the complementary nucleotide.

The complementary sequence is not limited as long as it can anneal to the expression inhibitory sequence, for example. The complementarity between the complementary nucleotide in the complementary sequence and the as nucleotide in the expression inhibitory sequence is, for example, at least 90%, preferably at least 93%, at least 94%, at least 95%, at least 96%, at least 97%, at least 98%, at least 99%, and more preferably 100%. The expression inhibitory sequence and the complementary sequence preferably are such that, for example, the as nucleotide in the former and the complementary nucleotide in the latter exhibit the above-described complementarity, and a region other than the as nucleotide in the former and a region other than the complementary nucleotide in the latter may be either complementary or non-complementary. When the expression inhibitory sequence is aligned with the complementary sequence, sequences to be paired with each other may or may not be present between the region other than the as nucleotide in the former and the region other than the complementary nucleotide in the latter, for example. Specifically, the region other than the as nucleotide in the former and the region other than the complementary nucleotide in the latter may be the overhangs, for example. That is, when the expression inhibitory sequence is aligned with the complementary sequence, the expression inhibitory sequence may have a shape with an overhang protruding toward the 3' side (or the 5' side), while the complementary sequence may have a shape with an overhang protruding toward the 5' side (or the 3' side).

The complementary nucleotide may be, for example, the following nucleotide (s1), (s2), or (s3):

(s1) a nucleotide that has a base sequence complementary to the base sequence of the nucleotide (as1);
(s2) a nucleotide that has a base sequence complementary to the base sequence of the nucleotide (as2); and
(s3) a nucleotide that has a base sequence complementary to the base sequence of the nucleotide (as3).

Hereinafter, the nucleotides (s1), (s2), and (s3) are collectively referred to as "s nucleotides", and they are referred to as "s1 nucleotide", "s2 nucleotide", and "s3 nucleotide", respectively.

The complementary sequence may consist of the s nucleotide or may include the s nucleotide, for example.

The length of the complementary sequence is not particularly limited, and is, for example, 18- to 32-mer, preferably 19- to 30-mer, and more preferably 19-, 20-, or 21-mer.

Specific examples of the sequence of the s1 nucleotide are shown below. The sequences represented by SEQ ID NOs: 39 to 57 are perfectly complementary to the as1 nucleotides that have the base sequences represented by the above SEQ ID NOs: 1 to 19, respectively. The names shown below (the names indicated ahead of the sequence identification numbers) also can be used to refer to nucleotides having the base sequences represented by SEQ ID NOs: 39 to 57, expression inhibitory sequences consisting of the nucleotides, expression inhibitory sequences including the nucleotides, and nucleic acid molecules including the nucleotides.

NI-0079 (SEQ ID NO: 39)
5'-GCACCAAAAAGAAAUACUU-3'
NI-0080 (SEQ ID NO: 40)
5'-CACCAAAAAGAAAUACUUC-3'
NI-0081 (SEQ ID NO: 41)
5'-CAUCCAUGGGAACCAGAUU-3'
NI-0082 (SEQ ID NO: 42)
5'-CCACGAGGUGUCCUAGAAA-3'
NI-0083 (SEQ ID NO: 43)
5'-UCCUGAUUCUGCCAAACAA-3'
NI-0084 (SEQ ID NO: 44)
5'-CUGCCAAACAAGUUAUUGA-3'
NI-0085 (SEQ ID NO: 45)
5'-CCAAACAAGUUAUUGAGCU-3'
NI-0086 (SEQ ID NO: 46)
5'-GCUGGCUGGAAAACAGCAA-3'
NI-0087 (SEQ ID NO: 47)
5'-GCUGGAAAACAGCAAACCA-3'
NI-0088 (SEQ ID NO: 48)
5'-CCCAAUUAGGCUUGGCAUC-3'
NI-0091 (SEQ ID NO: 49)

5'-GUUAAUUGUGCUCGAAUCA-3'
NI-0092 (SEQ ID NO: 50)
5'-CGGUGACAGUAUAACAGUA-3'
NI-0093 (SEQ ID NO: 51)
5'-GUCAUUGACCGUGUGCUUA-3'
NI-0094 (SEQ ID NO: 52)
5'-CGACCUGGUAGCCCAAUUA-3'
NI-0095 (SEQ ID NO: 53)
5'-CCUAAAUCAACGAUCUGAU-3'
NI-0096 (SEQ ID NO: 54)
5'-GAGUCACUAAUAUCCUGAA-3'
NI-0097 (SEQ ID NO: 55)
5'-CGAGGUGUCCUAGAAAGGA-3'
NI-0098 (SEQ ID NO: 56)
5'-GAGGUGUCCUAGAAAGGAU-3'
NI-0099 (SEQ ID NO: 57)
5'-CUGGCUGGAAAACAGCAAA-3'

As described above, the complementary sequence may consist of the complementary nucleotide or may include the complementary nucleotide. In the latter case, the complementary sequence may further include an overhang(s), for example. In the complementary sequence, the overhang may be added to at least one of the 3' end and the 5' end of the complementary nucleotide, preferably to the 3' end of the complementary nucleotide, for example.

There is no particular limitation on the overhang, and the description regarding the overhang in the expression inhibitory sequence also applies to the overhang in the complementary sequence. As the sequence of the overhang $(N)_n$ in the complementary sequence, an overhang of a sense strand of siRNA is applicable, for example. Examples of the $(N)_n$ include, from the 3' side or the 5' side, UU, CU, UC, CA, AC, GA, AG, GC, UA, AA, CC, UG, GU, CG, AU, TT, and GG.

When the complementary sequence has the overhang, the complementary sequence may be such that the s1 nucleotide and the overhang are linked to each other, for example. Specific examples thereof include a nucleotide having a base sequence represented by any one of SEQ ID NOs: 58 to 76 (where n is a positive integer) in which the s nucleotide and the overhang are linked to each other. In each of the following sequences, $(N)_n$ is an overhang, which is not particularly limited and is as described above, and the length (n) thereof preferably is 2-mer. Although an example of the overhang is shown beside each sequence, the present invention is not limited thereto. In each of the following sequences, a region excluding $(N)_n$ may be the s2 nucleotide or the s3 nucleotide.

NI-0079 (SEQ ID NO: 58)
5'-GCACCAAAAAGAAAUACUU$(N)_n$-3' TT
NI-0080 (SEQ ID NO: 59)
5'-CACCAAAAAGAAAUACUUC$(N)_n$-3' TT
NI-0081 (SEQ ID NO: 60)
5'-CAUCCAUGGGAACCAGAUU$(N)_n$-3' TT
NI-0082 (SEQ ID NO: 61)
5'-CCACGAGGUGUCCUAGAAA$(N)_n$-3' TT
NI-0083 (SEQ ID NO: 62)
5'-UCCUGAUUCUGCCAAACAA$(N)_n$-3' TT
NI-0084 (SEQ ID NO: 63)
5'-CUGCCAAACAAGUUAUUGA$(N)_n$-3' TT
NI-0085 (SEQ ID NO: 64)
5'-CCAAACAAGUUAUUGAGCU$(N)_n$-3' TT
NI-0086 (SEQ ID NO: 65)
5'-GCUGGCUGGAAAACAGCAA$(N)_n$-3' TT
NI-0087 (SEQ ID NO: 66)
5'-GCUGGAAAACAGCAAACCA$(N)_n$-3' TT
NI-0088 (SEQ ID NO: 67)
5'-CCCAAUUAGGCUUGGCAUC$(N)_n$-3' TT
NI-0091 (SEQ ID NO: 68)
5'-GUUAAUUGUGCUCGAAUCA$(N)_n$-3' UC
NI-0092 (SEQ ID NO: 69)

5'-CGGUGACAGUAUAACAGUA$(N)_n$-3' AA
NI-0093 (SEQ ID NO: 70)
5'-GUCAUUGACCGUGUGCUUA$(N)_n$-3' CA
NI-0094 (SEQ ID NO: 71)
5'-CGACCUGGUAGCCCAAUUA$(N)_n$-3' GG
NI-0095 (SEQ ID NO: 72)
5'-CCUAAAUCAACGAUCUGAU$(N)_n$-3' UU
NI-0096 (SEQ ID NO: 73)
5'-GAGUCACUAAUAUCCUGAA$(N)_n$-3' GA
NI-0097 (SEQ ID NO: 74)
5'-CGAGGUGUCCUAGAAAGGA$(N)_n$-3' UC
NI-0098 (SEQ ID NO: 75)
5'-GAGGUGUCCUAGAAAGGAU$(N)_n$-3' CA
NI-0099 (SEQ ID NO: 76)
5'-CUGGCUGGAAAACAGCAAA$(N)_n$-3' CC

Examples of the combination of the as nucleotide and the s nucleotide are shown in Tables 1 and 2 below. It is to be noted, however, that the present invention is not limited to these illustrative examples. In the following sequences, the as nucleotide and the s nucleotide each may have an overhang $(N)_n$ at its 3' end or at its 5' end. Preferably, $(N)_n$ is 2-mer.

[Table 1]

| | |
|---|---|
| NI-0079 | |
| (SEQ ID NO: 3 9) | 5' -GCACCAAAAAGAAAUACUU-3' |
| (SEQ ID NO:1) | 3' -CGUGGUUUUUCUUUAUGAA-5' |
| NI-0080 | |
| (SEQ ID NO: 40) | 5' -CACCAAAAAGAAAUACUUC-3' |
| (SEQ ID NO: 2) | 3' -GUGGLUUUUCUUUAUGAAG-5' |
| NI-008 | |
| (SEQ ID NO: 41) | 5' -CAUCCAUGGGAACCAGAUU-3' |
| (SEQ ID NO: 3) | 3' -GUAGGUACCCUUGGUCUAA-5' |
| NI-0082 | |
| (SEQ ID NO: 42) | 5' -CCACGAGGUGUCCUAGAAA-3' |
| (SEQ ID NO: 4) | 3' -GGUCCUGCACACCAUCUUU-5' |
| NI-0083 | |
| (SEQ ID NO: 43) | 5' -UCCUGAUUCUGCCAAACAA-3' |
| (SEQ ID NO: 5) | 3' -AGGACUAAGACGGUUUGUU-5' |
| NI-0084 | |
| (SEQ ID NO: 44) | 5' -CUGCCAAACAAGUUAUUGA-3' |
| (SEQ ID NO: 6) | 3' -GACGGUUUGUUCAAUAACU-5' |
| NI-0085 | |
| (SEQ ID NO: 45) | 5' CCAAACAAGUUAUUGAGCU-3' |
| (SEQ ID NO: 7) | 3' -GGUUUGUUCAAUAACUCGA-5' |
| NI-0086 | |
| (SEQ ID NO: 46) | 5' -GCUGGCUGGAAAACAGCAA-3' |
| (SEQ ID NO: 8) | 3' -CGACCCACCUUUUGUCGUU-5' |
| NI-0087 | |
| (SEQ ID NO: 47) | 5' -GCUGGAAAACAGCAAACCA-3' |
| (SEQ ID NO: 9) | 3' -CGACCUUUUGLCGUUUGGU-5' |
| NI-0088 | |
| (SEQ ID NO: 48) | 5' -CCCAAUUAGGCUUGGCAUC-3' |
| (SEQ ID NO: 10) | 3' -GGGUUAAUCCGAACCGUAG-5' |

[Table 2]

| | |
|---|---|
| NI-0091 | |
| (SEQ ID NO: 49) | 5' -GUUAAUUGUGCUCGAAUCA-3' |
| (SEQ ID NO: 11) | 3' -CAAUUAACACGAGCUUACU-5' |
| NI-0092 | |
| (SEQ ID NO:90) | 5' -CGGUGACAGUAUAACAGUA-3' |
| (SEQ ID NO: 12) | 3' -GCCACUGUCAUAUUGUCAU-5' |
| NI-0093 | |
| (SEQ ID NO: 51) | 5' -GUCAUUGACCGUGUGCUUA-3' |
| (SEQ ID NO: 1 3) | 3' -CAGUAACUGGCACACGAAU-5' |
| NI-0094 | |
| (SEQ ID NO: 5 2) | 5' -CGACCUGGUAGCCCAAUUA-3' |
| (SEQ ID NO: 14) | 3' -GCUGGACCAUCGGGUUAAU-5' |
| NI-0095 | |
| (SEQ ID NO: 5 3) | 5' -CCUAAAUCAACGAUCUGAU-3' |
| (SEQ ID NO: 15) | 3' -GGAUUUAGUUGCUAGACUA-5' |
| NI-0096 | |
| (SEQ ID NO: 54) | 5' -GAGUCACUAAUAUCCUGAA-3' |
| (SEQ ID NO: 16) | 3' -CUCAGUGAUUAUAGGACUU-5' |
| NI-0097 | |
| (SEQ ID NO: 55) | 5' -CGAGGUGUCCUAGAAAGGA-3' |
| (SEQ ID NO: 17) | 3' -GCUCCACAGGAUCUUUCCU-5' |
| NI-0098 | |
| (SEQ ID NO: 56) | 5' -GAGGUGUCCUAGAAAGGAU-3' |
| (SEQ ID NO: 18) | 3' -CUCCACAGGAUCUUUCCUA-5' |
| NI-0099 | |
| (SEQ ID NO: 57) | 5' -CUGGCUGGAAAACAGCAAA-3' |
| (SEQ ID NO: 19) | 3' -GACCGACCUUUUGUCGUUU-5' |

The building block of the nucleic acid molecule of the present invention is not particularly limited, and may be, for example, a nucleotide residue. Examples of the nucleotide residue include a ribonucleotide residue and a deoxyribonucleotide residue. The nucleotide residue may be the one that is not modified (unmodified nucleotide residue) or the one that is modified (modified nucleotide residue), for example.

The nucleic acid molecule of the present invention may be an RNA molecule, for example. The nucleic acid molecule of the present invention may be an RNA molecule consisting of ribonucleotide residues, or may be an RNA molecule including, in addition to a ribonucleotide residue(s), a deoxyribonucleotide residue(s) and/or a non-nucleotide residue(s), for example.

The nucleic acid molecule of the present invention may be a double-stranded nucleic acid molecule or a single-stranded nucleic acid molecule, for example. The nucleic acid molecule of the present invention will be described below with reference to an example where it is a double-stranded nucleic acid molecule and an example where it is a single-stranded nucleic acid molecule.

(1) Double-stranded nucleic acid molecule

When the nucleic acid molecule of the present invention is a double-stranded nucleic acid molecule, it includes two single-stranded nucleic acids, and either one of the single-stranded nucleic acids may include the expression inhibitory sequence. The double-stranded nucleic acid molecule may be, for example, a so-called siRNA, or a precursor or the like of siRNA. The double-stranded nucleic acid molecule preferably is such that, for example, one of the single-stranded nucleic acids, i.e., an antisense strand of the double-stranded nucleic acid molecule, includes the expression inhibitory sequence, and the other single-stranded nucleic acid, i.e., a sense strand of the double-stranded nucleic acid molecule, includes the complementary sequence. The antisense strand may be a single-stranded nucleic acid consisting of the expression inhibitory sequence or may be a single-stranded nucleic acid including the expression inhibitory sequence, for example. The sense strand may be a single-stranded nucleic acid consisting of the complementary sequence or may be a single-stranded nucleic acid including the complementary sequence, for example.

In the double-stranded nucleic acid molecule, there is no particular limitation on the length of each single-stranded nucleic acid. The antisense strand is, for example, 18- to 32-mer, preferably 19- to 30-mer, and more preferably 19-, 20-, or 21-mer. The sense strand is, for example, 18- to 32-mer, preferably 19- to 30-mer, and more preferably 19-, 20-, or 21-mer.

It is preferable that the antisense strand and the sense strand each have an overhang in at least one of the 3' end and the 5' end. The length of the overhang is, for example, 1-, 2-, or 3-mer, preferably 1- or 2-mer, and more preferably 2-mer. There is no particular limitation on the sequence of the overhang, and examples thereof include those described above.

(2) Single-stranded nucleic acid molecule

When the nucleic acid molecule of the present invention is a single-stranded nucleic acid molecule, the single-stranded nucleic acid molecule is not limited as long as it includes the expression inhibitory sequence, and there are no particular limitations on other configurations.

The nucleic acid molecule is, for example, a single-stranded nucleic acid molecule composed of a single strand in which, for example, the expression inhibitory sequence and the complementary sequence are arranged in a direction in which they can anneal to each other.

There is no particular limitation on the order in which the expression inhibitory sequence and the complementary sequence are linked. For example, the 3' end of the expression inhibitory sequence may be linked to the 5' end of the complementary sequence, or the 5' end of the expression inhibitory sequence may be linked to the 3' end of the complementary sequence. Among them, the former is preferable. In the single-stranded nucleic acid molecule, the expression inhibitory sequence and the complementary sequence may be linked to each other either directly or indirectly, for example. Examples of the direct linkage include linkage by phosphodiester linkage. Examples of the indirect linkage include linkage via a linker region.

The linker region may be composed of a nucleotide residue(s) or a non-nucleotide residue(s), for example, and may be composed of the above-described nucleotide residue(s) and non-nucleotide residue(s). Examples of the nucleotide residue include a ribonucleotide residue and a deoxyribonucleotide residue.

As specific examples of the single-stranded nucleic acid molecule, a single-stranded nucleic acid molecule according to a first embodiment in which a loop is formed at one site by intramolecular annealing, and a single-stranded nucleic acid molecule according to a second embodiment in which loops are formed at two sites by intramolecular annealing are illustrated below. It is to be noted, however, that the present invention is not limited thereto.

(2-1) First Embodiment

The single-stranded nucleic acid molecule according to the first embodiment is a molecule in which a 5' side region and a 3' side region anneal to each other, whereby a double-stranded structure (a stem structure) is formed. It can be said that this form corresponds to shRNA (small hairpin RNA or short hairpin RNA). The shRNA has a hairpin structure, which generally has one stem region and one loop region.

The nucleic acid molecule according to the present embodiment may be configured so that, for example, it includes a region (X), a linker region (Lx), and a region (Xc), and the regions (X) and (Xc) are linked via the linker region (Lx). It is preferable that the region (Xc) is complementary to the region (X). More specifically, it is preferable that one of the regions (X) and (Xc) includes the expression inhibitory sequence, and the other includes the complementary sequence. The regions (X) and (Xc) each include either the expression inhibitory sequence or the complementary sequence. Accordingly, for example, the nucleic acid molecule can form a stem structure between the regions (X) and (Xc) by intramolecular annealing, and the linker region (Lx) forms a loop structure.

The nucleic acid molecule may include, for example, the region (Xc), the linker region (Lx), and the region (X) in this order from the 5' side to the 3' side, or may include the region (Xc), the linker region (Lx), and the region (X) in this order from the 3' side to the 5' side. The expression inhibitory sequence may be arranged either in the region (X) or in the region (Xc), for example. Preferably, the expression inhibitory sequence is arranged upstream of the complementary sequence, i.e., on the 5' side with respect to the complementary sequence.

FIG. 1 shows schematic views of an example of the nucleic acid molecule according to the present embodiment. FIG. 1A is a schematic view schematically showing the order of the respective regions in the nucleic acid molecule, and FIG. 1B is a schematic view showing a state where the nucleic acid molecule forms a double strand within the molecule. As shown in FIG. 1B, in the nucleic acid molecule, a double strand is formed between the regions (Xc) and (X), and the Lx region forms a loop structure depending on its length. FIG. 1 merely illustrates the order in which the respective regions are linked to each other and the positional relationship of the respective regions forming the double strand, and for example, the length of each region, the shape of the linker region (Lx), and the like are not limited to those shown in FIG. 1.

In the nucleic acid molecule, there is no particular limitation on the number of bases in each of the regions (Xc) and (X). Examples of the lengths of the respective regions are given below. It is to be noted, however, that the present invention is by no means limited thereto.

In the nucleic acid molecule, the relationship between the number of bases (X) in the region (X) and the number of bases (Xc) in the region (Xc) satisfies the condition of Expression (3) or (5) below, for example. In the former case, the relationship specifically satisfies the condition of Expression (11) below, for example.

$$X > Xc \qquad \ldots (3)$$

$$X - Xc = 1 \text{ to } 10, \text{ preferably } 1, 2, \text{ or } 3, \text{ more preferably } 1 \text{ or } 2 \qquad \ldots (11)$$

$$X = Xc \qquad \ldots (5)$$

When the region (X) or the region (Xc) includes the expression inhibitory sequence, the region may consist of the expression inhibitory sequence or may include the expression inhibitory sequence, for example. The number of bases in the expression inhibitory sequence is, for example, as described above. The region including the expression inhibitory sequence further may include an additional sequence on the 5' side and/or the 3' side of the expression inhibitory sequence, for example. The number of bases in the additional sequence is, for example, 1 to 31, preferably 1 to 21, and more preferably 1 to 11.

There is no particular limitation on the number of bases in the region (X). When the region (X) includes the expression inhibitory sequence, the lower limit thereof is 19, for example. The upper limit thereof is, for example, 50, preferably 30, and more preferably 25. Specifically, the number of bases in the region (X) is, for example, 19 to 50, preferably 19 to 30, and more preferably 19 to 25.

There is no particular limitation on the number of bases in the region (Xc). The lower limit thereof is, for example, 19, preferably 20, and more preferably 21. The upper limit thereof is, for example, 50, preferably 40, and more preferably 30.

Preferably, the linker region (Lx) has a structure such that self-annealing is not caused inside itself. For example, as described above, the linker region (Lx) may be composed of the nucleotide residue(s) or the non-nucleotide residue(s), or may include both of them.

When the linker region (Lx) includes the nucleotide residue(s), there is no particular limitation on the length of the linker region (Lx). The length of the linker region (Lx) preferably is such that, for example, the regions (X) and (Xc) can form a double strand. The number of bases in the linker region (Lx) is such that the lower limit thereof is, for example, 1, preferably 2, and more preferably 3, and the upper limit thereof is, for example, 100, preferably 80, and more preferably 50, 40, 30, 20, or 10.

There is no particular limitation on the full length of the nucleic acid molecule. In the nucleic acid molecule, the lower limit of the total number of bases (the number of bases in the full-length nucleic acid molecule) is, for example, 38, preferably 42, more preferably 50, still more preferably 51, and particularly preferably 52, and the upper limit of the same is, for example, 300, preferably 200, more preferably 150, still more preferably 100, and particularly preferably 80. In the nucleic acid molecule, the lower limit of the total number of bases excluding those in the linker region (Lx) is, for example, 38, preferably 42, more preferably 50, still more preferably 51, and particularly preferably 52, and the upper limit of the same is, for example, 300, preferably 200, more preferably 150, still more preferably 100, and particularly preferably 80.

(2-2) Second Embodiment

The single-stranded nucleic acid molecule according to the second embodiment is a molecule in which intramolecular annealing occurs in each of a 5' side region and a 3' side region, whereby two double-stranded structures (stem structures) are formed. Regarding this embodiment, the disclosures in WO 2012/005368 and WO 2012/017979 are incorporated herein by reference, for example.

The nucleic acid molecule according to the present embodiment may be configured so that, for example, it includes a 5' side region (Xc), an inner region (Z), and a 3' side region (Yc) in this order from the 5' side to the 3' side. It is preferable that the inner region (Z) is composed of an inner 5' side region (X) and an inner 3' side region (Y) that are linked to each other, the 5' side region (Xc) is complementary to the inner 5' side region (X), and the 3' side region

(Yc) is complementary to the inner 3' side region (Y). Also, it is preferable that at least one of the inner region (Z), the 5' side region (Xc), and the 3' side region (Yc) includes the expression inhibitory sequence.

In the nucleic acid molecule, the 5' side region (Xc) is complementary to the inner 5' side region (X), and the 3' side region (Yc) is complementary to the inner 3' side region (Y). Thus, on the 5' side, a double strand can be formed by fold-back of the region (Xc) toward the region (X) and self-annealing of the regions (Xc) and (X), and on the 3' side, a double strand can be formed by fold-back of the region (Yc) toward the region (Y) and self-annealing of the regions (Yc) and (Y).

As described above, the inner region (Z) is composed of the inner 5' side region (X) and the inner 3' side region (Y) that are linked to each other. The region (X) and the region (Y) are linked directly to each other with no intervening sequence therebetween, for example. The inner region (Z) is defined as being "composed of the inner 5' side region (X) and the inner 3' side region (Y) that are linked to each other" merely to indicate the sequence context between the 5' side region (Xc) and the 3' side region (Yc). This definition does not intend to limit that, in the use of the nucleic acid molecule, the inner 5' side region (X) and the inner 3' side region (Y) in the inner region (Z) are discrete independent regions. That is, for example, when the inner region (Z) includes the expression inhibitory sequence, the expression inhibitory sequence may be arranged so as to extend across the region (X) and the region (Y) in the inner region (Z).

In the nucleic acid molecule, the 5' side region (Xc) and the inner 5' side region (X) may be linked to each other either directly or indirectly, for example. In the former case, the direct linkage may be linkage by phosphodiester linkage, for example. In the latter case, the nucleic acid molecule may be configured so that it has a linker region (Lx) between the region (Xc) and the region (X), and the region (Xc) and the region (X) are linked to each other via the linker region (Lx), for example.

In the nucleic acid molecule, the 3' side region (Yc) and the inner 3' side region (Y) may be linked to each other either directly or indirectly, for example. In the former case, the direct linkage may be linkage by phosphodiester linkage, for example. In the latter case, the nucleic acid molecule may be configured so that it has a linker region (Ly) between the regions (Yc) and (Y), and the regions (Yc) and (Y) are linked to each other via the linker region (Ly), for example.

The nucleic acid molecule of the present invention may have both the linker regions (Lx) and (Ly), or may have either one of them, for example. In the latter case, the nucleic acid molecule may be configured so that, for example, it has the linker region (Lx) between the 5' side region (Xc) and the inner 5' side region (X) and does not have the linker region (Ly) between the 3' side region (Yc) and the inner 3' side region (Y), i.e., the regions (Yc) and (Y) are linked directly to each other. Also, in the latter case, the nucleic acid molecule may be configured so that, for example, it has the linker region (Ly) between the 3' side region (Yc) and the inner 3' side region (Y) and does not have the linker region (Lx) between the 5' side region (Xc) and the inner 5' side region (X), i.e., the regions (Xc) and (X) are linked directly to each other.

Preferably, the linker regions (Lx) and (Ly) each have a structure such that self-annealing is not caused inside themselves. For example, as described above, each of the linker regions (Lx) and (Ly) may be composed of the nucleotide residue(s) or the non-nucleotide residue(s) or may include both of them.

FIG. 2 shows schematic views illustrating an example of the nucleic acid molecule of the present embodiment without the linker regions. FIG. 2A is a schematic view showing the order of the respective regions from the 5' side to the 3' side in the nucleic acid molecule. FIG. 2B is a schematic view showing a state where double strands are formed in the nucleic acid molecule. As shown in FIG. 2B, in the nucleic acid molecule, the 5' side region (Xc) folds back, whereby a double strand is formed between the 5' side region (Xc) and the inner 5' side region (X), and the 3' side region (Yc) folds back, whereby a double strand is formed between the 3' side region (Yc) and the inner 3' side region (Y). FIG. 2 merely illustrates the order in which the respective regions are linked to each other and the positional relationship of the respective regions forming the double strands, and for example, the length of each region and the like are not limited to those shown in FIG. 2.

FIG. 3 shows schematic views of an example of the nucleic acid molecule of the present invention, including the linker regions. FIG. 3A is a schematic view showing the order of the respective regions from the 5' side to the 3' side in the nucleic acid molecule. FIG. 3B is a schematic view showing a state where double strands are formed in the nucleic acid molecule. As shown in FIG. 3B, in the nucleic acid molecule, the double strands are formed between the 5' side region (Xc) and the inner 5' side region (X) and between the inner 3' side region (Y) and the 3' side region (Yc), respectively, and the Lx region and the Ly region form loop structures. FIG. 3 merely illustrates the order in which the respective regions are linked to each other and the positional relationship of the respective regions forming the double strands, and for example, the length of each region and the like are not limited to those shown in FIG. 3.

In the nucleic acid molecule, there is no particular limitation on the number of bases in each of the 5' side region (Xc), the inner 5' side region (X), the inner 3' side region (Y), and the 3' side region (Yc). Examples of the lengths of the respective regions are given below. It is to be noted, however, that the present invention is by no means limited thereto.

As described above, the 5' side region (Xc) may be complementary to the entire region of the inner 5' side region (X), for example. In this case, it is preferable that, for example, the 5' side region (Xc) has the same base length as the inner 5' side region (X), and has a base sequence complementary to the entire region extending from the 5' end

to the 3' end of the inner 5' side region (X). It is more preferable that the 5' side region (Xc) has the same base length as the inner 5' side region (X), and all the bases in the 5' side region (Xc) are complementary to all the bases in the inner 5' side region (X), i.e., the 5' side region (Xc) is perfectly complementary to the inner 5' side region (X), for example. It is to be noted, however, that the configuration of the 5' side region (Xc) is not limited thereto, and one or a few bases in the 5' side region (Xc) may be non-complementary to the corresponding bases in the inner 5' side region (X), for example, as described above.

Furthermore, as described above, the 5' side region (Xc) may be complementary to part of the inner 5' side region (X), for example. In this case, it is preferable that, for example, the 5' side region (Xc) has the same base length as the part of the inner 5' side region (X), i.e., the 5' side region (Xc) has a base sequence whose base length is shorter than the base length of the inner 5' side region (X) by one or more bases. It is more preferable that the 5' side region (Xc) has the same base length as the part of the inner 5' side region (X), and all the bases in the 5' side region (Xc) are complementary to all the bases in the part of the inner 5' side region (X), i.e., the 5' side region (Xc) is perfectly complementary to the part of the inner 5' side region (X), for example. The part of the inner 5' side region (X) preferably is a region (segment) having a base sequence composed of successive bases starting from the base at the 5' end (the 1st base) in the inner 5' side region (X), for example.

As described above, the 3' side region (Yc) may be complementary to the entire region of the inner 3' side region (Y), for example. In this case, it is preferable that, for example, the 3' side region (Yc) has the same base length as the inner 3' side region (Y), and has a base sequence complementary to the entire region extending from the 5' end to the 3' end of the inner 3' side region (Y). It is more preferable that the 3' side region (Yc) has the same base length as the inner 3' side region (Y), and all the bases in the 3' side region (Yc) are complementary to all the bases in the inner 3' side region (Y), i.e., the 3' side region (Yc) is perfectly complementary to the inner 3' side region (Y), for example. It is to be noted, however, that the configuration of the 3' side region (Yc) is not limited thereto, and one or a few bases in the 3' side region (Yc) may be non-complementary to the corresponding bases in the inner 3' side region (Y), for example, as described above.

Furthermore, as described above, the 3' side region (Yc) may be complementary to part of the inner 3' side region (Y), for example. In this case, it is preferable that, for example, the 3' side region (Yc) has the same base length as the part of the inner 3' side region (Y), i.e., the 3' side region (Yc) has a base sequence whose base length is shorter than the base length of the inner 3' side region (Y) by one or more bases. It is more preferable that the 3' side region (Yc) has the same base length as the part of the inner 3' side region (Y), and all the bases in the 3' side region (Yc) are complementary to all the bases in the part of the inner 3' side region (Y), i.e., the 3' side region (Yc) is perfectly complementary to the part of the inner 3' side region (Y), for example. The part of the inner 3' side region (Y) preferably is a region (segment) having a base sequence composed of successive bases starting from the base at the 3' end (the 1st base) in the inner 3' side region (Y), for example.

In the nucleic acid molecule of the present invention, the relationship of the number of bases (Z) in the inner region (Z) with the number of bases (X) in the inner 5' side region (X) and the number of bases (Y) in the inner 3' side region (Y), and the relationship of the number of bases (Z) in the inner region (Z) with the number of bases (Xc) in the 5' side region (Xc) and the number of bases (Yc) in the 3' side region (Yc) satisfy the conditions of Expressions (1) and (2), for example.

$$Z = X + Y \qquad \ldots (1)$$

$$Z \geq Xc + Yc \qquad \ldots (2)$$

In the nucleic acid molecule, there is no particular limitation on the length relationship between the number of bases (X) in the inner 5' side region (X) and the number of bases (Y) in the inner 3' side region (Y), and may satisfy any of the conditions of the following expressions, for example.

$$X = Y \qquad \ldots (19)$$

$$X < Y \qquad \ldots (20)$$

$$X > Y \qquad \ldots (21)$$

In the nucleic acid molecule of the present invention, the relationship between the number of bases (X) in the inner 5' side region (X) and the number of bases (Xc) in the 5' side region (Xc), and the relationship between the number of bases (Y) in the inner 3' side region (Y) and the number of bases (Yc) in the 3' side region (Yc) satisfy any of the following conditions (a) to (d), for example.

(a) Conditions of Expressions (3) and (4) are satisfied.

$$X > Xc \quad \ldots (3)$$

$$Y = Yc \quad \ldots (4)$$

(b) Conditions of Expressions (5) and (6) are satisfied.

$$X = Xc \quad \ldots (5)$$

$$Y > Yc \quad \ldots (6)$$

(c) Conditions of Expressions (7) and (8) are satisfied.

$$X > Xc \quad \ldots (7)$$

$$Y > Yc \quad \ldots (8)$$

(d) Conditions of Expressions (9) and (10) are satisfied.

$$X = Xc \quad \ldots (9)$$

$$Y = Yc \quad \ldots (10)$$

In the above-described conditions (a) to (d), the difference between the number of bases (X) in the inner 5' side region (X) and the number of bases (Xc) in the 5' side region (Xc), and the difference between the number of bases (Y) in the inner 3' side region (Y) and the number of bases (Yc) in the 3' side region (Yc) preferably satisfy the following conditions, for example.

(a) Conditions of Expressions (11) and (12) are satisfied.

$$X - Xc = 1 \text{ to } 10\text{, preferably } 1, 2, 3\text{, or } 4\text{, and more preferably } 1, 2\text{, or } 3 \quad \ldots (11)$$

$$Y - Yc = 0 \quad \ldots (12)$$

(b) Conditions of Expressions (13) and (14) are satisfied.

$$X - Xc = 0 \quad \ldots (13)$$

$$Y - Yc = 1 \text{ to } 10, \text{ preferably } 1, 2, 3, \text{ or } 4, \text{ and more preferably } 1, 2, \text{ or } 3 \quad \ldots (14)$$

(c) Conditions of Expressions (15) and (16) are satisfied.

$$X - Xc = 1 \text{ to } 10, \text{ preferably } 1, 2, \text{ or } 3, \text{ and more preferably } 1 \text{ or } 2 \quad \ldots (15)$$

$$Y - Yc = 1 \text{ to } 10, \text{ preferably } 1, 2, \text{ or } 3, \text{ and more preferably } 1 \text{ or } 2 \quad \ldots (16)$$

(d) Conditions of Expressions (17) and (18) are satisfied.

$$X - Xc = 0 \quad \ldots (17)$$

$$Y - Yc = 0 \quad \ldots (18)$$

Regarding the nucleic acid molecules satisfying the conditions (a) to (d), examples of their structures are shown respectively in the schematic views of FIG. 4. FIG. 4 shows the nucleic acid molecules including the linker regions (Lx) and (Ly). FIG. 4A shows an example of the nucleic acid molecule satisfying the condition (a); FIG. 4B shows an example of the nucleic acid molecule satisfying the condition (b); FIG. 4C shows an example of the nucleic acid molecule satisfying the condition (c); and FIG. 4D shows an example of the nucleic acid molecule satisfying the condition (d). In FIG. 4, dotted lines indicate a state where double strands are formed by self-annealing. The nucleic acid molecules shown in FIG. 4 are all directed to examples where the relationship between the number of bases (X) in the inner 5' side region (X) and the number of bases (Y) in the inner 3' side region (Y) satisfy "X < Y" of Expression (20). However, it is to be noted that the relationship is not limited thereto, and "X = Y" of Expression (19) or "X > Y" of Expression (21) may be satisfied. The schematic views shown in FIG. 4 merely illustrate the relationship between the inner 5' side region (X) and the 5' side region (Xc) and the relationship between the inner 3' side region (Y) and the 3' side region (Yc), and for example, the length, the shape, and the like of each region, and also the presence or absence of the linker regions (Lx) and (Ly) are not limited to those shown in FIG. 4.

Each of the nucleic acid molecules satisfying the conditions (a) to (c) is configured so that, for example, when the double strands are formed by the 5' side region (Xc) and the inner 5' side region (X) and by the 3' side region (Yc) and the inner 3' side region (Y), respectively, the inner region (Z) includes at least one base that cannot be aligned with either of the 5' side region (Xc) and the 3' side region (Yc). In other words, these nucleic acid molecules are configured so as to include at least one base that does not form a double strand. In the inner region (Z), the base that cannot be aligned (a base that does not form the double strand) hereinafter also is referred to as an "unpaired base". In FIG. 4, a region composed of the unpaired base(s) is shown as "F". There is no particular limitation on the number of bases in the region (F). The number of bases (F) in the region (F) is as follows, for example: "X - Xc" in the case of the nucleic acid molecule satisfying the condition (a); "Y-Yc" in the case of the nucleic acid molecule satisfying the condition (b); and the total of "X - Xc" and "Y - Yc" in the case of the nucleic acid molecule satisfying the condition (c).

On the other hand, the nucleic acid molecule satisfying the condition (d) is configured so that, for example, the entire region of the inner region (Z) is aligned with the 5' side region (Xc) and the 3' side region (Yc); in other words, the entire region of the inner region (Z) forms a double strand. In the nucleic acid molecule satisfying the condition (d), the 5' end of the 5' side region (Xc) and the 3' end of the 3' side region (Yc) are not linked to each other.

Examples of the lengths of the respective regions in the nucleic acid molecule of the present invention are given below. It is to be noted, however, that the present invention is by no means limited thereto.

The total number of the bases in the 5' side region (Xc), the bases in the 3' side region (Yc), and the unpaired bases (F) in the inner region (Z) is equal to the number of the bases in the inner region (Z), for example. Thus, the length of the 5' side region (Xc) and the length of the 3' side region (Yc) can be determined as appropriate depending on the

length of the inner region (Z), the number of the unpaired bases (F), and the positions of the unpaired bases, for example.

The number of the bases in the inner region (Z) is 19 or more, for example. The lower limit of the number of the bases is, for example, 19, preferably 20, and more preferably 21. The upper limit of the number of the bases is, for example, 50, preferably 40, and more preferably 30. A specific example of the number of the bases in the inner region (Z) is 19, 20, 21, 22, 23, 24, 25, 26, 27, 28, 29, or 30.

When the inner region (Z) includes the expression inhibitory sequence, the inner region (Z) may be a region consisting of the expression inhibitory sequence or a region including the expression inhibitory sequence, for example. The number of bases in the expression inhibitory sequence is as described above, for example. When the inner region (Z) includes the expression inhibitory sequence, the inner region (Z) further may include an additional sequence on the 5' side and/or 3' side of the expression inhibitory sequence. The number of bases in the additional sequence is, for example, 1 to 31, preferably 1 to 21, more preferably 1 to 11, and still more preferably 1 to 7.

In the inner region (Z), there is no particular limitation on the position of the expression inhibitory sequence. As described above, the expression inhibitory sequence may be in the inner 5' side region (X) or in the inner 3' side region (Y), or may extend across the inner 5' side region (X) and the inner 3' side region (Y). When the nucleic acid molecule has the complementary sequence, for example, there is no particular limitation on the position of the complementary sequence. For example, the complementary sequence may be in the 5' side region (Xc) to be paired with the inner 5' side region (X), or may be in the 3' side region (Yc) to be paired with the inner 3' side region (Y). When the expression inhibitory sequence is arranged so as to extend across the inner 5' region (X) and the inner 3' side region (Y), for example, the complementary sequence may be divided into two segments that are present in the 5' side region (Xc) and the 3' side region (Yc). When the complementary sequence is divided into two segments, the complementary sequence may be such that, for example, a part to be paired with the unpaired base(s) is deleted.

The number of bases in the 5' side region (Xc) is, for example, 1 to 29, preferably 1 to 11, more preferably 1 to 7, still more preferably 1 to 4, and particularly preferably 1, 2, or 3. When the inner region (Z) or the 3' side region (Yc) includes the expression inhibitory sequence, the number of bases as described above is preferable, for example. A specific example is as follows: when the number of bases in the inner region (Z) is 19 to 30 (e.g., 19), the number of bases in the 5' side region (Xc) is, for example, 1 to 11, preferably 1 to 9 or 1 to 7, more preferably 1 to 4, and still more preferably 1, 2, or 3.

When the 5' side region (Xc) includes the expression inhibitory sequence, the 5' side region (Xc) may be a region consisting of the expression inhibitory sequence or a region including the expression inhibitory sequence, for example. The length of the expression inhibitory sequence is as described above, for example. When the 5' side region (Xc) includes the expression inhibitory sequence, the 5' side region (Xc) further may include an additional sequence on the 5' side and/or 3' side of the expression inhibitory sequence. The number of bases in the additional sequence is, for example, 1 to 11 and preferably 1 to 7.

The number of bases in the 3' side region (Yc) is, for example, 1 to 29, preferably 1 to 11, more preferably 1 to 7, still more preferably 1 to 4, and particularly preferably 1, 2, or 3. When the inner region (Z) or the 5' side region (Xc) includes the expression inhibitory sequence, the number of bases as described above is preferable, for example. A specific example is as follows: when the number of bases in the inner region (Z) is 19 to 30 (e.g., 19), the number of bases in the 3' side region (Yc) is, for example, 1 to 11, preferably 1 to 9 or 1 to 7, more preferably 1 to 4, and still more preferably 1, 2, or 3.

When the 3' side region (Yc) includes the expression inhibitory sequence, the 3' side region (Yc) may be a region consisting of the expression inhibitory sequence or a region including the expression inhibitory sequence, for example. The length of the expression inhibitory sequence is as described above, for example. When the 3' side region (Yc) includes the expression inhibitory sequence, the 3' side region (Yc) further may include an additional sequence on the 5' side and/or 3' side of the expression inhibitory sequence. The number of bases in the additional sequence is, for example, 1 to 11 and preferably 1 to 7.

As described above, the relationship among the number of bases in the inner region (Z), the number of bases in the 5' side region (Xc), and the number of bases in the 3' side region (Yc) can be expressed by Expression (2): "$Z \geq Xc + Yc$", for example. Specifically, the number of bases represented by "$Xc + Yc$" is equal to or less than the number of bases in the inner region (Z), for example. In the latter case, "$Z - (Xc + Yc)$" is, for example, 1 to 10, preferably 1 to 4, and more preferably 1, 2, or 3. The "$Z - (Xc + Yc)$" corresponds to the number of bases (F) in the unpaired base region (F) in the inner region (Z), for example.

In the single-stranded nucleic acid molecule, the end of the 5' side region (Xc) and the end of the 3' side region (Yc) preferably are on the 5' side or the 3' side with respect to the inner region (Z) in the state where intramolecular annealing has occurred, for example. In the former case, the number of bases (Xc) in the 5' side region (Xc) and the number of bases (Yc) in the 3' side region (Yc) satisfy the relationship of $Xc < Yc$. The number of bases (Xc) is, for example, 1 to 11, preferably 1 to 9, more preferably 1 to 7, still more preferably 1 to 4, and particularly preferably 1, 2, or 3, and the relationship (Xc/Z) between the number of bases (Xc) in the 5' side region (Xc) and the number of bases (Z) in the inner region (Z) is, for example, 1/50 to 1/2, preferably 1/40 to 1/3, and more preferably 1/30 to 1/4. In the latter

case, the number of bases (Xc) in the 5' side region (Xc) and the number of bases (Yc) in the 3' side region (Yc) satisfy the relationship of Xc > Yc. The number of bases (Yc) is, for example, 1 to 11, preferably 1 to 9, more preferably 1 to 7, still more preferably 1 to 4, and particularly preferably 1, 2, or 3, and the relationship (Yc/Z) between the number of bases (Yc) in the 3' side region (Yc) and the number of bases (Z) in the inner region (Z) is, for example, 1/50 to 1/2, preferably 1/40 to 1/3, and more preferably 1/30 to 1/4.

Preferably, the linker regions (Lx) and (Ly) each have a structure such that self-annealing is not caused inside themselves. For example, as described above, the linker regions (Lx) and (Ly) each may be composed of the nucleotide residue(s) or the non-nucleotide residue(s) or may include both of them.

When the linker regions (Lx) and (Ly) include nucleotide residues as described above, there are no particular limitations on the lengths of the linker regions (Lx) and (Ly). The length of the linker region (Lx) preferably is such that, for example, the inner 5' side region (X) and the 5' side region (Xc) can form a double strand. The length of the linker region (Ly) preferably is such that, for example, the inner 3' side region (Y) and the 3' side region (Yc) can form a double strand. The lengths of the linker regions (Lx) and (Ly) may be the same or different, for example, and also, their base sequences may be the same or different. The lower limit of the number of bases in each of the linker regions (Lx) and (Ly) is, for example, 1, preferably 2, and more preferably 3, and the upper limit of the same is, for example, 100, preferably 80, and more preferably 50, 40, 30, 20, or 10. The number of bases in each of the linker regions specifically is 1 to 50, 1 to 30, 1 to 20, 1 to 10, 1 to 7, or 1 to 4, for example, but it is not limited to these illustrative examples.

There is no particular limitation on the full length of the nucleic acid molecule of the present invention. In the nucleic acid molecule of the present invention, the lower limit of the total number of bases (the number of bases in the full length nucleic acid molecule) is, for example, 38, preferably 42, more preferably 50, still more preferably 51, and particularly preferably 52, and the upper limit of the same is, for example, 300, preferably 200, more preferably 150, still more preferably 100, and particularly preferably 80. In the nucleic acid molecule of the present invention, the lower limit of the total number of bases excluding those in the linker regions (Lx) and (Ly) is, for example, 38, preferably 42, more preferably 50, still more preferably 51, and particularly preferably 52, and the upper limit of the same is, for example, 300, preferably 200, more preferably 150, still more preferably 100, and particularly preferably 80.

In the nucleic acid molecule according to the present embodiment, for example, the 5' end and the 3' end may or may not be linked to each other. In the former case, the nucleic acid molecule according to the present embodiment is a circular single-stranded nucleic acid molecule. In the latter case, the nucleic acid molecule according to the present embodiment preferably is such that, for example, the 5' end thereof is not a phosphate group, because it allows the state where both the ends are not linked to each other to be maintained.

(2-3) Third Embodiment

The single-stranded nucleic acid molecule according to a third embodiment is a molecule in which the linker region(s) has a non-nucleotide structure. Regarding this embodiment, the disclosures in WO 2012/005368 and WO2012/017979 are incorporated herein by reference, for example.

The above descriptions as to the nucleic acid molecules according to the first and second embodiments also are applicable to the nucleic acid molecule according to the present embodiment, except that, in the nucleic acid molecule according to the present embodiment, the linker region (Lx) and/or the linker region (Ly) has a non-nucleotide structure.

The non-nucleotide structure is not particularly limited, and may be, for example, a pyrrolidine skeleton, a piperidine skeleton, polyalkylene glycol, or the like. The polyalkylene glycol may be, for example, polyethylene glycol.

The pyrrolidine skeleton may be the skeleton of a pyrrolidine derivative obtained through substitution of at least one carbon constituting the 5-membered ring of pyrrolidine, for example. In the case of substitution, it is preferable to substitute the carbon(s) other than C-2, for example. The carbon may be substituted with nitrogen, oxygen, or sulfur, for example. The pyrrolidine skeleton may contain, for example, a carbon-carbon double bond or a carbon-nitrogen double bond in, for example, the 5-membered ring of pyrrolidine. In the pyrrolidine skeleton, carbons and nitrogen constituting the 5-membered ring of pyrrolidine each may have hydrogen bound thereto, or a substituent to be described below bound thereto, for example. The linker region (Lx) may be linked to the regions (X) and (Xc), and the linker region (Ly) may be linked to the region (Y) and the region (Yc), via, for example, any group in the pyrrolidine skeleton, preferably any one carbon atom or nitrogen in the 5-membered ring, and more preferably the 2-position carbon (C-2) or nitrogen in the 5-membered ring. Examples of the pyrrolidine skeleton include proline skeletons and prolinol skeletons. The proline skeletons, the prolinol skeletons, and the like are excellent in safety because they are substances present in living organisms and reductants thereof, for example.

The piperidine skeleton may be the skeleton of a piperidine derivative obtained through substitution of at least one carbon constituting the 6-membered ring of piperidine, for example. In the case of substitution, it is preferable to substitute the carbon(s) other than C-2, for example. The carbon may be substituted with nitrogen, oxygen, or sulfur, for example. The piperidine skeleton may contain, for example, a carbon-carbon double bond or a carbon-nitrogen double bond in, for example, the 6-membered ring of piperidine. In the piperidine skeleton, carbons and nitrogen constituting the 6-membered ring of piperidine each may have a hydrogen group bound thereto, or a substituent to be described below bound thereto, for example. The linker region (Lx) may be linked to the regions (X) and (Xc), and the linker region (Ly) may be linked to the region (Y) and the region (Yc), via, for example, any group in the piperidine skeleton, preferably any one carbon atom or nitrogen in the 6-membered ring, and more preferably the 2-position carbon (C-2) or nitrogen in the 6-membered ring.

Each of the linker regions may include only the non-nucleotide residue(s) having the non-nucleotide structure, or may include the non-nucleotide residue(s) having the non-nucleotide structure and the nucleotide residue(s), for example.

The linker region is represented by the following formula (I), for example.

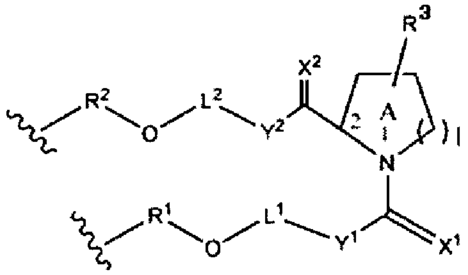

• • • ( I )

In the formula (I),

$X^1$ and $X^2$ are each independently $H_2$, O, S, or NH;

$Y^1$ and $Y^2$ are each independently a single bond, $CH_2$, NH, O, or S;

$R^3$ is a hydrogen atom or substituent that is bound to C-3, C-4, C-5, or C-6 on a ring A;

$L^1$ is an alkylene chain composed of n atoms, and a hydrogen atom(s) on an alkylene carbon atom(s) may or may not be substituted with OH, $OR^a$, $NH_2$, $NHR^a$, $NR^aR^b$, SH, or $SR^a$, or

$L^1$ is a polyether chain obtained by substituting at least one carbon atom on the alkylene chain with an oxygen atom, provided that: when $Y^1$ is NH, O, or S, an atom bound to $Y^1$ in $L^1$ is carbon, an atom bound to $OR^1$ in $L^1$ is carbon, and oxygen atoms are not adjacent to each other;

$L^2$ is an alkylene chain composed of m atoms, and a hydrogen atom(s) on an alkylene carbon atom(s) may or may not be substituted with OH, $OR^c$, $NH_2$, $NHR^c$, $NR^cR^d$, SH, or $SR^c$, or

$L^2$ is a polyether chain obtained by substituting at least one carbon atom on the alkylene chain with an oxygen atom, provided that: when $Y^2$ is NH, O, or S, an atom bound to $Y^2$ in $L^2$ is carbon, an atom bound to $OR^2$ in $L^2$ is carbon, and oxygen atoms are not adjacent to each other;

$R^a$, $R^b$, Re, and $R^d$ are each independently a substituent or a protecting group; l is 1 or 2;

m is an integer in the range from 0 to 30;

n is an integer in the range from 0 to 30;

on the ring A, one carbon atom other than C-2 may be substituted with nitrogen, oxygen, or sulfur,

the ring A may contain a carbon-carbon double bond or a carbon-nitrogen double bond;

the regions (Xc) and (X) are each linked to the linker region (Lx) via $-OR^1-$ or $-OR^2-$,

the regions (Yc) and (Y) are each linked to the linker region (Ly) via $-OR^1-$ or $-OR^2-$, and

$R^1$ and $R^2$ may or may not be present, and when they are present, $R^1$ and $R^2$ are each independently a nucleotide residue or the structure of the formula (I).

In the formula (I), $X^1$ and $X^2$ are each independently $H_2$, O, S, or NH, for example. In the formula (I), "$X^1$ is $H_2$" means that $X^1$ forms $CH_2$ (a methylene group) together with a carbon atom to which $X^1$ binds. The same applies to $X^2$.

In the formula (I), $Y^1$ and $Y^2$ are each independently a single bond, $CH_2$, NH, O, or S.

In the formula (I), 1 in the ring A is 1 or 2. When l=1, the ring A is a 5-membered ring, which is, for example, the pyrrolidine skeleton. The pyrrolidine skeleton is, for example, a proline skeleton, a prolinol skeleton, or the like, and specific examples include divalent structures of the proline skeleton and the prolinol skeleton. When l=2, the ring A is a 6-membered ring, which is, for example, the piperidine skeleton. On the ring A, one carbon atom other than C-2 may be substituted with nitrogen, oxygen, or sulfur. Furthermore, the ring A may contain a carbon-carbon double bond or a carbon-nitrogen double bond. The ring A may be in either L-form or D-form, for example.

In the formula (I), $R^3$ is a hydrogen atom or a substituent that is bound to C-3, C-4, C-5, or C-6 on the ring A. When $R^3$ is the substituent, there may be one substituent $R^3$, two or more substituents $R^3$, or no substituent $R^3$, and when there are a plurality of substituents $R^3$, they may be the same or different.

The substituent $R^3$ is, for example, a halogen, OH, $OR^4$, $NH_2$, $NHR^4$, $NR^4R^5$, SH, $SR^4$, an oxo group (=O), or

the like.

$R^4$ and $R^5$ are each independently a substituent or a protecting group, and they may be the same or different. Examples of the substituent include halogens, alkyls, alkenyls, alkynyls, haloalkyls, aryls, heteroaryls, arylalkyls, cycloalkyls, cycloalkenyls, cycloalkylalkyls, cyclylalkyls, hydroxyalkyls, alkoxyalkyls, aminoalkyls, heterocyclylalkenyls, heterocyclylalkyls, heteroarylalkyls, silyls, and silyloxyalkyls. The same applies hereinafter. Examples of the substituent $R^3$ include the above-listed substituents.

The protecting group is a functional group that inactivates a highly reactive functional group, for example. Examples of the protecting group include known protecting groups. Regarding the protecting group, the description in the literature (J. F. W. McOmie, "Protecting Groups in Organic Chemistry", Plenum Press, London and New York, 1973) is incorporated herein by reference, for example. There is no particular limitation on the protecting group, and examples thereof include a tert-butyldimethylsilyl group (TBDMS), a bis(2-acetoxyethyloxy)methyl group (ACE), a triisopropylsilyloxymethyl group (TOM), a 1-(2-cyanoethoxy)ethyl group (CEE), a 2-cyanoethoxymethyl group (CEM), a tolylsulfonylethoxymethyl group (TEM), and a dimethoxytrityl group (DMTr). When $R^3$ is $OR^4$, there is no particular limitation on the protecting group, and examples thereof include a TBDMS group, an ACE group, a TOM group, a CEE group, a CEM group, and a TEM group. Other examples of the protecting group include silyl-containing groups. The same applies hereinafter.

In the formula (I), $L^1$ is an alkylene chain composed of n atoms. A hydrogen atom(s) on the alkylene carbon atom(s) may or may not be substituted with OH, $OR^a$, $NH_2$, $NHR^a$, $NR^aR^b$, SH, or $SR^a$, for example. Alternatively, $L^1$ may be a polyether chain obtained by substituting at least one carbon atom on the alkylene chain with an oxygen atom. The polyether chain is, for example, polyethylene glycol. When $Y^1$ is NH, O, or S, an atom bound to $Y^1$ in $L^1$ is carbon, an atom bound to $OR^1$ in $L^1$ is carbon, and oxygen atoms are not adjacent to each other. That is, for example, when $Y^1$ is O, this oxygen atom and the oxygen atom in $L^1$ are not adjacent to each other, and the oxygen atom in $OR^1$ and the oxygen atom in $L^1$ are not adjacent to each other.

In the formula (I), $L^2$ is an alkylene chain composed of m atoms. A hydrogen atom(s) on the alkylene carbon atom(s) may or may not be substituted with OH, $OR^c$, $NH_2$, $NHR^c$, $NR^cR^d$, SH, or $SR^c$, for example. Alternatively, $L^2$ may be a polyether chain obtained by substituting at least one carbon atom on the alkylene chain with an oxygen atom. When $Y^2$ is NH, O, or S, an atom bound to $Y^2$ in $L^2$ is carbon, an atom bound to $OR^2$ in $L^2$ is carbon, and oxygen atoms are not adjacent to each other. That is, for example, when $Y^2$ is O, this oxygen atom and the oxygen atom in $L^2$ are not adjacent to each other, and the oxygen atom in $OR^2$ and the oxygen atom in $L^2$ are not adjacent to each other.

There are no particular limitations on n of $L^1$ and m of $L^2$, and the lower limit of each of them may be 0, for example, and there is no particular limitation on the upper limit of the same. n and m can be set as appropriate depending on a desired length of the linker region (Lx) or (Ly), for example. For example, from the viewpoint of manufacturing cost, yield, and the like, each of n and m preferably is 0 to 30, more preferably 0 to 20, and still more preferably 0 to 15. n and m may be the same (n = m) or different. n + m is, for example, 0 to 30, preferably 0 to 20, and more preferably 0 to 15.

$R^a$, $R^b$, $R^c$, and $R^d$ are each independently a substituent or a protecting group, for example. The substituent and the protecting group are the same as described above, for example.

In the formula (I), hydrogen atoms each independently may be substituted with a halogen such as Cl, Br, F, or I, for example.

The regions (Xc) and (X) are linked to the linker region (Lx) and the regions (Yc) and (Y) are linked to the linker region (Ly), via $-OR^1-$ or $-OR^2-$, for example. $R^1$ and $R^2$ may or may not be present. When $R^1$ and $R^2$ are present, $R^1$ and $R^2$ are each independently a nucleotide residue or the structure represented by the formula (I). When $R^1$ and/or $R^2$ is the nucleotide residue, the linker regions (Lx) and (Ly) are each composed of the non-nucleotide residue having the structure of the formula (I) excluding the nucleotide residue $R^1$ and/or $R^2$, and the nucleotide residue(s), for example. When $R^1$ and/or $R^2$ is the structure represented by the formula (I), the structures of the linker regions (Lx) and (Ly) are such that, for example, two or more of the non-nucleotide residues having the structure of the formula (I) are linked to each other. The number of the structures of the formula (I) may be 1, 2, 3, or 4, for example. When the linker region (Lx) includes a plurality of the structures, the structures of the formula (I) may be linked either directly or via the nucleotide residues, for example. On the other hand, when $R^1$ and $R^2$ are not present, the linker regions (Lx) and (Ly) are each composed of the non-nucleotide residue(s) having the structure of the formula (I) only, for example.

The combination of the regions (Xc) and (X) with $-OR^1-$ and $-OR^2-$, and the combination of the regions (Yc) and (Y) with $-OR^1-$ and $-OR^2-$ are not particularly limited, and may be, for example, any of the following conditions.

Condition (1):

The regions (Xc) and (X) are linked to the structure of the formula (I) via $-OR^2-$ and $-OR^1-$, respectively; and the regions (Yc) and (Y) are linked to the structure of the formula (I) via $-OR^1-$ and $-OR^2-$, respectively.

Condition (2):

The regions (Xc) and (X) are linked to the structure of the formula (I) via $-OR^2-$ and $-OR^1-$, respectively; and the regions (Yc) and (Y) are linked to the structure of the formula (I) via $-OR^2-$ and $-OR^1-$, respectively.

Condition (3):

The regions (Xc) and (X) are linked to the structure of the formula (I) via $-OR^1-$ and $-OR^2-$, respectively; and the regions (Yc) and (Y) are linked to the structure of the formula (I) via $-OR^1-$ and $-OR^2-$, respectively.

Condition (4):

The regions (Xc) and (X) are linked to the structure of the formula (I) via $-OR^1-$ and $-OR^2-$, respectively; and the regions (Yc) and (Y) are linked to the structure of the formula (I) via $-OR^2-$ and $-OR^1-$, respectively.

Examples of the structure of the formula (I) include the structures of the following formulae (I-1) to (I-9). In the following formulae, n and m are the same as in the formula (I). In the following formulae, q is an integer from 0 to 10.

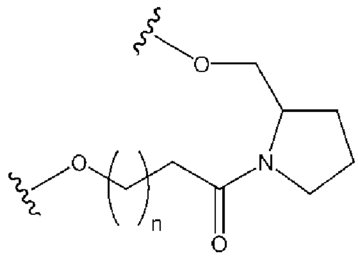

· · · (I-1)

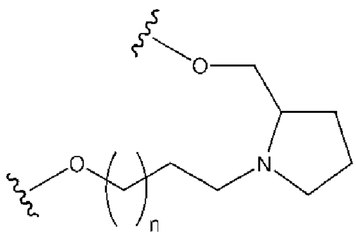

· · · (I-2)

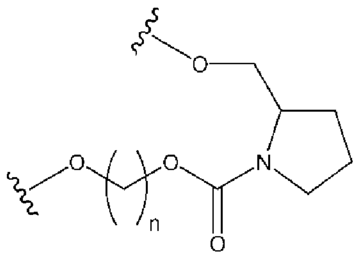

· · · (I-3)

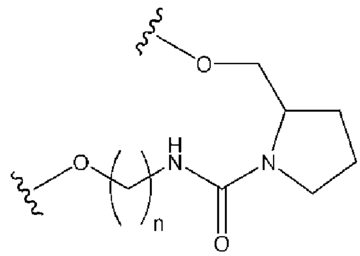

· · · (I-4)

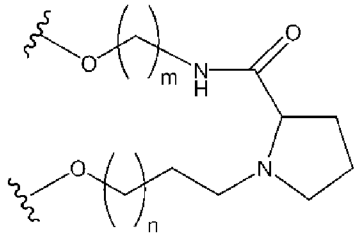

· · · (I-5)

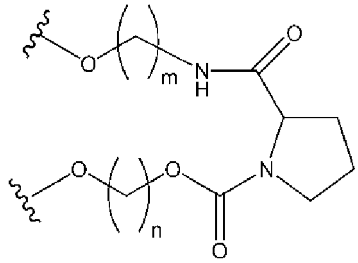

· · · (I-6)

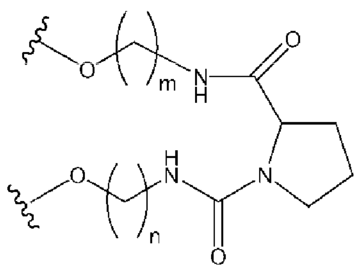

· · · (I-7)

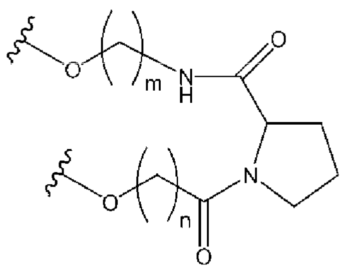

· · · (I-8)

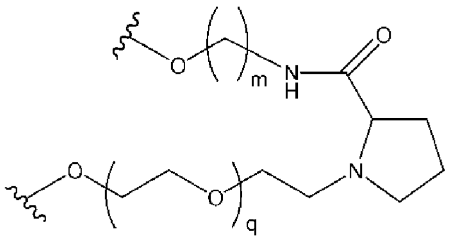

・・・(I 9)

In the formulae (I-1) to (I-9), n, m, and q are not particularly limited and are as described above. Specific examples are as follows: in the formula (I-1), n = 8; in the formula (I-2), n = 3; in the formula (I-3), n = 4 or 8; in the formula (I-4), n = 7 or 8; in the formula (I-5), n = 3 and m = 4; in the formula (I-6), n = 8 and m = 4; in the formula (I-7), n = 8 and m = 4; in the formula (I-8), n = 5 and m = 4; and in the formula (I-9), q = 1 and m = 4. The following formula (I-4a) shows an example of the formula (I-4) (n = 8), and the following formula (I-8a) shows an example of the formula (I-8) (n = 5, m = 4).

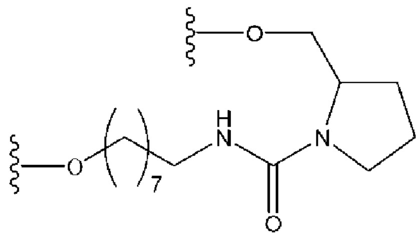

・・・(I－4a)

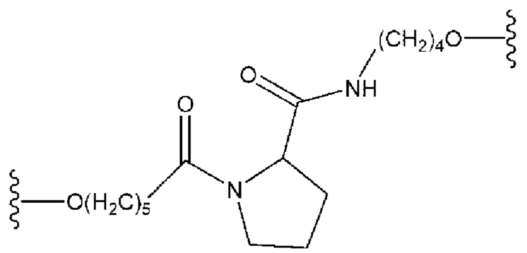

・・・(I－8a)

In the nucleic acid molecule, regions other than the linkers preferably are composed of the nucleotide residue(s). Each of the regions is composed of any of the following residues (1) to (3), for example.

(1) an unmodified nucleotide residue(s)
(2) a modified nucleotide residue(s)
(3) an unmodified nucleotide residue(s) and a modified nucleotide residue(s)

In the nucleic acid molecule, there is no particular limitation on the building block of the linker region, and examples thereof include the nucleotide residue and the non-nucleotide residue. The linker region may be composed of the nucleotide residue only, may be composed of the non-nucleotide residue only, or may be composed of the nucleotide residue and the non-nucleotide residue, for example. The linker region is composed of the following residues (1) to (7), for example.

(1) unmodified nucleotide residue
(2) modified nucleotide residue
(3) unmodified nucleotide residue and modified nucleotide residue
(4) non-nucleotide residue
(5) non-nucleotide residue and unmodified nucleotide residue
(6) non-nucleotide residue and modified nucleotide residue
(7) non-nucleotide residue, unmodified nucleotide residue, and modified nucleotide residue

When the nucleic acid molecule of the present invention has both the linker regions (Lx) and (Ly), the building blocks of both the regions may be the same or different, for example. Specific examples are as follows: the building

blocks of both the regions are the nucleotide residues; the building blocks of both the regions are the non-nucleotide residues; and the building blocks of one of the regions is the nucleotide residue(s) while the component of the other linker region is the non-nucleotide residue(s).

As specific examples of the single-stranded nucleic acid molecule, a nucleic acid molecule according to the second embodiment (hereinafter also referred to as NK) and a nucleic acid molecule according to the third embodiment (hereinafter also referred to as PK) are shown below.

Specific examples of the NK are shown below. The sequence of each NK is shown in a direction from the 5' end to the 3' end. A boxed region on the 5' side is the linker (Lx), a boxed region on the 3' side is the linker (Lx), and an underlined part is the as nucleotide. In each sequence, the sequences of the linkers (Lx) and (Ly) are not particularly limited, and preferably are such that annealing is not caused inside each region. In other words, the sequences of the linkers (Lx) and (Ly) preferably are sequences that allow loop formation. Regarding each NK, a sequence in which the linkers (Lx) and (Ly) are represented by arbitrary bases (n) and a sequence in which the linkers (Lx) and (Ly) are represented by specific bases are given as examples. n is, for example, a, c, g, or u (the same applies hereinafter). It is to be noted, however, that they are merely illustrative and do not limit the present invention by any means.

[Table 3]

NK－０１４４

（SEQ ID NO:８３）

AGCACCAAAAAGAAAUACUUUUCCnnnnnnnGGAAAAGUAUUUCUUUUUGGUGCUUCnnnnG

（SEQ ID NO:８４）

AGCACCAAAAAGAAAUACUUUUCCCCACACCGGAAAAGUAUUUCUUUUUGGUGCUUCUUCGG

NK－０１４５

（SEQ ID NO:８５）

AUCCUGAUUCUGCCAAACAAUUCCnnnnnnnGGAAUUGUUUGGCAGAAUCAGGAUUCnnnnG

（SEQ ID NO:８６）

AUCCUGAUUCUGCCAAACAAUUCCCCACACCGGAAUUGUUUGGCAGAAUCAGGAUUCUUCGG

NK－０１４６

（SEQ ID NO:８７）

ACUGCCAAACAAGUUAUUGAUUCCnnnnnnnGGAAUCAAUAACUUGUUUGGCAGUUCnnnnG

（SEQ ID NO:８８）

ACUGCCAAACAAGUUAUUGAUUCCCCACACCGGAAUCAAUAACUUGUUUGGCAGUUCUUCGG

NK－０１４７

（SEQ ID NO:８９）

ACGGUGACAGUAUAACAGUAAACCnnnnnnnGGUUUACUGUUAUACUGUCACCGUCCnnnnG

（SEQ ID NO:９０）

ACGGUGACAGUAUAACAGUAAACCCCACACCGGUUUACUGUUAUACUGUCACCGUCCUUCGG

NK－０１４８

（SEQ ID NO:９１）

UGUCAUUGACCGUGUGCUUACACCnnnnnnnGGUGUAAGCACACGGUCAAUGACAUCnnnnG

（SEQ ID NO:９２）

UGUCAUUGACCGUGUGCUUACACCCCACACCGGUGUAAGCACACGGUCAAUGACAUCUUCGG

Specific examples of the PK are shown below. The sequence of each PK is shown in a direction from the 5' end to the 3' end. A boxed region on the 5' side is the linker (Lx), a boxed region on the 3' side is the linker (Lx), and an underlined part is the as nucleotide. In each sequence, there are no particular limitations on the structures of the linkers (Lx) and (Ly), and examples thereof include the structures such as the above-described pyrrolidine skeleton and piperidine skeleton. It is to be noted, however, that they are merely illustrative and do not limit the present invention by any means.

[Table 4]

ＰＫ－００７６

（SEQ ID NO:９３）

AGCACCAAAAAGAAAUACUUUUCC-Lx-GGAAAAGUAUUUCUUUUUGGUGCUUC-Ly-G

ＰＫ－００７７

（SEQ ID NO:９４）

AUCCUGAUUCUGCCAAACAAUUCC-Lx-GGAAUUGUUUGGCAGAAUCAGGAUUC-Ly-G

ＰＫ－００７８

（SEQ ID NO:９５）

ACUGCCAAACAAGUUAUUGAUUCC-Lx-GGAAUCAAUAACUUGUUUGGCAGUUC-Ly-G

ＰＫ－００７９

（SEQ ID NO:９６）

ACGGUGACAGUAUAACAGUAAACC-Lx-GGUUUACUGUUAUACUGUCACCGUCC-Ly-G

ＰＫ－００８０

（SEQ ID NO:９７）

UGUCAUUGACCGUGUGCUUACACC-Lx-GGUGUAAGCACACGGUCAAUGACAUC-Ly-G

Regarding NK-0144 (SEQ ID NO: 84), NK-0145 (SEQ ID NO: 86), NK-0146 (SEQ ID NO: 88), NK-0147 (SEQ ID NO: 90), and NK-0148 (SEQ ID NO: 92), and PK-0076 (SEQ ID NO: 93), PK-0077 (SEQ ID NO: 94), PK-0078 (SEQ ID NO: 95), PK-0079 (SEQ ID NO: 96), and PK-0080 (SEQ ID NO: 97), the state of stem formation and loop formation is shown below. In the following sequences, the arrow indicates that the 5' end and the 3' end are not linked to each other, and "5'" indicates the 5' end. In the following sequences, the underlined part is the as nucleotide.

[Table 5]

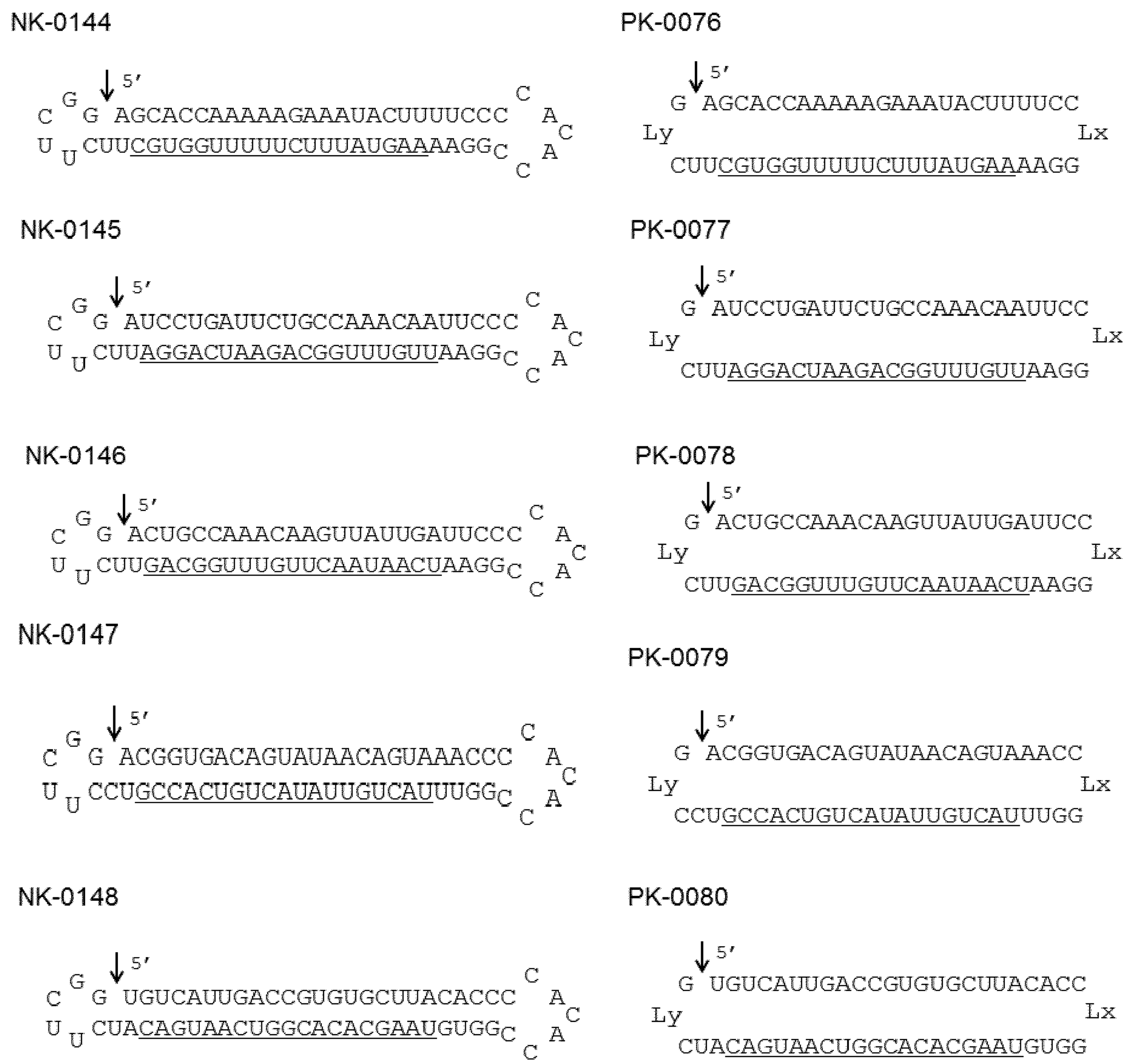


The kinds of as nucleotide in the NKs and PKs are shown below.

[Table 6]

| NK | PK | as nucleotide |
|---|---|---|
| NK-0144 SEQ ID NO: 84 | PK-0076 SEQ ID NO: 93 | NI-0079 SEQ ID NO: 1 |
| NK-0145 SEQ ID NO: 86 | PK-0077 SEQ ID NO: 94 | NI-0083 SEQ ID NO: 5 |
| NK-0146 SEQ ID NO: 88 | PK-0078 SEQ ID NO: 95 | NI-0084 SEQ ID NO: 6 |
| NK-0147 SEQ ID NO: 90 | PK-0079 SEQ ID NO: 96 | NI-0092 SEQ ID NO: 12 |
| NK-0148 SEQ ID NO: 92 | PK-0080 SEQ ID NO: 97 | NI-0093 SEQ ID NO: 13 |

Examples of the nucleic acid molecule of the present invention include molecules composed of the nucleotide residues only and molecules including the non-nucleotide residue(s) in addition to the nucleotide residue(s). In the nucleic acid molecule of the present invention, the nucleotide residues may be the unmodified nucleotide residues only; the modified nucleotide residues only; or both the unmodified nucleotide residue(s) and the modified nucleotide residue(s), as described above, for example. When the nucleic acid molecule includes both the unmodified nucleotide residue(s)

and the modified nucleotide residue(s), the number of the modified nucleotide residue(s) is not particularly limited, and is, for example, "one to several", specifically, for example, 1 to 5, preferably 1 to 4, more preferably 1 to 3, and most preferably 1 or 2. When the nucleic acid molecule of the present invention includes the non-nucleotide residue(s), the number of the non-nucleotide residue(s) is not particularly limited, and is, for example, "one to several", specifically, for example, 1 to 8, 1 to 6, 1 to 4, or 1, 2, or 3.

When the nucleic acid molecule includes the modified ribonucleotide residue(s) in addition to the unmodified ribonucleotide residues, for example, the number of the modified ribonucleotide residue(s) is not particularly limited, and is, for example, "one to several", specifically, for example, 1 to 5, preferably 1 to 4, more preferably 1 to 3, and most preferably 1 or 2. The modified ribonucleotide residue as contrasted to the unmodified ribonucleotide residue may be the deoxyribonucleotide residue obtained by substituting a ribose residue with a deoxyribose residue, for example. When the nucleic acid molecule includes the deoxyribonucleotide residue(s) in addition to the unmodified ribonucleotide residues, for example, the number of the deoxyribonucleotide residue(s) is not particularly limited, and is, for example, "one to several", specifically, for example, 1 to 5, preferably 1 to 4, more preferably 1 to 3, and most preferably 1 or 2.

The nucleotide residue includes, as its components, a sugar, a base, and a phosphate. The nucleotide residue may be, for example, a ribonucleotide residue or a deoxyribonucleotide residue, as described above. The ribonucleotide residue has, for example: a ribose residue as the sugar; and adenine (A), guanine (G), cytosine (C), or uracil (U) as the base. The deoxyribose residue has, for example: a deoxyribose residue as the sugar; and adenine (A), guanine (G), cytosine (C), or thymine (T) as the base.

The nucleotide residue may be, for example, an unmodified nucleotide residue or a modified nucleotide residue. The components of the unmodified nucleotide residue are the same or substantially the same as the components of a naturally occurring nucleotide residue, for example. Preferably, the components of the unmodified nucleotide residue are the same or substantially the same as the components of a nucleotide residue occurring naturally in a human body.

The modified nucleotide residue is a nucleotide residue obtained by modifying the unmodified nucleotide residue, for example. The modified nucleotide residue may be such that any of the components of the unmodified nucleotide residue is modified, for example. In the present invention, "modification" means, for example: substitution, addition, and/or deletion of any of the components; and substitution, addition, and/or deletion of an atom(s) and/or a functional group(s) in the component(s). It also can be referred to as "alteration". Examples of the modified nucleotide residue include naturally occurring nucleotide residues and artificially-modified nucleotide residues. Regarding the naturally derived modified nucleotide residues, Limbach et al. (1994, Summary: the modified nucleosides of RNA, Nucleic Acids Res. 22: pp. 2183 to 2196) can be referred to, for example. The modified nucleotide residue may be a residue of an alternative of the nucleotide, for example.

Examples of the modification of the nucleotide residue include modification of a ribose-phosphate backbone (hereinafter referred to as a "ribophosphate backbone")

In the ribophosphate backbone, a ribose residue can be modified, for example. In the ribose residue, for example, the 2'-position carbon can be modified. Specifically, a hydroxyl group bound to the 2'-position carbon can be substituted with hydrogen or a halogen such as fluoro, for example. By substituting the hydroxyl group bound to the 2'-position carbon with hydrogen, it is possible to substitute the ribose residue with deoxyribose. The ribose residue can be substituted with its stereoisomer, for example, and may be substituted with an arabinose residue, for example.

The ribophosphate backbone may be substituted with a non-ribophosphate backbone having a non-ribose residue and/or a non-phosphate, for example. The non-ribophosphate backbone may be, for example, the ribophosphate backbone modified so as to be uncharged. Examples of an alternative obtained by substituting the ribophosphate backbone with the non-ribophosphate backbone in the nucleotide include morpholino, cyclobutyl, and pyrrolidine. Other examples of the alternative include artificial nucleic acid monomer residues. Specific examples thereof include PNA (Peptide Nucleic Acid), LNA (Locked Nucleic Acid), and ENA (2'-0,4'-C-Ethylenebridged Nucleic Acid). Among them, PNA is preferable.

In the ribophosphate backbone, a phosphate group can be modified, for example. In the ribophosphate backbone, a phosphate group in the closest proximity to the sugar residue is called an "$\alpha$-phosphate group". The $\alpha$-phosphate group is charged negatively, and the electric charges are distributed evenly over two oxygen atoms that are not linked to the sugar residue. Among the four oxygen atoms in the $\alpha$-phosphate group, the two oxygen atoms that are not linked to the sugar residue in the phosphodiester linkage between the nucleotide residues hereinafter are referred to as "non-linking oxygens". On the other hand, two oxygen atoms that are linked to the sugar residue in the phosphodiester linkage between the nucleotide residues hereinafter are referred to as "linking oxygens". The $\alpha$-phosphate group preferably is modified so as to be uncharged, or so as to render the charge distribution between the non-linking oxygens asymmetric, for example.

In the phosphate group, the non-linking oxygen(s) may be substituted, for example. The oxygen(s) can be substituted with any atom selected from S (sulfur), Se (selenium), B (boron), C (carbon), H (hydrogen), N (nitrogen), and OR (R is an alkyl group or an aryl group), for example, and substitution with S is preferable. It is preferable that both the non-linking oxygens are substituted, for example, and it is more preferable that both the non-linking oxygens are

substituted with S. Examples of the thus-modified phosphate group include phosphorothioates, phosphorodithioates, phosphoroselenates, borano phosphates, borano phosphate esters, hydrogen phosphonates, phosphoroamidates, alkyl or aryl phosphonates, and phosphotriesters. In particular, phosphorodithioate in which both the two non-linking oxygens are substituted with S is preferable.

In the phosphate group, the linking oxygen(s) may be substituted, for example. The oxygen(s) can be substituted with any atom selected from S (sulfur), C (carbon), and N (nitrogen), for example. Examples of the thus-modified phosphate group include: bridged phosphoroamidates resulting from the substitution with N; bridged phosphorothioates resulting from the substitution with S; and bridged methylenephosphonates resulting from the substitution with C. Preferably, substitution of the linking oxygen(s) is performed in at least one of the 5' end nucleotide residue and the 3' end nucleotide residue of the nucleic acid molecule of the present invention, for example. When the substitution is performed on the 5' side, substitution with C is preferable. When the substitution is performed on the 3' side, substitution with N is preferable.

The phosphate group may be substituted with the phosphate-free linker, for example. Examples of the linker include siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo, and methyleneoxymethylimino. Preferable examples of the linker include a methylene carbonyl amino group and a methylenemethylimino group.

In the nucleic acid molecule of the present invention, for example, at least one of a nucleotide residue at the 3' end and a nucleotide residue at the 5' end may be modified. The nucleotide residue at either one of the 3' end and the 5' end may be modified, or the nucleotide residues at both the 3' end and the 5' end may be modified, for example. The modification may be as described above, for example, and it is preferable to modify a phosphate group(s) at the end(s). The entire phosphate group may be modified, or one or more atoms in the phosphate group may be modified, for example. In the former case, for example, the entire phosphate group may be substituted or deleted.

Modification of the nucleotide residue(s) at the end(s) may be the addition of any other molecule, for example. Examples of the other molecule include functional molecules such as labeling substances and protecting groups to be described below. Examples of the protecting groups include S (sulfur), Si (silicon), B (boron), and ester-containing groups. The functional molecules such as the labeling substances can be used in the detection and the like of the nucleic acid molecule of the present invention, for example.

The other molecule may be added to the phosphate group of the nucleotide residue, or may be added to the phosphate group or the sugar residue via a spacer, for example. The terminal atom of the spacer can be added to or can substitute for either one of the linking oxygens of the phosphate group, or O, N, S, or C of the sugar residue, for example. The binding site in the sugar residue preferably is, for example, C at the 3'-position, C at the 5'-position, or any atom bound thereto. The spacer also can be added to or can substitute for a terminal atom of the nucleotide alternative such as PNA, for example.

There is no particular limitation on the spacer, and examples thereof include $-(CH_2)_n-$, $-(CH_2)_nN-$, $-(CH_2)_nO-$, $-(CH_2)_nS-$, $O(CH_2CH_2O)_nCH_2CH_2OH$, abasic sugars, amide, carboxy, amine, oxyamine, oxyimine, thioether, disulfide, thiourea, sulfonamide, and morpholino, and also biotin reagents and fluorescein reagents. In the above formulae, n is a positive integer, and n = 3 or 6 is preferable.

Other examples of the molecule to be added to the end include dyes, intercalating agents (e.g., acridines), crosslinking agents (e.g., psoralen, mitomycin C), porphyrins (TPPC4, texaphyrin, sapphyrine), polycyclic aromatic hydrocarbons (e.g., phenazine, dihydrophenazine), artificial endonucleases (e.g., EDTA), lipophilic carriers (e.g., cholesterol, cholic acid, adamantane acetic acid, 1-pyrene butyric acid, dihydrotestosterone, 1,3-Bis-O (hexadecyl)glycerol, a geranyloxyhexyl group, hexadecylglycerol, borneol, menthol, 1,3-propanediol, a heptadecyl group, palmitic acid, myristic acid, O3-(oleoyl)lithochol acid, O3-(oleoyl)cholic acid, dimethoxytrityl, and phenoxazine), peptide complexes (e.g., Antennapedia peptide, Tat peptide), alkylating agents, phosphate, amino, mercapto, PEG (e.g., PEG-40K), MPEG, $[MPEG]_2$, polyamino, alkyl, substituted alkyl, radiolabeled markers, enzymes, haptens (e.g., biotin), transport/absorption promoters (e.g., aspirin, vitamin E, folic acid), and synthetic ribonucleases (e.g., imidazole, bisimidazole, histamine, imidazole clusters, acridine-imidazole complexes, $Eu^{3+}$ complexes of tetraazamacrocycles).

In the nucleic acid molecule of the present invention, the 5' end may be modified with a phosphate group or a phosphate group analog, for example. Examples of the phosphate group include:

5'-monophosphate ($(HO)_2(O)P-O-5'$);
5'-diphosphate ($(HO)_2(O)P-O-P(HO)(O)-O-5'$);
5'-triphosphate ($(HO)_2(O)P-O-(HO)(O)P-O-P(HO)(O)-O-5'$);
5'-guanosine cap (7-methylated or non-methylated, 7m-G-O-5'-(HO)(O)P-O-(HO)(O)P-O-P(HO)(O)-O-5');
5'-adenosine cap (Appp);
any modified or unmodified nucleotide cap structure (N-O-5'-(HO)(O)P-O-(HO)(O)P-O-P(HO)(O)-O-5');
5'-monothiophosphate (phosphorothioate: $(HO)_2(S)P-O-5'$);

5'-monodithiophosphate (phosphorodithioate: (HO)(HS)(S)P-O-5');
5'-phosphorothiolate ($(HO)_2(O)P$-S-5');
sulfur substituted monophosphate, diphosphate, and triphosphates (e.g., 5'-α-thiotriphosphate, 5'-γ- thiotriphosphate, and the like);
5'-phosphoramidates ($(HO)_2(O)P$-NH-5', (HO)($NH_2$)(O)P-O-5');
5'-alkylphosphonates (e.g., RP(OH)(O)-O-5', $(OH)_2(O)P$-5'-$CH_2$, where R is alkyl (e.g., methyl, ethyl, isopropyl, propyl, or the like)); and
5'-alkyletherphosphonates (e.g., RP(OH)(O)-O-5', where R is alkylether (e.g., methoxymethyl, ethoxymethyl, or the like)).

In the nucleotide residue, there is no particular limitation on the base. The base may be a natural base or a non-natural base, for example. The base may be a naturally derived base or a synthetic base, for example. As the base, a general base, a modified analog thereof, and the like can be used, for example.

Examples of the base include: purine bases such as adenine and guanine; and pyrimidine bases such as cytosine, uracil, and thymine. Other examples of the base include inosine, thymine, xanthine, hypoxanthine, nubularine, isoguanisine, and tubercidine. Examples of the base also include: alkyl derivatives such as 2-aminoadenine and 6-methylated purine; alkyl derivatives such as 2-propylated purine; 5-halouracil and 5-halocytosine; 5-propynyl uracil and 5-propynyl cytosine; 6-azo uracil, 6-azo cytosine, and 6-azo thymine; 5-uracil (pseudouracil), 4-thiouracil, 5-halouracil, 5-(2-aminopropyl)uracil, 5-amino allyl uracil; 8-halogenated, aminated, thiolated, thioalkylated, hydroxylated, and other 8-substituted purines; 5-trifluoromethylated and other 5-substituted pyrimidines; 7-methylguanine; 5-substituted pyrimidines; 6-azapyrimidines; N-2, N-6, and O-6 substituted purines (including 2-aminopropyladenine); 5-propynyluracil and 5-propynylcytosine; dihydrouracil; 3-deaza-5-azacytosine; 2-aminopurine; 5-alkyluracil; 7-alkylguanine; 5-alkylcytosine; 7-deazaadenine; N6,N6-dimethyladenine; 2,6-diaminopurine; 5-amino-allyl-uracil; N3-methyluracil; substituted 1,2,4-triazoles; 2-pyridinone; 5-nitroindole; 3-nitropyrrole; 5-methoxyuracil; uracil-5-oxyacetic acid; 5-methoxycarbonylmethyluracil; 5-methyl-2-thiouracil; 5-methoxycarbonylmethyl-2-thiouracil; 5-methylaminomethyl-2-thiouracil; 3-(3-amino-3-carboxypropyl)uracil; 3-methylcytosine; 5-methylcytosine; N4-acetylcytosine; 2-thiocytosine; N6-methyladenine; N6-isopentyladenine; 2-methylthio-N6-isopentenyladenine; N-methylguanine; and O-alkylated bases. Examples of the purines and pyrimidines include those disclosed in U.S. Patent No. 3,687,808, "Concise Encyclopedia of Polymer Science and Engineering", pp. 858 to 859, edited by Kroschwitz J. I, John Wiley & Sons, 1990, and Englisch et al., Angewandte Chemie, International Edition, 1991, vol. 30, p. 613.

Other examples of the modified nucleotide residue include those having no base, i.e., those having an abasic ribophosphate backbone. Furthermore, as the modified nucleotide residue, those described in U.S. Provisional Application 60/465,665 (filing date: April 25, 2003) and International Application No. PCT/US04/07070 (filing date: March 8, 2004) can be used, for example, and these documents are incorporated herein by reference.

The nucleic acid molecule of the present invention may include a labeling substance, and may be labeled with the labeling substance, for example. The labeling substance is not particularly limited and may be a fluorescent substance, a dye, an isotope, or the like, for example. Examples of the fluorescent substance include: fluorophores such as pyrene, TAMRA, fluorescein, a Cy3 dye, and a Cy5 dye. Examples of the dye include Alexa dyes such as Alexa 488. Examples of the isotope include stable isotopes and radioisotopes. Among them, stable isotopes are preferable. Stable isotopes have a low risk of radiation exposure, and they require no dedicated facilities, for example. Thus, stable isotopes are excellent in handleability and can contribute to cost reduction. Moreover, a stable isotope does not change the physical properties of a compound labeled therewith, for example, and thus has an excellent property as a tracer. The stable isotope is not particularly limited, and examples thereof include $^{2}H$, $^{13}C$, $^{15}N$, $^{17}O$, $^{18}O$, $^{33}S$, $^{34}S$, and $^{36}S$.

The nucleic acid molecule of the present invention can inhibit the expression of the periostin gene as described above. Thus, the nucleic acid molecule of the present invention can be used as, for example, a therapeutic drug for a disease caused by the expression of periostin except for an eye disease. In the present invention, the term "treatment" encompasses, for example: prevention of the diseases; improvement of the diseases; and improvement in prognosis of the diseases, and it can mean any of them.

There is no particular limitation on the diseases caused by the expression of periostin except for an eye disease, and examples thereof include skin diseases, respiratory diseases, and gastrointestinal diseases. Examples of the skin diseases include atopic dermatitis, wounds, psoriasis, scleroderma, keloids, hypertrophic scars, and melanoma. Examples of the respiratory diseases include bronchial asthma, idiopathic interstitial pneumonia, and non-idiopathic interstitial pneumonia. Examples of the gastrointestinal diseases include cholangiocarcinoma and the like.

The method of using the nucleic acid molecule of the present invention is not particularly limited. For example, the nucleic acid molecule may be administered to an administration target.

Examples of the administration target include cells, tissues, and organs. Examples of the administration target also include humans and non-human animals excluding humans. Examples of the non-human animals include non-human mammals such as mice, rats, rabbits, sheep, cows, horses, and dogs. The administration may be performed in

vivo or in vitro, for example.

The cells are not particularly limited, and may be, for example, human and murine cells isolated from living organisms. Examples of such cells include: various cultured cells including cultured cells of retinal pigment epithelial cells such as ARPE-19 and cultured cells of fibroblast and the like such as NIH3T3; stem cells such as ES cells and hematopoietic stem cells; and primary cultured cells. The cells exclude, for example, human fertilized eggs and cells that are present in human embryos and human individuals.

As to the nucleic acid molecule of the present invention, the following description regarding the treatment method for a disease caused by the expression of periostin except for an eye disease according to the present invention can be referred, for example.

Because the nucleic acid molecule of the present invention can inhibit the expression of the periostin gene as described above, it is useful as, for example, a drug for a disease caused by the expression of periostin except for an eye disease.

<Expression Vector>

An expression vector of the present invention is characterized in that it contains DNA encoding the nucleic acid molecule of the present invention. The expression vector of the present invention is characterized in that it contains the above-described DNA, and other configurations are by no means limited. The expression vector of the present invention is, for example, a vector to which the above-described DNA has been inserted in such a manner that it can be expressed. There is no particular limitation on the vector to which the DNA is to be inserted. For example, it is possible to use any commonly used vector, which may be a viral vector or a non-viral vector.

Examples of the non-viral vector include plasmid vectors.

According to the vector of the present invention, for example, by administering the vector in vivo or in vitro, it is possible to express the expression inhibitory nucleic acid molecule of the present invention in a target to which the vector has been administered.

According to the present invention, for example, by administering the expression inhibitory nucleic acid molecule to a patient with a disease caused by the expression of periostin except for an eye disease, it is possible to treat the disease by inhibiting the expression of the periostin gene. The disease caused by the expression of periostin except for an eye disease is, for example, as described above.

There is no particular limitation on the administration method, and examples thereof include parenteral administration and oral administration. Examples of the parenteral administration include transdermal administration, local administration, and intravenous administration. The administration site for the skin disease may be, for example, skin, or the like. When the drug is administered to skin, examples of the administration method include application, injection, and patching. There are no particular limitations on the administration conditions (e.g., the frequency of administration, the dose, etc.) of the drug for disease according to the present invention.

There is no particular limitation on the form of the drug for disease according to the present invention, and examples thereof include an ointment, a patch, an injection solution, and an intravenous drip solution.

In the drug for disease of the present invention, there is no particular limitation on the blended amount of the nucleic acid molecule. There is no particular limitation on the administration conditions of the nucleic acid molecule. When the nucleic acid molecule is applied to skin, the dose to be administered at one time (total amount) with respect to, for example, the skin area of 100 $cm^2$ of a human adult male is, for example, 0.01 to 10000 $\mu g$, preferably 0.1 to 100 $\mu g$, and the frequency of administration is, for example, once a day to a week. In the drug for disease of the present invention, it is preferable that the nucleic acid molecule be blended at a concentration that can achieve the exemplified administration conditions.

The drug for disease of the present invention may contain the nucleic acid molecule of the present invention only, or may further contain an additive(s) in addition to the nucleic acid molecule, for example. The blended amount of the additive is not particularly limited, as long as the function of the nucleic acid molecule is not hindered. The additive is not particularly limited, and preferably is a pharmaceutically acceptable additive, for example. The kind of the additive is not particularly limited, and can be selected as appropriate depending on the kind of the administration target, for example.

In the drug for disease of the present invention, the additive preferably is the one that forms a complex with the nucleic acid molecule, for example. In this case, the additive also can be referred to as a complexing agent, for example. In the drug for disease of the present invention, by forming a complex of the nucleic acid molecule with the additive, it is possible to deliver the nucleic acid molecule efficiently, for example. There is no particular limitation on the bond between the nucleic acid molecule and the complexing agent, and examples thereof include non-covalent bonds. The complex may be an inclusion complex, for example.

There is no particular limitation on the complexing agent, and examples thereof include polymers, cyclodextrins, and adamantine. Examples of the cyclodextrins include linear cyclodextrin copolymers and linear oxidized cyclodextrin copolymers.

Other examples of the additive include a carrier, a binding substance that binds to a target cell, a condensing agent, a fusogenic agent, an excipient, a base, a stabilizer, and a preservative.

<Treatment Method>

As described above, the method for treating a disease caused by the expression of periostin except for an eye disease according to the present invention includes the step of administering the drug for a disease caused by the expression of periostin except for an eye disease according to the present invention to a patient. The treatment method of the present invention is characterized in that the drug for a disease caused by the expression of periostin except for an eye disease of the present invention is used in treatment of the disease caused by the expression of periostin except for an eye disease, and other steps and conditions are by no means limited. The disease caused by the expression of periostin except for an eye disease to which the present invention is applicable is, for example, as described above.

As to the treatment method of the present invention, the description regarding the administration method for the drug for disease according to the present invention can be referred, for example.

<Use of Expression Inhibitory Nucleic Acid Molecule>

The expression inhibitory nucleic acid molecule of the present invention is a nucleic acid molecule for treatment of a disease caused by the expression of periostin except for an eye disease. Also, the expression inhibitory nucleic acid molecule of the present invention is a nucleic acid molecule for use in production of a drug for a disease caused by the expression of periostin except for an eye disease.

In the following, the present invention will be described in detail with reference to examples etc. It is to be noted, however, that the present invention is by no means limited thereto.

Examples

(Example 1)

siRNAs, nkRNAs, and PnkRNAs were synthesized to examine the inhibition of expression of the human periostin gene in vitro.

(1) Expression inhibitory nucleic acid molecule

As the expression inhibitory nucleic acid molecule of the present example, the double-stranded RNA having the following sequences was synthesized. In the double-stranded RNA, the upper sequence is a sense strand and the lower sequence is an antisense strand. The overhang at the 3' end of the sense strand and the overhang at the 3' end of the antisense strand are both n = 2, and the sequences thereof are both TT.

[Table 7]

NI-0079
(SEQ ID NO: 58) 5'-GCACCAAAAAGAAAUACUU(N)$_n$-3'
(SEQ ID NO: 20) 3'-(N)n CGUGGUUUUUCUUUAUGAA-5'

Also, as the expression inhibitory nucleic acid molecule of the present example, single-stranded RNAs represented by the sequences shown below were synthesized. In each of the single-stranded RNAs, the as1 nucleotide of SEQ ID NO: 1, which is the same as the as1 nucleotide in the antisense strand of the siRNA, is underlined.

[Table 8]

NK-0144

```
      ↓5'                           C
C  G G  AGCACCAAAAAGAAAUACUUUUCCC     A
                                        C
U  U CUUCGUGGUUUUUCUUUAUGAAAAGGC      A
                                    C
```

PK-0076

```
      ↓5'
    G  AGCACCAAAAAGAAAUACUUUUCC
  Ly                              Lx
    CUUCGUGGUUUUUCUUUAUGAAAAGG
```

In PK-0076, linkers (Lx) and (Ly) each have the following structure represented by the above formula (I-8϶)

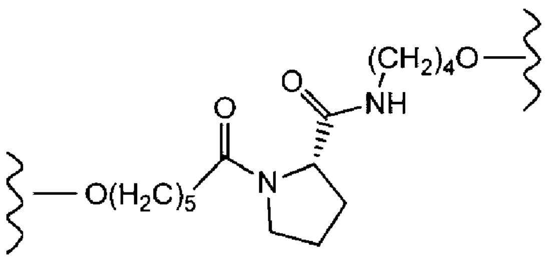

Furthermore, FITC-labeled expression inhibitory nucleic acid molecules were produced by labeling the 5' end of the sense strand of the double-stranded RNA and the 5' ends of the single-stranded RNAs with FITCs. Hereinafter, NI-0079, NK-0144, and PK-0076 each labeled with FITC are referred to as FITC-NI-0079, FITC-NK-0144, and FITC-PK-0076, respectively.

(2) Measurement of periostin gene expression level

A human dermal fibroblast (NB1RGB, furnished from Riken BioResource Center) was used to provide cells. The culture conditions were set to 37°C and 5% $CO_2$. As a medium, a 10% FBS-containing DMEM (SIGMA) was used.

First, the cells were cultured in the medium, and the resultant liquid culture was dispensed to a 24-well plate so that each well contained 400 μl of the liquid culture to achieve a density of $4 \times 10^4$ cells/well. Then, the cells were transfected with the double-stranded RNA or the single-stranded RNA using a transfection reagent Lipofectamine (registered trademark) 2000 (Invitrogen) in accordance with the protocol attached thereto. Specifically, 100 μl of the complex of the double-stranded RNA or the single-stranded RNA and the transfection reagent was added per well, so that, in each well, the total amount was 500 μl and the final concentration of the double-stranded RNA or the single-stranded RNA was 0.1, 0.3, 1, or 3 nmol/L.

After the transfection, the cells in the wells were cultured for 24 hours. Then, RNA was collected using an RNeasy (registered trademark) Mini Kit (Qiagen) in accordance with the protocol attached thereto. Next, cDNA was synthesized from the RNA using a reverse transcriptase (ReverTra Ace qPCR RT Master Mix with gDNA Remover, TOYOBO) in accordance with the protocol attached thereto. Then, PCR was carried out using a PCR reagent (THUNDERBIRD™ SYBR (registered trademark) qPCR Mix, TOYOBO) with the thus-obtained cDNA as a template, and the expression level of the periostin gene and the expression level of the human β-actin gene as the internal standard were measured. The expression level of the periostin gene was normalized with the expression level of the human β-actin gene. In the PCR, the periostin gene and the human ß-actin gene were amplified using the following primer sets, respectively. The relative value of the expression level of the gene was calculated, assuming that the expression level thereof in the cell group (-) to which the double-stranded RNA, the single-stranded RNA, and the transfection reagent had not been added was 1.

Primer set for periostin gene amplification

5'-TGCCCAGCAGTTTTGCCCAT-3' (SEQ ID NO: 79)
5'-CGTTGCTCTCCAAACCTCTA-3' (SEQ ID NO: 80)

Primer set for human ß-actin gene amplification

5'-GCCACGGCTGCTTCCAGCTCCTC-3' (SEQ ID NO: 81)
5'-AGGTCTTTGCGGATGTCCACGTCAC-3' (SEQ ID NO: 82)

As a control, the relative value of the gene expression level also was calculated in cells (mock) to which, in the transfection step, the double-stranded RNA had not been added and only the transfection reagent had been added.

(3) Results

The results thereof are shown in FIG. 5. FIG. 5 is a graph showing the relative value of the expression level of mRNA of the human periostin gene, and the vertical axis indicates the relative value of the gene expression level. As can be seen from FIG. 5, when the double-stranded RNAs and single-stranded RNAs of the present example were used, the expression levels were lower than those when the cell group (-) and control (mock) were used. From these results, it was confirmed that the double-stranded RNAs and single-stranded RNAs of the present example all have expression inhibitory activity. In particular, NI-0079 and FITC-NI-0079 exhibited very potent expression inhibitory activity.

(Example 2)

siRNAs, nkRNAs, and PnkRNAs were synthesized to examine the inhibition of expression of the mouse periostin gene in vitro.

(1) Expression inhibitory nucleic acid molecule

The expression inhibitory nucleic acid molecules and FITC-labeled expression inhibitory nucleic acid molecules in Example 1 were used.

(2) Measurement of periostin gene expression level

The relative value of the gene expression level was calculated in the same manner as in Example 1 (2), except that a mouse primary cultured fibroblast (isolated in Division of Medical Biochemistry, Department of Biomolecular Sciences, Saga Medical School, Faculty of Medicine, Saga University) was used instead of the human dermal fibroblast (NB1RGB), the final concentration of the double-stranded RNA or the single-stranded RNA in the transfection step was 10 nmol/L, and the mouse ß-actin gene was used instead of the human ß-actin gene as the internal standard. The mouse periostin gene and the mouse ß-actin gene were amplified using the following primer sets, respectively.

Primer set for mouse periostin gene amplification

5'-GAACGAATCATTACAGGTCC-3' (SEQ ID NO: 106)
5'-GGAGACCTCTTTTTGCAAGA-3' (SEQ ID NO: 107)

Primer set for mouse ß-actin gene amplification

5'-GTCGTACCACAGGCATTGTGATGG-3' (SEQ ID NO: 104)
5'-GCAATGCCTGGGTACATGGTGG-3' (SEQ ID NO: 105)

(3) Results

The results thereof are shown in FIG. 6. FIG. 6 is a graph showing the relative value of the expression level of mRNA of the mouse periostin gene, and the vertical axis indicates the relative gene expression level. As can be seen from FIG. 6, when the double-stranded RNAs and single-stranded RNAs of the present example were used, the expression levels were lower than those when the cell group (-) and the control (mock) were used. From these results, it was confirmed that the double-stranded RNAs and single-stranded RNAs of the present example all have very potent expression inhibitory activity.

(Example 3)

siRNAs and PnkRNAs were synthesized to examine the inhibition of expression of the mouse periostin gene in vitro.

(1) Expression inhibitory nucleic acid molecule

NI-0079 and PK-0076 in Example 1 were used. Also, as negative controls, the following double-stranded RNA and single-stranded RNA with the base sequences being scrambled were used.

NI-0000

(SEQ ID NO: 77) 5'-UACUAUUCGACACGCGGAGTT-3'
(SEQ ID NO: 78) 5'-CUUCGCGUGUCGAAUAGUATT-3'
PK-0000 (SEQ ID NO: 98) 5'-AUACUAUUCGACACGCGAAGUUCC-Lx-GGAACUUCGCGUGUCGAAUAGUAUUC-Ly-G-3'

(2) Measurement of periostin gene expression level

The relative value of the gene expression level was calculated in the same manner as in Example 1 (2), except that a NIH3T3 cell (furnished from Saga Medical School, Faculty of Medicine, Saga University) was used instead of the human dermal fibroblast (NB1RGB) and the mouse GAPDH gene was used instead of the human ß-actin gene as the internal standard. The mouse periostin gene and the mouse GAPDH were amplified using the following primer sets, respectively. As the negative control, the relative value of the gene expression level was calculated in the same manner as in Example 1 (2), except that NI-0000 or PK-0000 was used instead of the expression inhibitory nucleic acid molecule in Example 1.

Primer for mouse periostin gene amplification

5'-AAGCTGCGGCAAGACAAG-3' (SEQ ID NO: 99)
5'-GGGCTGTGTCAGGAGATCTTT-3' (SEQ ID NO: 100)

Primer set for mouse GAPDH gene amplification

5'-TGCACCACCAACTGCTTAGC-3' (SEQ ID NO: 101)
5'-GGCATGGACTGTGGTCATGAG-3' (SEQ ID NO: 102)

(3) Results

The results thereof are shown in FIG. 7. FIG. 7 is a graph showing the relative value of the expression level of mRNA of the mouse periostin gene, and the vertical axis indicates the relative gene expression level. As can be seen from FIG. 6, when NI-0079 was used, the expression level was lower than those when the cell group (-), the control (mock), and the negative control (NI-0000) were used. Also, when PK-0076 was used, the expression level was lower than those when the cell group (-), the control (mock), and the negative control (PK-0000) were used. From these results, it was confirmed that NI-0079 and PK-0076 have expression inhibitory activity.

(Example 4)

nkRNAs were synthesized to examine the inhibition of expression of the mouse periostin gene in vitro.

(1) Expression inhibitory nucleic acid molecule

nkRNA in Example 1 was used. Also, as a negative control, the following single-stranded RNA with the base sequence being scrambled was used.

NK-0000 (SEQ ID NO: 103) 5'-AUACUAUUCGACACGCGAAGUUCCCCACACCGGAACUUCGCGUGUCGAAUAGUAUUCUUCGG-3'

(2) Measurement of periostin gene expression level

The relative value of the gene expression level was calculated in the same manner as in Example 3 (2). As the negative control, the relative value of the gene expression level was calculated in the same manner as in Example 3 (2), except that NK-0000 was used instead of the expression inhibitory nucleic acid molecule in Example 3.

(3) Results

The results thereof are shown in FIG. 8. FIG. 8 is a graph showing the relative value of the expression level of mRNA of the mouse periostin gene, and the vertical axis indicates the relative gene expression level. As can be seen from FIG. 6, when NK-0144 was used, the expression level was lower than those when the cell group (-), the control (mock), and the negative control (NK-0000) were used. From these results, it was confirmed that NK-0144 has expression inhibitory activity.

(Example 5)

siRNA was administered to a damaged area of skin to evaluate the scar formation.

(1) Expression inhibitory nucleic acid molecule

NI-0079 in Example 1 was solved in a physiological saline so as to provide a NI-0079 solution having a predetermined concentration (1.5mg/mL). As a negative control, a physiological saline was used.

(2) Evaluation on scar formation

Four damages were provided on the auricle skin of the right ear of each of four rabbits (New Zealand White, female) (n = 4) by resecting the skin on the cartilage tissue into a circle using a biopsy punch having a diameter of 7 mm. Then, the NI-0079 solution was administered by injection to three of the four damages per rabbit. The NI-0079 solution was administered after predetermined days (7, 14, 21, 28, and 35 days), assuming that the day when the damage had been provided was day 0. The dose to be administered at one time with respect to one damaged part was 20 μL, and the NI-0079 solution was administered to different sites of the damaged part (inside or under the skin) by 10 μL. When 1.5mg/mL NI-0079 solution was used, the dose to be administered at one time with respect to one damaged part was 30 μg. Then, the right ears of the rabbits were collected after 42 days, and the evaluation was made on the scar formation with respect to each damaged part as follows.

In the evaluation of the scar formation, the damaged part of each of four rabbits, to which the NI-0079 solution had not been administered (one damaged part per rabbit, four damaged parts in total) was used as a control. Furthermore, with respect to each of 12 rabbits of the same kind, one circular damage was provided on the right ear. The scar formation was examined after 42 days without administering the NI-0079. The results thereof were also used as controls. That is, the results of 16 damaged parts in total were used as controls.

For examining the healing of the damaged part, each collected right rabbit ear was sliced in the thickness direction to provide a slice, and the slice was immobilized in 10% neutral buffered formalin, and then the HE staining was carried out. Then, the immobilized slice was observed with a light microscope to examine a layer newly formed on the cartilage tissue in the cross section in the thickness direction. As described above, the damage was formed by resecting the skin on the cartilage tissue. Thus, when the damage is healed, a new skin layer is formed on the cartilage tissue in place of the resected skin. However, in the model of the present experiment, the damaged part was not recovered to a normal skin tissue, and a hypertrophic scar was formed in which the damaged site is raised by causing hyperproliferation of the fibrous tissue. Hence, in the observation of the layer of the cross section in the thickness direction, the area (B) of the damaged site provided on the cartilage tissue and the area (A) of the scar raised in the damaged site in the damaged part were measured and the SEI was calculated by the following expression according to the paper (Morris et al., Plast. Reconstr. Surg., 1997, vol. 100, pp.674 to 681) to evaluate the scar formation. When a hypertrophic scar is formed, the area (A) of the raised part is increased. From this result, it can be evaluated that the scar formation is inhibited more effectively as the SEI value is closer to 1.

$$SEI = (A + B) / B$$

Regarding the measured SEI, the average value of the 12 damaged parts (four rabbits × three damaged parts) to which 1.5mg/mL NI-0079 solution had been administered and the average value of the controls of 16 damaged parts to which NI-0079 had not been administered were obtained.

(3) Results

The results thereof are shown in FIG. 9. FIG. 9 is a graph showing the results of the SEI corresponding to scar formation, and the vertical axis indicates the average value of SEI. As can be seen from FIG. 9, the SEI of the group to which the NI-0079 solution had been administered was lower than that of the control to which the NI-0079 had not been administered. From this result, it was confirmed that the scar formation of the group to which the NI-0079 solution had been administered was inhibited.

While the present invention has been described above with reference to illustrative embodiments, the present invention is by no means limited thereto. Various changes and modifications that may become apparent to those skilled in the art may be made in the configuration and specifics of the present invention without departing from the scope of the present invention.

This application claims priority from: Japanese Patent Application No. 2013-202051 filed on September 27, 2013. The entire disclosure of this Japanese Patent Application is incorporated herein by reference.

Industrial Applicability

According to the drug for a disease caused by the expression of periostin except for an eye disease of the present invention, it is possible to inhibit the expression of the periostin gene or the function of the periostin protein. Thus, for example, the present invention is effective in treatment of diseases (except for eye diseases) caused by the expression of the periostin gene or a periostin protein, specifically skin diseases, respiratory diseases, gastrointestinal diseases, and the like.

## Claims

1. A drug for a disease caused by expression of periostin except for an eye disease, comprising an expression inhibitory nucleic acid molecule for the periostin gene, the expression inhibitory nucleic acid molecule comprising the following nucleotide (as1), (as2), or (as3):

   (as1) a nucleotide that has a base sequence represented by any one of SEQ ID NOs: 1 to 19;
   (as2) a nucleotide that has a base sequence obtained by deletion, substitution, and/or addition of one to several bases in the base sequence of the nucleotide (as1) and has a function of inhibiting expression of the periostin gene; and
   (as3) a nucleotide that has a base sequence with an identity of at least 90% to the base sequence of the nucleotide (as1) and has a function of inhibiting expression of the periostin gene.

2. The drug according to claim 1, wherein
   the expression inhibitory nucleic acid molecule is a single-stranded nucleic acid molecule,
   the single-stranded nucleic acid molecule comprises, in sequence from a 5' side to a 3' side: a 5' side region (Xc); an inner region (Z); and a 3' side region (Yc),
   the inner region (Z) is composed of an inner 5' side region (X) and an inner 3' side region (Y) that are linked to each other,
   the 5' side region (Xc) is complementary to the inner 5' side region (X),
   the 3' side region (Yc) is complementary to the inner 3' side region (Y), and
   the inner region (Z) comprises the expression inhibitory sequence.

3. The drug according to claim 2, further comprising:

   a linker region (Lx) between the 5' side region (Xc) and the inner 5' side region (X); and
   a linker region (Ly) between the 3' side region (Yc) and the inner 3' side region (Y), wherein
   the 5' side region (Xc) and the inner 5' side region (X) are linked to each other via the linker region (Lx), and
   the 3' side region (Yc) and the inner 3' side region (Y) are linked to each other via the linker region (Ly).

4. The drug according to claim 3, wherein
   the linker regions (Lx) and (Ly) each comprise a nucleotide residue.

5. The drug according to claim 3 or 4, wherein
   the linker regions (Lx) and (Ly) each comprise a non-nucleotide residue.

6. The drug according to claim 5, wherein
   the non-nucleotide residue comprises at least one of a pyrrolidine skeleton and a piperidine skeleton.

7. The drug according to claim 5 or 6, wherein
   the linker regions (Lx) and (Ly) are each represented by the following formula (I):

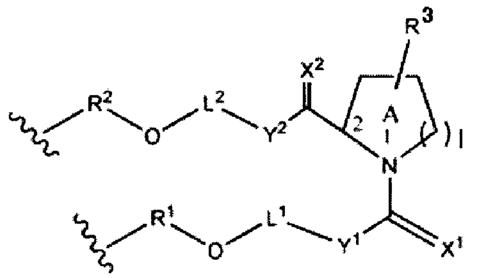

・・・(I)

where:

$X^1$ and $X^2$ are each independently $H_2$, O, S, or NH;
$Y^1$ and $Y^2$ are each independently a single bond, $CH_2$, NH, O, or S;
$R^3$ is a hydrogen atom or a substituent that is bound to C-3, C-4, C-5, or C-6 on a ring A;
$L^1$ is an alkylene chain composed of n atoms, and a hydrogen atom on an alkylene carbon atom may or may not be substituted with OH, $OR^a$, $NH_2$, $NHR^a$, $NR^aR^b$, SH, or $SR^a$, or
$L^1$ is a polyether chain obtained by substituting at least one carbon atom on the alkylene chain with an oxygen atom,
provided that: when $Y^1$ is NH, O, or S, an atom bound to $Y^1$ in $L^1$ is carbon, an atom bound to $OR^1$ in $L^1$ is carbon, and oxygen atoms are not adjacent to each other;
$L^2$ is an alkylene chain composed of m atoms, and a hydrogen atom on an alkylene carbon atom may or may not be substituted with OH, $OR^c$, $NH_2$, $NHR^c$, $NR^cR^d$, SH, or $SR^c$, or
$L^2$ is a polyether chain obtained by substituting at least one carbon atom on the alkylene chain with an oxygen atom,
provided that: when $Y^2$ is NH, O, or S, an atom bound to $Y^2$ in $L^2$ is carbon, an atom bound to $OR^2$ in $L^2$ is carbon, and oxygen atoms are not adjacent to each other;
$R^a$, $R^b$, $R^e$, and $R^d$ are each independently a substituent or a protecting group;
1 is 1 or 2;
m is an integer in the range from 0 to 30;
n is an integer in the range from 0 to 30;
on the ring A, one carbon atom other than C-2 may be substituted with nitrogen, oxygen, or sulfur,
the ring A may comprise a carbon-carbon double bond or a carbon-nitrogen double bond; and
the regions (Xc) and (X) are each linked to the linker region (Lx) via $-OR^1-$ or $-OR^2-$, where $R^1$ and $R^2$ may or may not be present, and when they are present, $R^1$ and $R^2$ are each independently a nucleotide residue or the structure of the formula (I).

**8.** The drug according to any one of claims 2 to 7, wherein
the number of bases (Z) in the inner region (Z), the number of bases (X) in the inner 5' side region (X), the number of bases (Y) in the inner 3' side region (Y), the number of bases (Xc) in the 5' side region (Xc), and the number of bases (Yc) in the 3' side region (Yc) satisfy conditions of Expressions (1) and (2) below:

$$Z = X + Y \qquad \dots (1)$$

$$Z \geq Xc + Yc \qquad \dots (2).$$

**9.** The drug according to claim 8, wherein
the difference between the number of bases (X) in the inner 5' side region (X) and the number of bases (Xc) in the 5' side region (Xc), and the difference between the number of bases (Y) in the inner 3' side region (Y) and the number of bases (Yc) in the 3' side region (Yc) satisfy the following conditions:

(a) Conditions of Expressions (11) and (12) are satisfied;

$$X - Xc = 1, 2, \text{or } 3 \quad \ldots (11)$$

$$Y - Yc = 0 \quad \ldots (12)$$

(b) Conditions of Expressions (13) and (14) are satisfied;

$$X - Xc = 0 \quad \ldots (13)$$

$$Y - Yc = 1, 2, \text{or } 3 \quad \ldots (14)$$

(c) Conditions of Expressions (15) and (16) are satisfied;

$$X - Xc = 1, 2, \text{or } 3 \quad \ldots (15)$$

$$Y - Yc = 1, 2, \text{or } 3 \quad \ldots (16)$$

(d) Conditions of Expressions (17) and (18) are satisfied;

$$X - Xc = 0 \quad \ldots (17)$$

$$Y - Yc = 0 \quad \ldots (18).$$

**10.** The drug according to any one of claims 1 to 9, wherein the expression inhibitory sequence has a length of 18- to 32-mer.

**11.** The drug according to any one of claims 1 to 9, wherein the sequence of the expression inhibitory nucleic acid molecule is at least one base sequence selected from the group consisting of SEQ ID NOs: 83 to 97.

**12.** The drug according to claim 1, wherein the expression inhibitory sequence further comprises an overhang sequence, and the overhang sequence is added to the 3' end of the nucleotide.

**13.** The drug according to claim 1 or 12, wherein the expression inhibitory sequence is a nucleotide that has a base sequence represented by any one of SEQ ID NOs: 20 to 38 (where n is a positive integer) comprising the nucleotide (as1).

**14.** The drug according to any one of claims 1, 12, and 13, wherein the expression inhibitory sequence has a length of 18- to 32-mer.

**15.** The drug according to any one of claims 1 and 12 to 14, further comprising a complementary sequence that anneals to the expression inhibitory sequence, wherein the complementary sequence comprises a nucleotide that is complementary to the nucleotide (as1), (as2), or (as3) in the expression inhibitory sequence.

**16.** The drug according to claim 15, wherein the nucleotide in the complementary sequence is the following nucleotide (s1), (s2), or (s3):

(s1) a nucleotide that has a base sequence complementary to the base sequence of the nucleotide (as1);
(s2) a nucleotide that has a base sequence complementary to the base sequence of the nucleotide (as2); and
(s3) a nucleotide that has a base sequence complementary to the base sequence of the nucleotide (as3).

**17.** The drug according to claim 16, wherein
the nucleotide (s1) has a base sequence represented by any one of SEQ ID NOs: 39 to 57.

**18.** The drug according to any one of claims 15 to 17, wherein
the complementary sequence further comprises an overhang sequence, and
the overhang sequence is added to the 5' end of the nucleotide.

**19.** The drug according to any one of claims 15 to 18, wherein
the complementary sequence is a nucleotide that has a base sequence represented by any one of SEQ ID NOs: 58 to 76 (where n is a positive integer) comprising the nucleotide (s1).

**20.** The drug according to any one of claims 12 to 19, wherein
the overhang sequence has a length of 1- to 3-mer.

**21.** The drug according to any one of claims 12 to 20, wherein
the expression inhibitory nucleic acid molecule is a double-stranded nucleic acid molecule composed of two single strands, and an antisense strand thereof comprises the expression inhibitory sequence and a sense strand thereof comprises the complementary sequence.

**22.** The drug according to any one of claims 1 to 21, wherein
the disease caused by expression of periostin except for an eye disease is at least one selected from the group consisting of skin diseases, respiratory diseases, and gastrointestinal diseases.

**23.** The drug according to claim 22, wherein
the skin disease is at least one selected from the group consisting of atopic dermatitis, wounds, psoriasis, scleroderma, keloids, hypertrophic scars, and melanoma.

**24.** The drug according to claim 22, wherein
the respiratory disease is at least one selected from the group consisting of bronchial asthma, idiopathic interstitial pneumonia, and non-idiopathic interstitial pneumonia.

**25.** The drug according to claim 22, wherein
the gastrointestinal disease is cholangiocarcinoma.

**26.** A method of treating a disease caused by expression of periostin except for an eye disease, the method comprising the step of
administering the drug according to any one of claims 1 to 25 to a patient.

**27.** The method according to claim 26, wherein
the disease caused by expression of periostin except for an eye disease is at least one selected from the group consisting of skin diseases, respiratory diseases, and gastrointestinal diseases.

**28.** The method according to claim 27, wherein
the skin disease is at least one selected from the group consisting of atopic dermatitis, wounds, psoriasis, scleroderma, keloids, hypertrophic scars, and melanoma.

**29.** The method according to claim 27, wherein
the respiratory disease is at least one selected from the group consisting of bronchial asthma, idiopathic interstitial pneumonia, and non-idiopathic interstitial pneumonia.

**30.** The method according to claim 27, wherein
the gastrointestinal disease is cholangiocarcinoma.

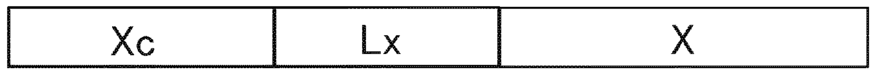


FIG. 1A

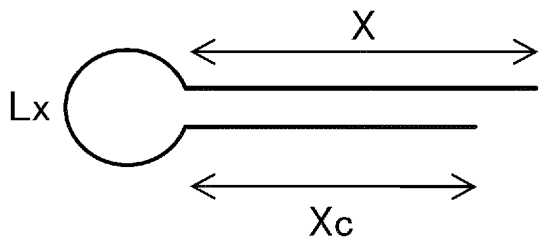


FIG. 1B

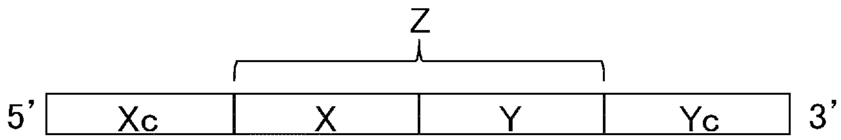


FIG. 2A

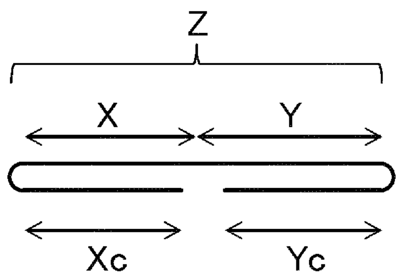


FIG. 2B

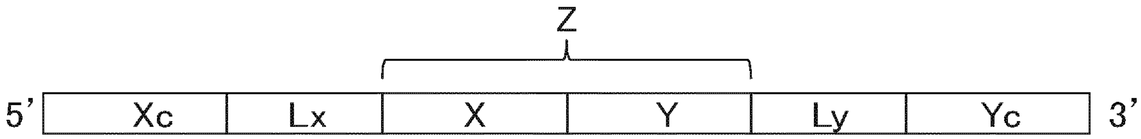


FIG. 3A

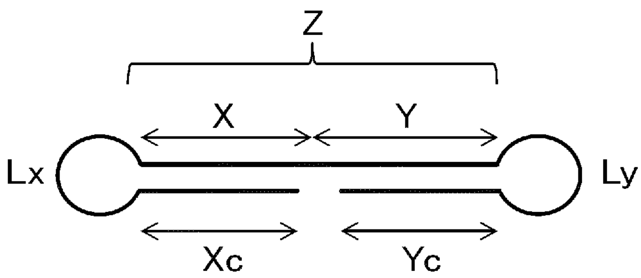


FIG. 3B

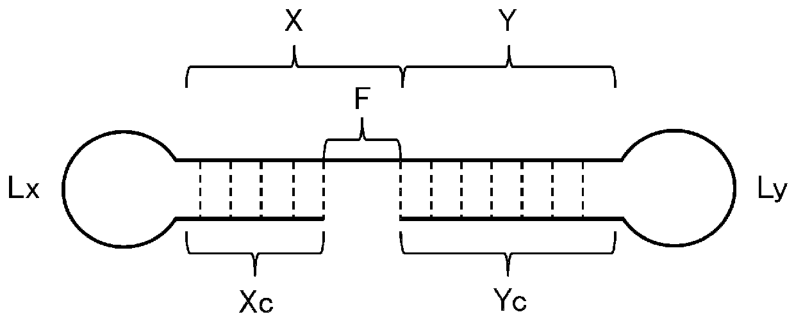


FIG. 4A

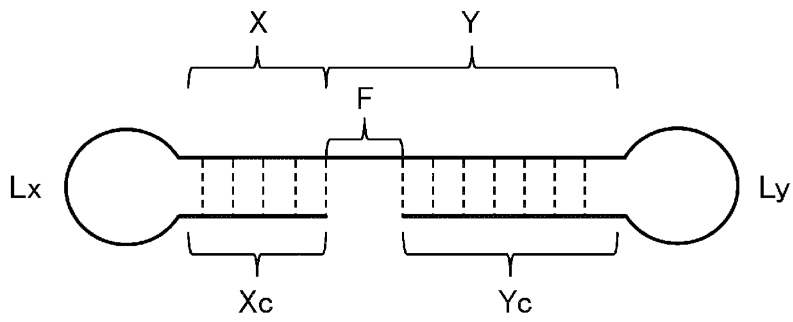


FIG. 4B

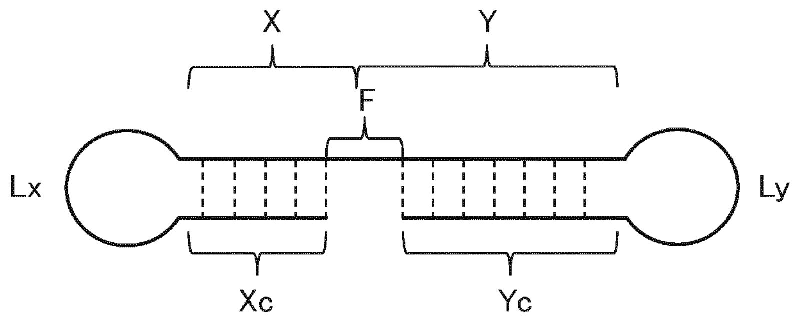


FIG. 4C

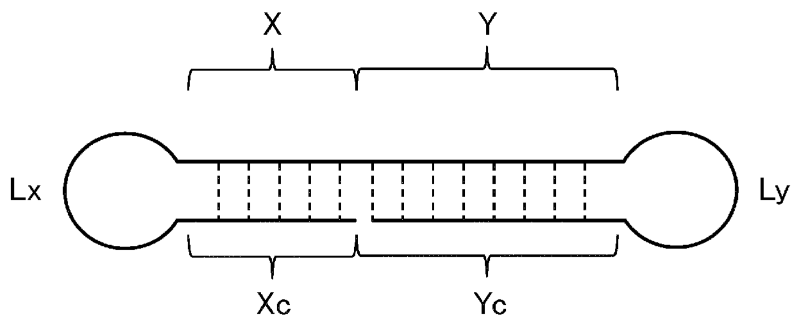


FIG. 4D

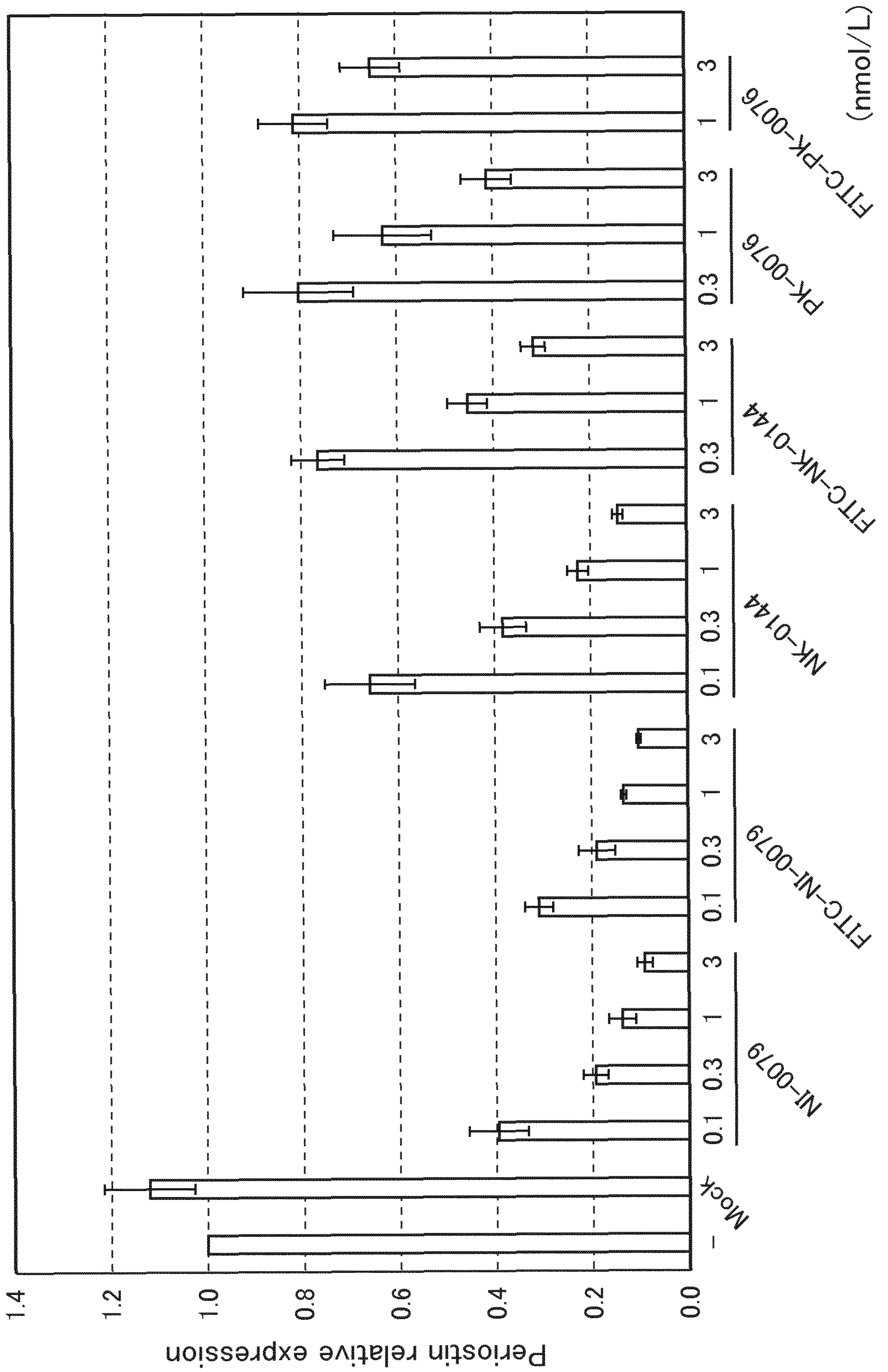

Periostin relative expression
0.0
0.2
0.4
0.6
0.8
1.0
1.2
1.4
–
Mock
0.1
0.3
1
3
NI-0079
FITC-NI-0079
NK-0144
FITC-NK-0144
PK-0076
FITC-PK-0076
(nmol/L)


FIG. 5

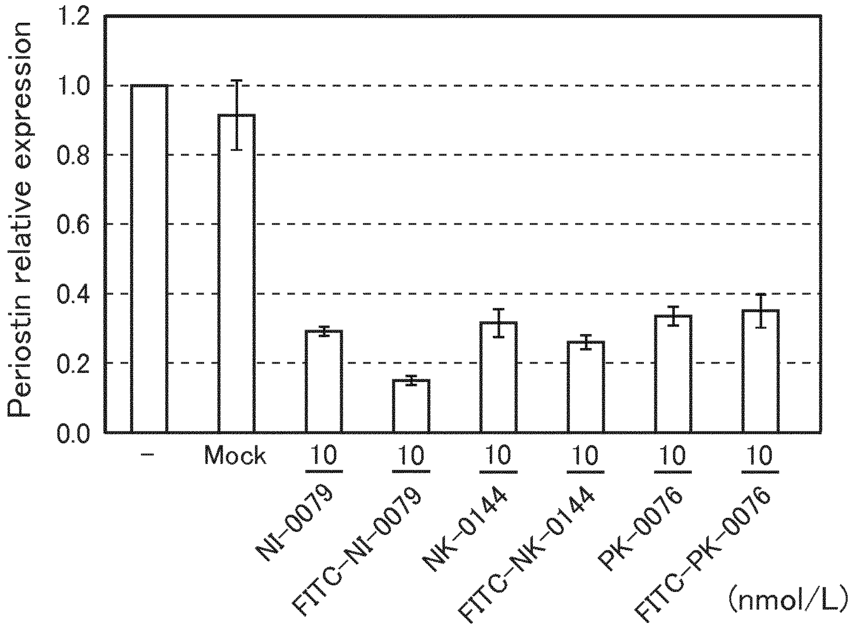
Periostin relative expression
1.2
1.0
0.8
0.6
0.4
0.2
0.0
–
Mock
10
10
10
10
10
10
NI-0079
FITC-NI-0079
NK-0144
FITC-NK-0144
PK-0076
FITC-PK-0076
(nmol/L)

FIG. 6

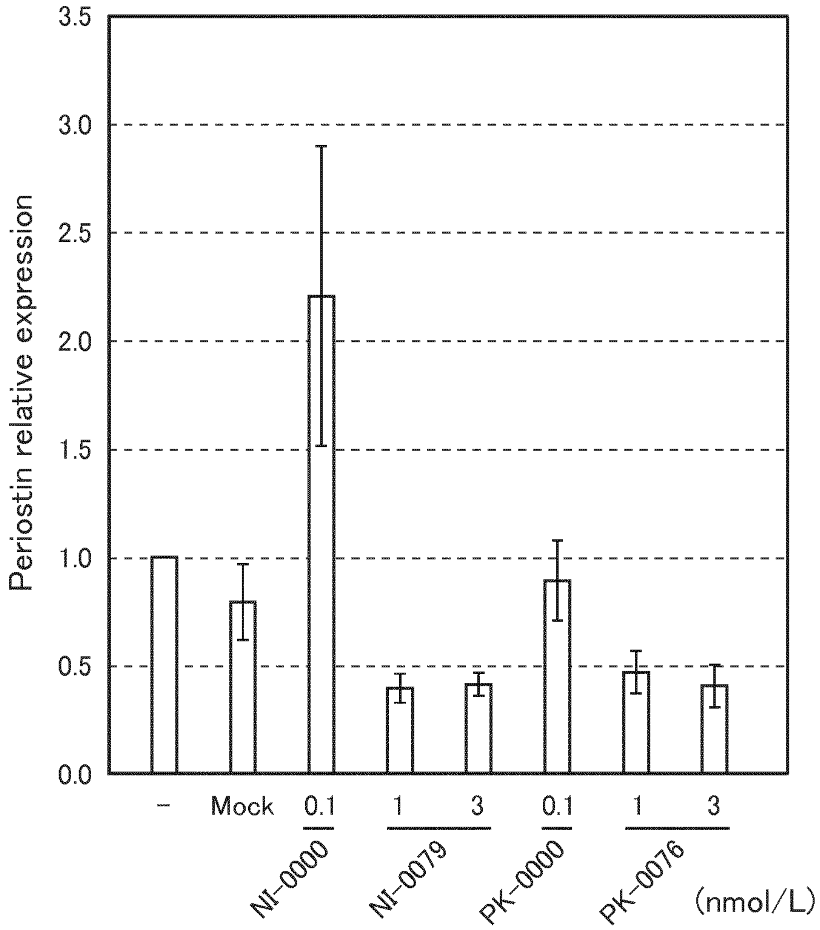
Periostin relative expression
3.5
3.0
2.5
2.0
1.5
1.0
0.5
0.0
–
Mock
0.1
1
3
0.1
1
3
NI-0000
NI-0079
PK-0000
PK-0076
(nmol/L)

FIG. 7

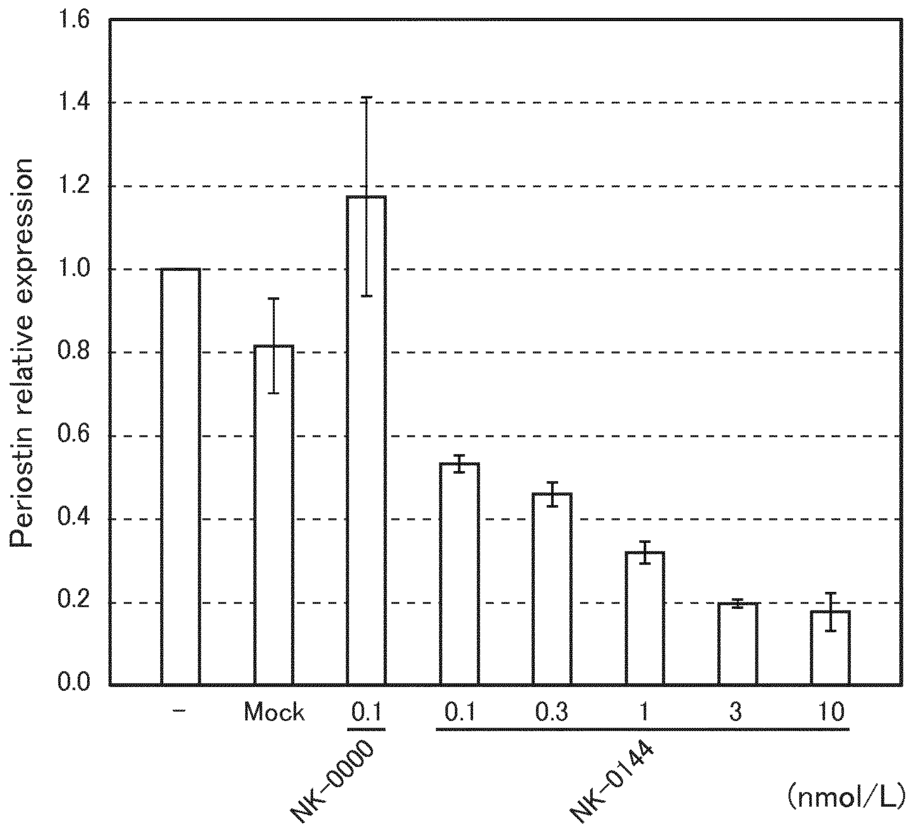

1.6
1.4
1.2
1.0
0.8
0.6
0.4
0.2
0.0
Periostin relative expression
−
Mock
0.1
0.1
0.3
1
3
10
NK-0000
NK-0144
(nmol/L)


FIG. 8

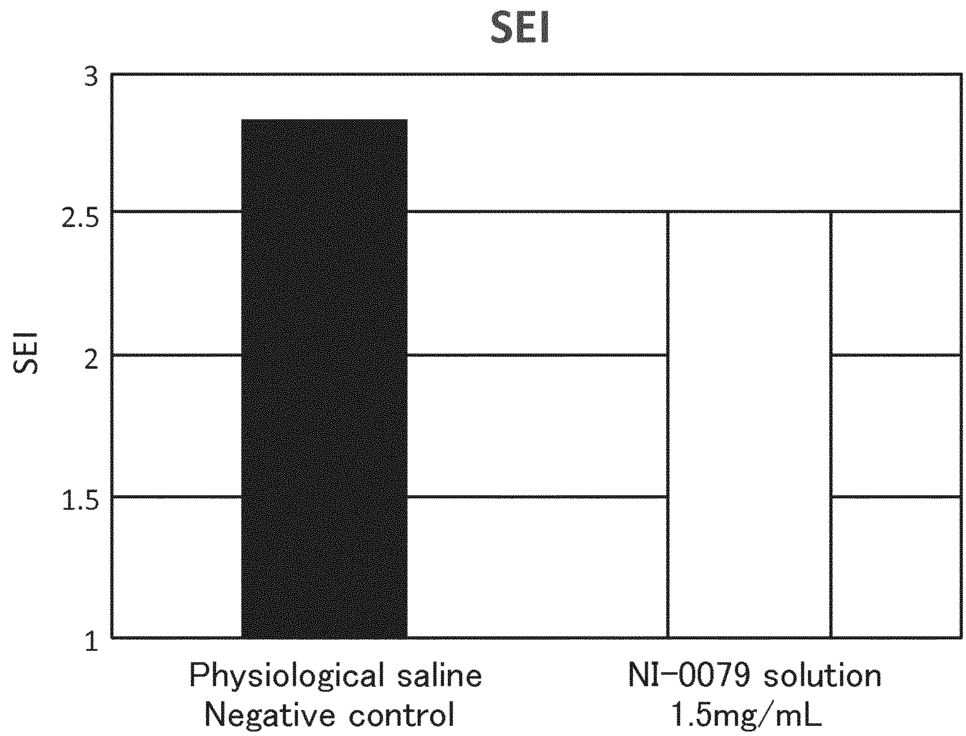

SEI
3
2.5
2
1.5
1
SEI
Physiological saline
Negative control
NI-0079 solution
1.5mg/mL


FIG. 9

## INTERNATIONAL SEARCH REPORT

International application No.
PCT/JP2014/075683

A. CLASSIFICATION OF SUBJECT MATTER
*A61K31/7088*(2006.01)i, *A61K35/76*(2006.01)i, *A61K48/00*(2006.01)i, *A61P1/00*(2006.01)i, *A61P11/00*(2006.01)i, *A61P11/06*(2006.01)i, *A61P17/00*(2006.01)i, *A61P17/02*(2006.01)i, *A61P35/00*(2006.01)i, *C12N15/113*(2010.01)n

According to International Patent Classification (IPC) or to both national classification and IPC

B. FIELDS SEARCHED

Minimum documentation searched (classification system followed by classification symbols)
A61K31/7088, A61K35/76, A61K48/00, A61P1/00, A61P11/00, A61P11/06, A61P17/00, A61P17/02, A61P35/00, C12N15/113

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2010-145294 A (Saga University), 01 July 2010 (01.07.2010), particularly, paragraphs [0012] to [0014]; SEQ ID NO:1<br>(Family: none) | 1<br>2-25 |
| Y | WO 2012/17919 A1 (Bonac Corp.), 09 February 2012 (09.02.2012), particularly, paragraphs [0017] to [0106]; fig. 3<br>& JP 4965745 B & JP 4968811 B<br>& JP 2012-130342 A & US 2012/0035246 A1<br>& US 2014/0171486 A1 & US 2014/0171633 A1<br>& US 2012/0010271 A1 & EP 2436767 A1<br>& EP 2628801 A1 & WO 2012/005368 A<br>& CN 103052711 A & KR 10-2013-0095738 A | 2-25 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>13 November, 2014 (13.11.14) | Date of mailing of the international search report<br>25 November, 2014 (25.11.14) |
| Name and mailing address of the ISA/<br>Japanese Patent Office<br>Facsimile No. | Authorized officer<br><br>Telephone No. |

Form PCT/ISA/210 (second sheet) (July 2009)

INTERNATIONAL SEARCH REPORT

International application No. PCT/JP2014/075683

C (Continuation). DOCUMENTS CONSIDERED TO BE RELEVANT

| Category* | Citation of document, with indication, where appropriate, of the relevant passages | Relevant to claim No. |
|---|---|---|
| Y | LIU,Y. et al, Enhanced proliferation, invasion, and epithelial-mesenchymal transition of nicotine-promoted gastric cancer by periostin, World Journal of Gastroenterology, 2011, Vol.17, No.21, p.2674-2680, particularly, page 2675, right column | 2-25 |
| Y | WO 2011/68176 A1 (Saga University), 09 June 2011 (09.06.2011), (Family: none) | 22-25 |
| Y | JP 2011-523350 A (Genentech, Inc.), 11 August 2011 (11.08.2011), & US 2011/0123530 A1 & EP 2271770 A & EP 2631302 A2 & WO 2009/124090 A1 & AU 2009231733 A & CA 2718120 A & KR 10-2010-0131003 A & MX 2010010746 A & CN 102232113 A & RU 2010144502 A & NZ 588853 A | 22-25 |
| Y | JP 2010-96748 A (Saga University), 30 April 2010 (30.04.2010), (Family: none) | 22-25 |
| Y | WO 2009/148184 A1 (Saga University), 10 December 2009 (10.12.2009), & US 2011/0086360 A1 & US 2013/0230861 A1 & EP 2295599 A1 | 22-25 |
| P,X | WO 2013/147140 A1 (Kyushu University), 03 October 2013 (03.10.2013), & TW 201343668 A | 1-25 |
| P,A | WO 2014/136910 A1 (Osaka University), 12 September 2014 (12.09.2014), (Family: none) | 1-25 |
| P,A | JP 5438239 B1 (Trend Sign Co., Ltd.), 20 December 2013 (20.12.2013), & WO 2014/073646 A | 1-25 |

Form PCT/ISA/210 (continuation of second sheet) (July 2009)

**INTERNATIONAL SEARCH REPORT**

International application No.
PCT/JP2014/075683

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 26-30
because they relate to subject matter not required to be searched by this Authority, namely:
Claims 26 to 30 pertain to methods for treatment of the human body or animal body by surgery or therapy and thus relate to a subject matter on which this International Searching Authority is not required to carry out an international search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4155561 B
- WO 2009148184 A
- JP 2010145294 A
- WO 2011068176 A
- WO 2012005368 A
- WO 2012017979 A
- US 3687808 A
- US 46566503 P
- US 0407070 W
- JP 2013202051 A


### Non-patent literature cited in the description


- **MASUTAKA FURUE et al.** atopic dermatitis practice guideline. *The Japanese Journal of Dermatology,* 2009, vol. 119 (8), 1515-1534
- **MIHO MASUOKA et al.** Periostin promotes chronic allergic inflammation in response to Th2 cytokines. *The Journal of Clinical Investigation,* 02 July 2012, vol. 122 (7), 2590-2600
- **J. F. W. MCOMIE.** Protecting Groups in Organic Chemistry. Plenum Press, 1973
- **LIMBACH et al.** Summary: the modified nucleosides of RNA. *Nucleic Acids Res.,* 1994, vol. 22, 2183-2196
- Concise Encyclopedia of Polymer Science and Engineering. John Wiley & Sons, 1990, 858-859
- **ENGLISCH et al.** *Angewandte Chemie, International Edition,* 1991, vol. 30, 613
- **MORRIS et al.** *Plast. Reconstr. Surg.,* 1997, vol. 100, 674-681